# EUROPEAN PATENT APPLICATION

(11) **EP 4 821 523 A1**
(43) Date of publication of application: **14.10.2026**
(21) Application number: 24900688.3
(22) Date of filing: 06.12.2024
(51) Int. Cl.: A61M 5/315

(54) **SYRINGE**

(30) Priority: 07.12.2023 JP 2023206990
(71) Applicant: Taisei Kako Co., Ltd., Kita-ku Osaka-shi Osaka 531-0072 (JP)
(72) Inventor: NAKAKUBO, Yutaro, Ibaraki-shi, Osaka 567-0054 (JP)
(74) Representative: DTS Patent- und Rechtsanwälte PartmbB
(86) International application number: PCT/JP2024/043139
(87) International publication number: WO 2025/121395

(57) **Abstract**

Provided is a syringe that includes a barrel, a plunger including a first shaft and a second shaft, and a biasing member. The first shaft includes a restriction locking part and a push-in locking part, and the second shaft includes a push-in locked part and a restriction part. The restriction part is configured to be pivotally movable about the starting end as a starting point between a restriction withdrawn position where a free end on the proximal end side of the restriction part is located radially inward and a restriction implementing position where the free end is located radially outward. The restriction part includes a restriction locked part at the free end that is locked to the restriction locking part by a biasing force of the biasing member in the restriction implementing position. The syringe further includes a restriction release part that moves the restriction part from the restriction implementing position to the restriction withdrawn position at a position at which the push-in for the administration of one separate portion of the content is completed, thereby releasing the restriction locked part from the locking engagement with the restriction locking part.

## Description

### FIELD OF THE INVENTION

### CROSS-REFERENCE TO RELATED APPLICATION

This application claims priority to Japanese Patent Application No. 2023-206990, the disclosure of which is incorporated herein by reference in its entirety.

The present invention relates to a syringe that can administer a medical liquid filled inside into the nasal cavity or oral cavity.

### BACKGROUND OF THE INVENTION

Conventionally, a syringe-type injector that divides a medical liquid or the like and discharges each divided portion is known (Patent Literature 1). As shown in Fig. 33, the syringe-type injector 901 includes a syringe 902 and a plunger 903 configured to be inserted into the syringe 902. The plunger 903 includes a first shaft member 903a and a second shaft member 903b held on a rear side of the first shaft member 903a.

The first shaft member 903a includes a first elastic piece 931, a second elastic piece 932, and a third elastic piece 933, respectively disposed at different axial positions. The first elastic piece 931, the second elastic piece 932, and the third elastic piece 933 have the same shape and are elastically deformable radially inward or radially outward.

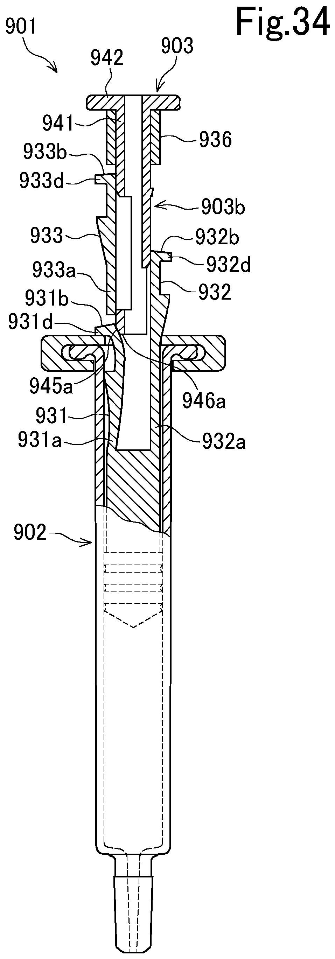

As shown in Fig. 34, the first elastic piece 931 extends from a proximal end part 931a toward a free end part 931b on a rear side and includes a locking projection 931d that can lock to a rear end of the syringe 902.

The second elastic piece 932 is located on the rear side of the first elastic piece 931. The second elastic piece 932 is disposed at a different circumferential position from that of the first elastic piece 931 in the circumferential direction. The second elastic piece 932 extends from a proximal end part 932a toward a free end part 932b on the rear side and includes a locking projection 932d that can lock to the rear end of the syringe 902.

The third elastic piece 933 is located on the rear side of the second elastic piece 932. The third elastic piece 933 is disposed at the same circumferential position as the first elastic piece 931. The third elastic piece 933 extends from a proximal end part 933a toward a free end part 933b on the rear side, and includes a locking projection 933d that can lock to the rear end of the syringe 902.

The second shaft member 903b is held on the rear side of the first shaft member 903a. The second shaft member 903b includes a pressing shaft 941 disposed inside a cylindrical portion 936 and a pressing flange 942 disposed at a rear portion of the pressing shaft 941.

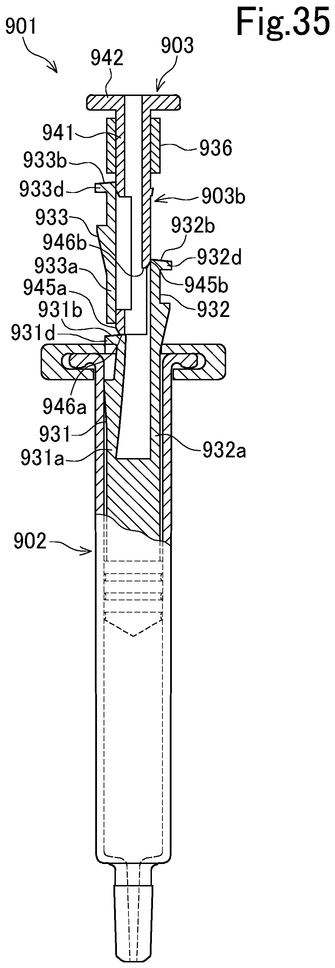

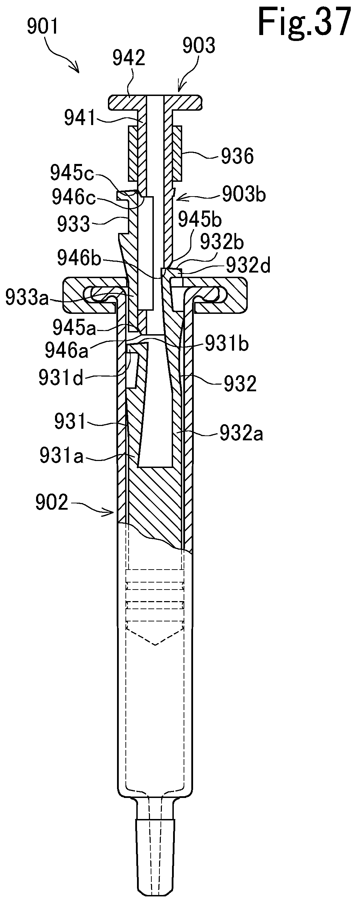

As shown in Fig. 35 and Fig. 37, the pressing shaft 941 includes a first support part 945a that supports the free end part 931b from the radially inner side when the first elastic piece 931 enters the syringe 902, thereby causing the locking projection 931d to lock to the rear end of the syringe 902. The pressing shaft 941 includes a second support part 945b that supports the free end part 932b from the radially inner side when the second elastic piece 932 enters the syringe 902. The pressing shaft 941 includes a third support part 945c that supports the free end part 933b from the radially inner side when the third elastic piece 933 enters the syringe 902.

The pressing shaft 941 includes a first pressing part 946a disposed at a distal end thereof. The first pressing part 946a can axially press the free end part 931b of the first elastic piece 931, in which the locking projection 931d has released the locking engagement with the rear end of the syringe 902. Similarly, the pressing shaft 941 includes a second pressing part 946b that can axially press the free end part 932b of the second elastic piece 932, and a third pressing part 946c that can axially press the free end part 933b of the third elastic piece 933. The first pressing part 946a is disposed at the distal end of the pressing shaft 941.

A description will be given hereinbelow for the usage of the syringe-type injector 901. This syringe-type injector 901 is configured to divide the content in a storage space in small portions each having a predetermined amount and discharge the same by gradually pushing the plunger 903.

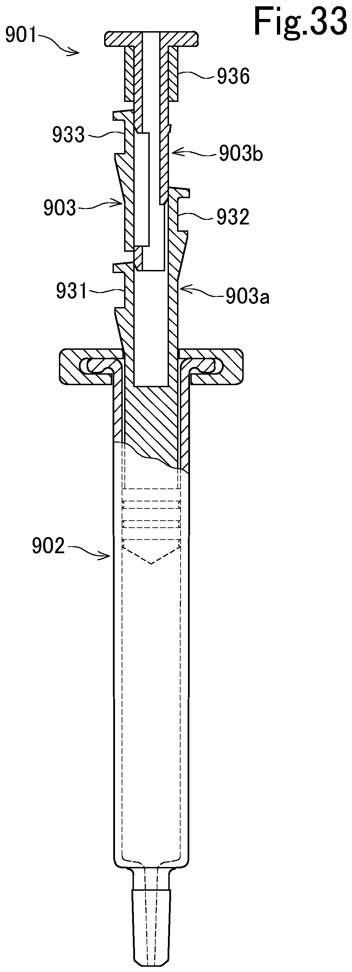

First, when the plunger 903 is pushed in from the initial state shown in Fig. 33, the first elastic piece 931 enters the syringe 902. At this time, a force is applied to the first elastic piece 931 radially inward. Since the free end part 931b of the first elastic piece 931 is supported by the first support part 945a, an area between the proximal end part 931a and the free end part 931b, of the first elastic piece 931 elastically bends and deforms radially inward. As shown in Fig. 34, the locking projection 931d of the first elastic piece 931, which is supported by the first support part 945a, locks to the rear end of the syringe 902, thereby stopping the pushing-in of the plunger 903 and ending the first discharge.

When the forward pressing force of the pressing flange 942 is weakened after the locking projection 931d locks to the rear end of the syringe 902, a restoring force of the first elastic piece 931 causes the second shaft member 903b to be pushed rearward and move. As the second shaft member 903b moves rearward, the first support part 945a moves further rearward than the free end part 931b of the first elastic piece 931. Therefore, as shown in Fig. 35, the free end part 931b of the first elastic piece 931 moves radially inward, and the locking projection 931d is released from the locking engagement with the rear end of the syringe 902.

Next, when the plunger 903 is pushed in from the state shown in Fig. 35, the second elastic piece 932 enters the syringe 902 while undergoing elastic deformation. The locking projection 932d of the second elastic piece 932, which is supported by the second support part 945b, locks to the rear end of the syringe 902, thereby stopping the entry of the plunger 903 and ending the second discharge. When the pressing force on the plunger 903 is weakened in this state, a restoring force of the second elastic piece 932 pushes the second shaft member 903b rearward, moving it to the state shown in Fig. 37, and enabling the third discharge.

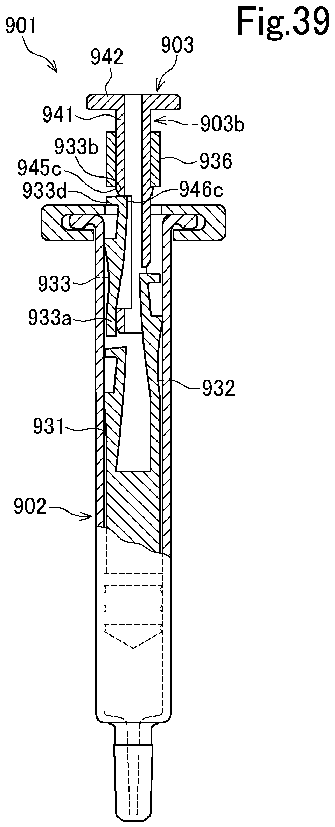

Next, when the plunger 903 is pushed in from the state shown in Fig. 37, the third elastic piece 933 enters the syringe 902 while undergoing elastic deformation, as shown in Fig. 37. The locking projection 933d, supported by the third support part 945c, locks to the rear end of the syringe 902, stopping the entry of the plunger 903 and ending the third discharge. When the pressing force on the plunger 903 is weakened in this state, a restoring force of the third elastic piece 933 pushes the second shaft member 903b rearward, moving it to the state shown in Fig. 39, and enabling the fourth discharge.

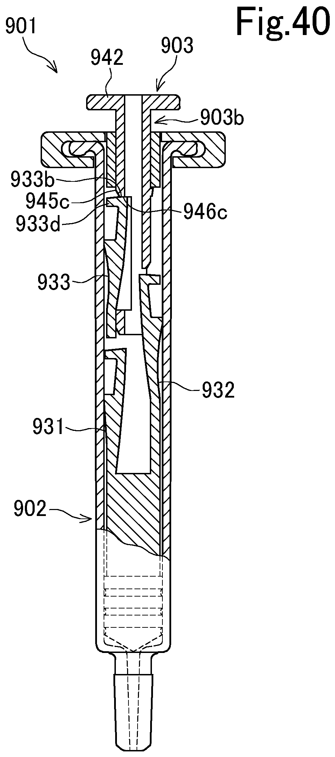

Finally, the fourth discharge is performed by pushing the plunger 903 into the syringe 902 from the state shown in Fig. 39 to the state shown in Fig. 40. In this way, the syringe-type injector 901 allows the content of the syringe 902 to be discharged in four separate portions.

### CITATION LIST

### Patent Literature

Patent Literature 1: JP 2018-108146 A

### SUMMARY

### Technical Problem

However, in the syringe-type injector 901 described above, there is a case where the elastic forces of the first, second, and third elastic pieces 931, 932, and 933 are insufficient to move the first, second, and third support parts 945a, 945b, and 945c completely rearward, and the first, second, and third pressing parts 946a, 946b, and 946c may not reach the positions at which they respectively press against the free end parts, which may not enable the next discharge.

An object of the present invention is to provide a syringe that can reliably discharge a content multiple times.

### [Solution to Problem]

According to the present invention, there is provided a syringe for dividing a content and administering a content in separate portions, the syringe including: a barrel having a distal end part for discharging the content and a proximal end part located on an opposite side to the distal end part in an axial direction; a plunger that can be pushed into the barrel from the proximal end part toward the distal end part, the plunger including a first shaft, and a second shaft that is inserted into the first shaft and movable in the axial direction relative to the first shaft; and a biasing member that biases the second shaft toward the proximal end side relative to the first shaft, the first shaft including: a restriction locking part for restricting the movement of the second shaft relative to the first shaft by a biasing force of the biasing member, and being composed of a plurality of restriction locking parts arranged at intervals in the axial direction; and a push-in locking part, to which the second shaft is locked, when the plunger for administration of one separate portion of the content is pushed into the barrel, the second shaft including: a push-in locked part that is locked to the push-in locking part when in the push-in; and a restriction part, the restriction part being configured to be pivotally movable about a starting end as a starting point between a restriction withdrawn position where a free end on the proximal end side of the restriction part is located radially inward and a restriction implementing position where the free end is located radially outward, and the restriction part including a restriction locked part at the free end that is locked to the restriction locking part by the biasing force of the biasing member in the restriction implementing position, the syringe further including a restriction release part that moves the restriction part from the restriction implementing position to the restriction withdrawn position at a position at which the push-in for the administration of the one separate portion of the content is completed, thereby releasing the restriction locked part from the locking engagement with the restriction locking part.

The syringe can be configured such that the restriction part is moved to the restriction withdrawn position by the restriction release part, and then moves to the restriction implementing position between the restriction locking part of the plurality of the restriction locking parts, to which the restriction part has locked for the administration of the one separate portion of the content, and the restriction locking part that is located adjacent to the restriction locking part locked for the administration of the one separate portion of the content, on the proximal end side in the axial direction.

The syringe can be configured such that the first shaft includes a first main shaft that extends in the axial direction, the push-in locking part includes a plurality of main-shaft push-in locking parts disposed on the first main shaft at intervals in the axial direction, the second shaft includes a second main shaft that extends in the axial direction, and a push-in part that is disposed on the second main shaft part, the push-in part being configured to be pivotally movable about a starting end as a starting point, which is disposed on the proximal end side of the push-in part, between a push-in withdrawn position where a free end disposed on the proximal end side of the push-in part is located radially inward and a push-in implementing position where the free end is located radially outward, the push-in locked part is a main-shaft push-in locked part disposed at the free end of the push-in part, and is composed of a main-shaft push-in locked part that is locked to the main-shaft push-in locking part when in the push-in in the push-in implementing position.

The syringe can be configured such that the syringe further includes a push-in guide part that guides the push-in part from the push-in implementing position to the push-in withdrawn position between two axially adjacent ones of the main-shaft push-in locking parts, the push-in guide part being configured to retain the push-in part in the push-in withdrawn position when the push-in part moves along the axial direction from the main-shaft push-in locking part on the distal end side to the main-shaft push-in locking part on the proximal end side, of the two main-shaft push-in locking parts, and release the retaining of the push-in part when the push-in part is positioned on the proximal end side relative to the main-shaft push-in locking part on the proximal end side.

### BRIEF DESCRIPTION OF DRAWINGS

Fig. 1A is a perspective view of a syringe in its initial state according to this embodiment.
Fig. 1B is a left side view of a main part of the syringe in its initial state.
Fig. 1C is a cross-sectional view taken along a line IC-IC in Fig. 1B.
Fig. 2A is an exploded perspective view of the syringe.
Fig. 2B is a front view of a second shaft of a plunger of the syringe.
Fig. 2C is a perspective view of the second shaft as seen from the left side.
Fig. 2D is a perspective view of the second shaft from the right side.
Fig. 2E is a perspective view of a first shaft of the plunger as seen from the left side.
Fig. 2F is a perspective view of the first shaft as seen from the right side.
Fig. 3A is a left side view of a main part of the syringe when in a first discharge.
Fig. 3B is a cross-sectional view taken along a line IIIB-IIIIB in Fig. 3A.
Fig. 3C is a cross-sectional view taken along a line IIIC-IIIC in Fig. 3A.
Fig. 4A is a left side view of a main part of the syringe immediately before a second discharge.
Fig. 4B is a cross-sectional view of a main part taken along a line IVB-IVB in Fig. 4A.
Fig. 5A is a left side view of a main part of the syringe when in the second discharge.
Fig. 5B is a cross-sectional view taken along a line VB-VB in Fig. 5A.
Fig. 5C is a cross-sectional view taken along a line VC-VC in Fig. 5A.
Fig. 6A is a left side view of a main part of the syringe immediately before a third discharge.
Fig. 6B is a cross-sectional view taken along a line VIB-VIB in Fig. 6A.
Fig. 7A is a left side view of a main part of the syringe when in the third discharge.
Fig. 7B is a cross-sectional view taken along a line VIIB-VIIB in Fig. 7A.
Fig. 7C is a cross-sectional view taken along a line VIIC-VIIC in Fig. 7A.
Fig. 8A is a left side view of a main part of the syringe immediately before a fourth discharge.
Fig. 8B is a cross-sectional view taken along a line VIIIB-VIIIIB in Fig. 8A.
Fig. 9A is a left side view of a main part of the syringe when in the fourth discharge.
Fig. 9B is a cross-sectional view taken along a line IXB-IXB in Fig. 9A.
Fig. 10 is a perspective view of the syringe in its initial state according to a modified example.
Fig. 11A is an exploded perspective view of the syringe.
Fig. 11B is a perspective view of a second shaft of a plunger of the syringe as seen from the right side.
Fig. 11C is a perspective view of a first shaft of the plunger as seen from the right side.
Fig. 12A is a left side view of a main part of the syringe in its initial state.
Fig. 12B is a cross-sectional view of a main part taken along a line XIIB-XIIB in Fig. 12A.
Fig. 13A is a left side view of a main part of the syringe when in a first discharge.
Fig. 13B is a cross-sectional view of a main part taken along a line XIIIIB-XIIIIB in Fig. 13A.
Fig. 13C is a cross-sectional view of a main part taken along a line XIIIIC-XIIIIC in Fig. 13A.
Fig. 14A is a left side view of a main part of the syringe immediately before a second discharge.
Fig. 14B is a cross-sectional view of a main part taken along a line XIVB-XIVB in Fig. 14A.
Fig. 15A is a left side view of a main part of the syringe when in the second discharge.
Fig. 15B is a cross-sectional view of a main part taken along a line XVB-XVB in Fig. 15A.
Fig. 16 is a perspective view of the syringe in its initial state according to another modified example.
Fig. 17A is an exploded perspective view of the syringe.
Fig. 17B is a perspective view of a second shaft of a plunger of the syringe as seen from the right side.
Fig. 17C is a perspective view of a first shaft of the plunger as seen from the right side.
Fig. 18A is a left side view of a main part of the syringe in its initial state.
Fig. 18B is a cross-sectional view of a main part taken along a line XVIIIIB-XVIIIIB in Fig. 18A.
Fig. 18C is a cross-sectional view of a main part taken along a line XVIIIIC-XVIIIIC in Fig. 18A.
Fig. 19A is a left side view of a main part of the syringe when in a first discharge.
Fig. 19B is a cross-sectional view of a main part taken along a line XIXB-XIXB in Fig. 19A.
Fig. 19C is a cross-sectional view of a main part taken along a line XIXC-XIXC in Fig. 19A.
Fig. 20A is a left side view of a main part of the syringe immediately before a second discharge.
Fig. 20B is a cross-sectional view of a main part taken along a line XXB-XXB in Fig. 20A.
Fig. 20C is a cross-sectional view of a main part taken along a line XXC-XXC in Fig. 20A.
Fig. 21A is a left side view of a main part of the syringe when in the second discharge.
Fig. 21B is a cross-sectional view of a main part taken along a line XXIB-XXIB in Fig. 21A.
Fig. 21C is a cross-sectional view of a main part taken along a line XXIC-XXIC line in Fig. 21A.
Fig. 22A is a left side view of a main part of the syringe immediately before a third discharge.
Fig. 22B is a cross-sectional view of a main part taken along a line XXIIB-XXIIB in Fig. 22A.
Fig. 22C is a cross-sectional view of a main part taken along a line XXIIC-XXIIC in Fig. 22A.
Fig. 23A is a left side view of a main part of the syringe when in the third discharge.
Fig. 23B is a cross-sectional view of a main part taken along a line XXIIIIB-XXIIIIB in Fig. 23A.
Fig. 23C is a cross-sectional view of a main part taken along a line XXIIIIC-XXIIIIC in Fig. 23A.
Fig. 24 is a perspective view of the syringe in its initial state according to another modified example.
Fig. 25A is an exploded perspective view of the syringe.
Fig. 25B is a front view of a second shaft of a plunger of the syringe.
Fig. 25C is a perspective view of a second shaft as seen from the left side.
Fig. 25D is a perspective view of a first shaft of the plunger as seen from the left side.
Fig. 26A is a left side view of a main part of the syringe in its initial state.
Fig. 26B is a cross-sectional view of a main part taken along a line XXVIB-XXVIB in Fig. 26A.
Fig. 27A is a left side view of a main part of the syringe when in a first discharge.
Fig. 27B is a cross-sectional view of a main part taken along a line XXVIIB-XXVIIB in Fig. 27A.
Fig. 27C is a cross-sectional view of a main part taken along a line XXVIIC-XXVIIC in Fig. 27A.
Fig. 28A is a left side view of a main part of the syringe immediately before a second discharge.
Fig. 28B is a cross-sectional view of a main part taken along a line XXVIIIIB-XXVIIIIB in Fig. 28A.
Fig. 29A is a left side view of a main part of the syringe when in the second discharge.
Fig. 29B is a cross-sectional view of a main part taken along a line XXIXB-XXIXB in Fig. 29A.
Fig. 30 is a cross-sectional view of a main part of the syringe in its initial state according to yet another modified example.
Fig. 31 is an exploded perspective view of the syringe.
Fig. 32 is a front view of a second shaft of a plunger of the syringe.
Fig. 33 is a cross-sectional view of a conventional syringe-type injector in its initial state.
Fig. 34 is a view for explaining about a first discharge of the conventional syringe-type injector.
Fig. 35 is a view showing the state after the first discharge of the conventional syringe-type injector has been completed and the pressing-in has been weakened.

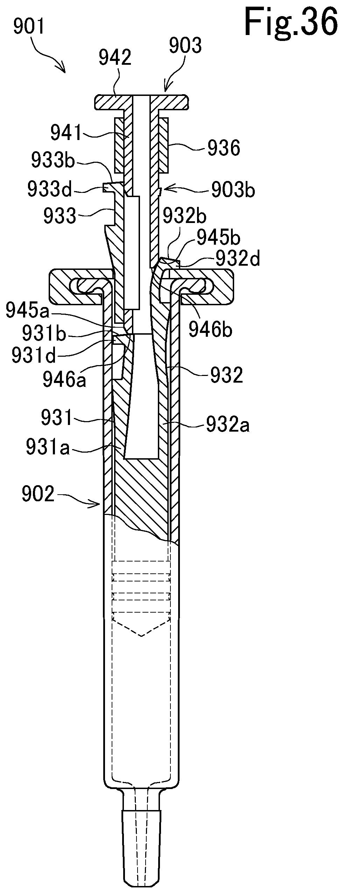

Fig. 36 is a view for explaining about a second discharge of the conventional syringe-type injector.
Fig. 37 is a view showing the state after the second discharge of the conventional syringe-type injector has been completed and the pressing-in has been weakened.

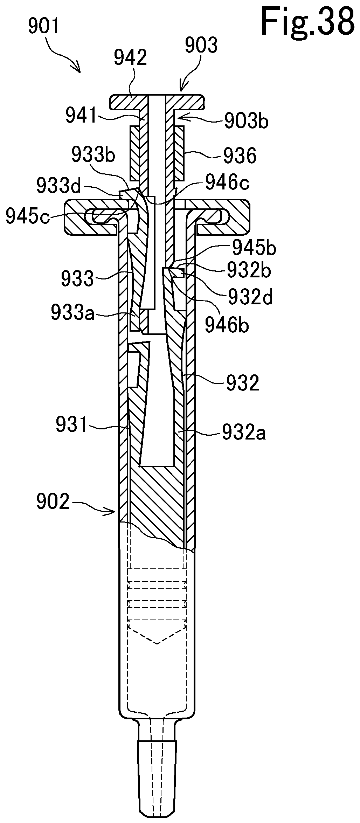

Fig. 38 is a view for explaining about a third discharge of the conventional syringe-type injector.
Fig. 39 is a view showing the state after the third discharge of the conventional syringe-type injector has been completed and the pressing-in has been weakened.
Fig. 40 is a view for explaining about a fourth discharge of the conventional syringe-type injector.

### DESCRIPTION OF EMBODIMENTS

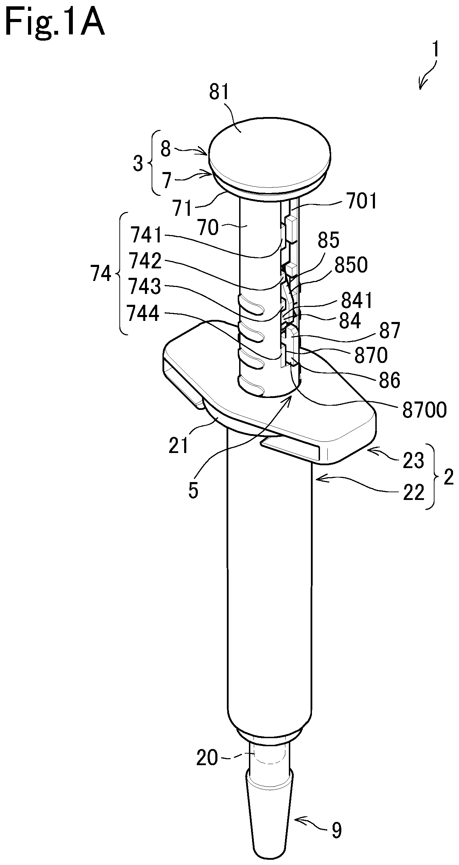

Hereinafter, a description will be hereinbelow given for a container according to an embodiment of the present invention with reference to Fig. 1A to Fig. 9B.

As shown in Fig. 1A, a syringe 1 is configured to divide a content and administer each divided portion of the content. Specifically, the syringe 1 is a nasal administration container that can divide and administer a mist of a medical liquid into both nostrils. The syringe 1 is configured to divide a medical liquid into four portions and administer them respectively, but it can also be configured to divide a medical liquid into two or three or more portions and administer them respectively. The medical liquid is in a liquid form, such as an influenza vaccine. The syringe 1 has a substantially cylindrical shape.

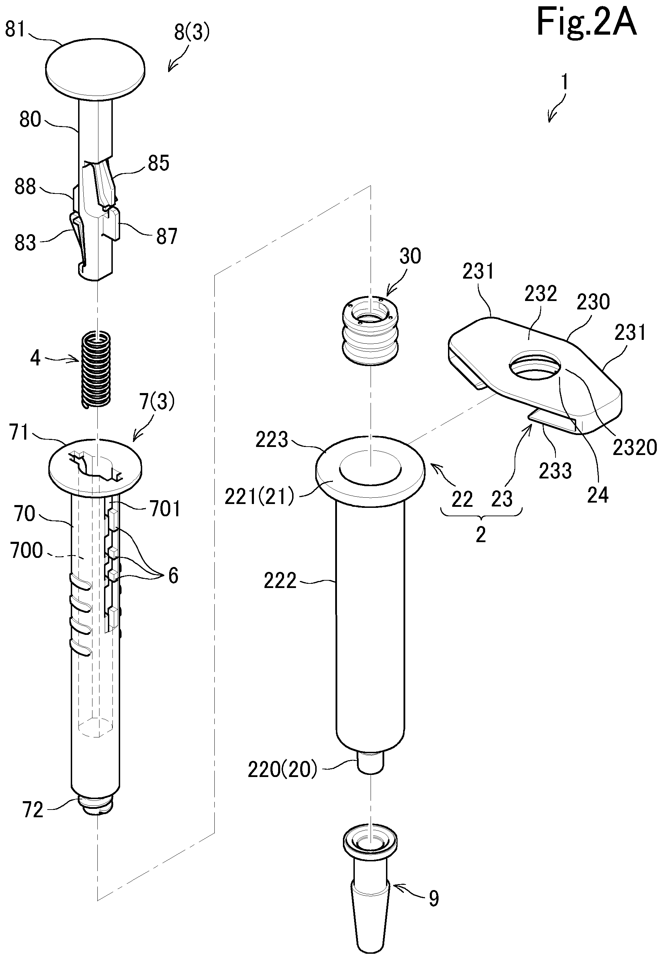

As shown in Fig. 1A and Fig. 2A, the syringe 1 includes a barrel 2 having a distal end part 20 for discharging the content and a proximal end part 21 located on the opposite side to the side of the distal end part 20 in an axial direction. The syringe 1 includes a plunger 3 and a biasing member 4. The syringe 1 includes a restriction release part 5. The syringe 1 includes a push-in guide part 6. The syringe 1 includes a discharge nozzle 9.

In the syringe 1, the side where the distal end part 20 of the barrel 2 is located (the lower side in Fig. 1A) is the "distal end side," and the side where the proximal end part 21 of the barrel 2 is located (the upper side in Fig. 1A) is the "proximal end side." The "axial direction" of the syringe 1 refers to the cylinder axial direction of the syringe 1. The radial direction of the syringe 1 also simply refers to as "radially."

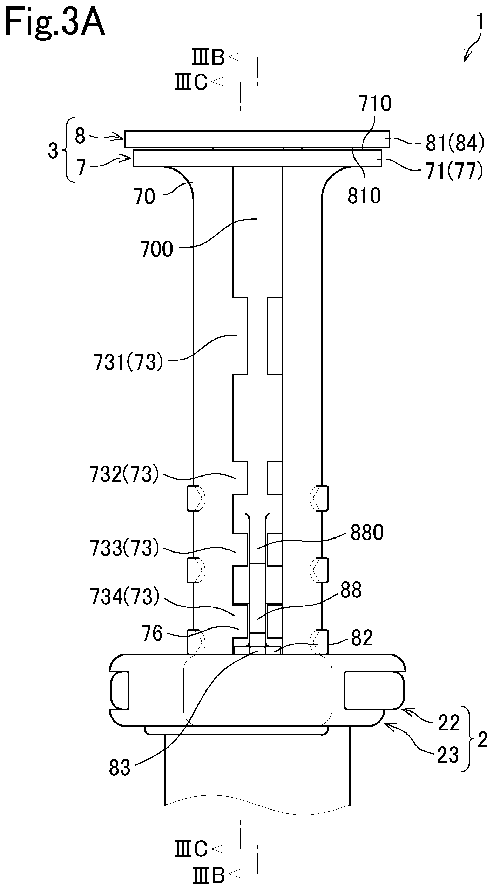

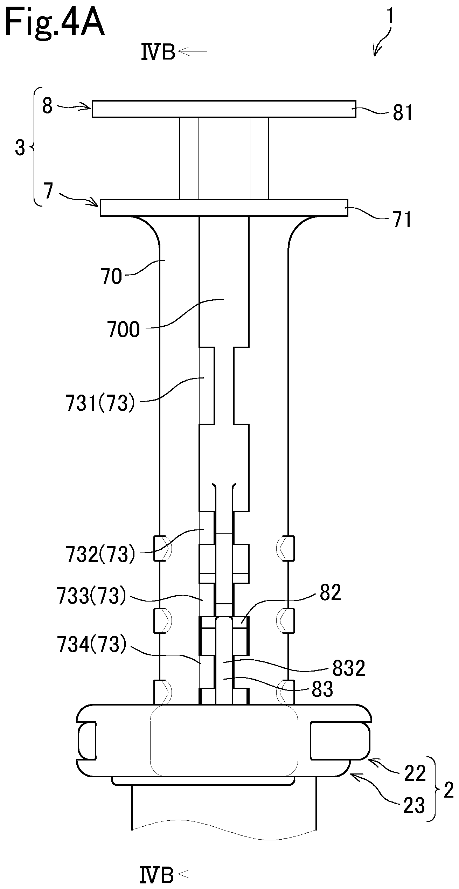

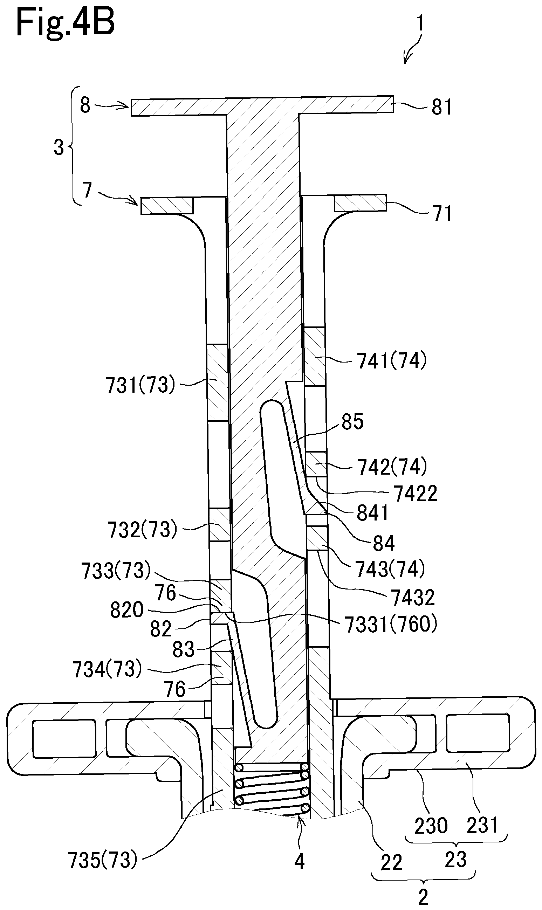

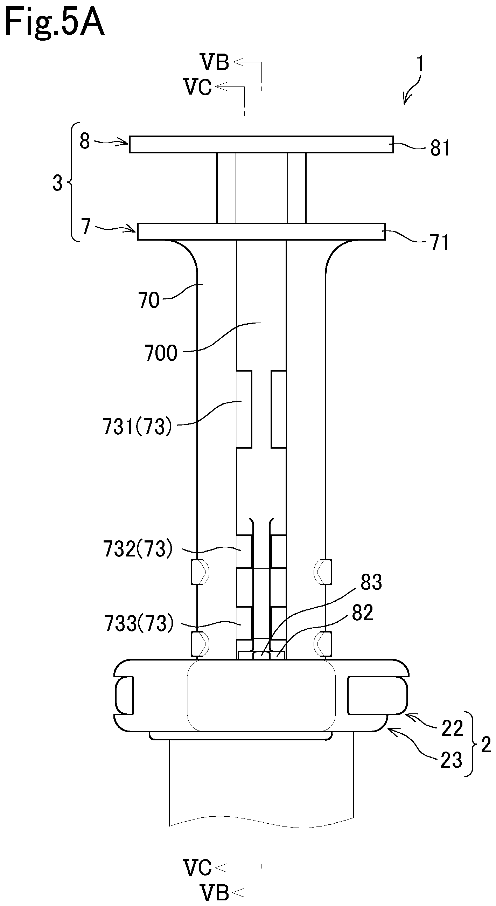

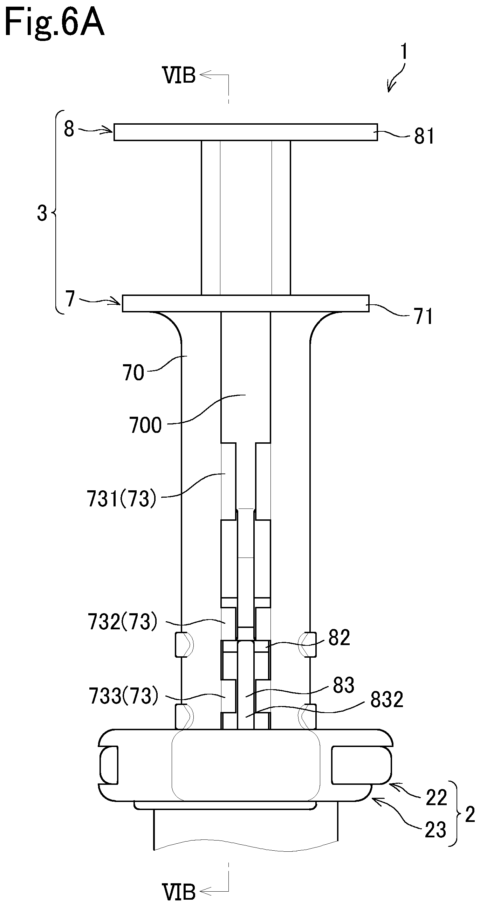

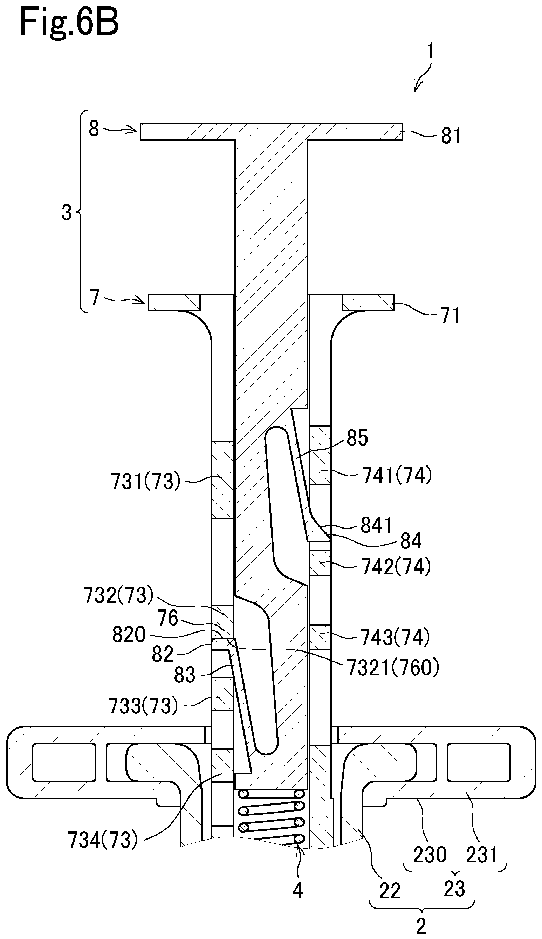

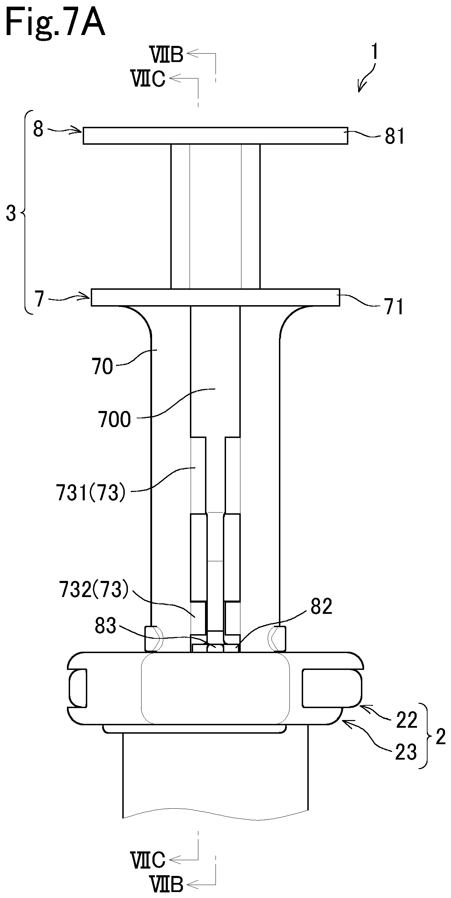

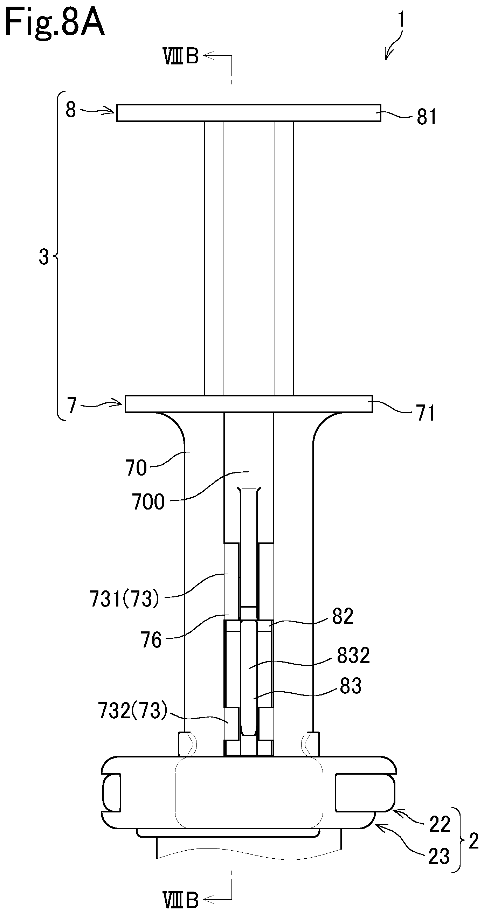

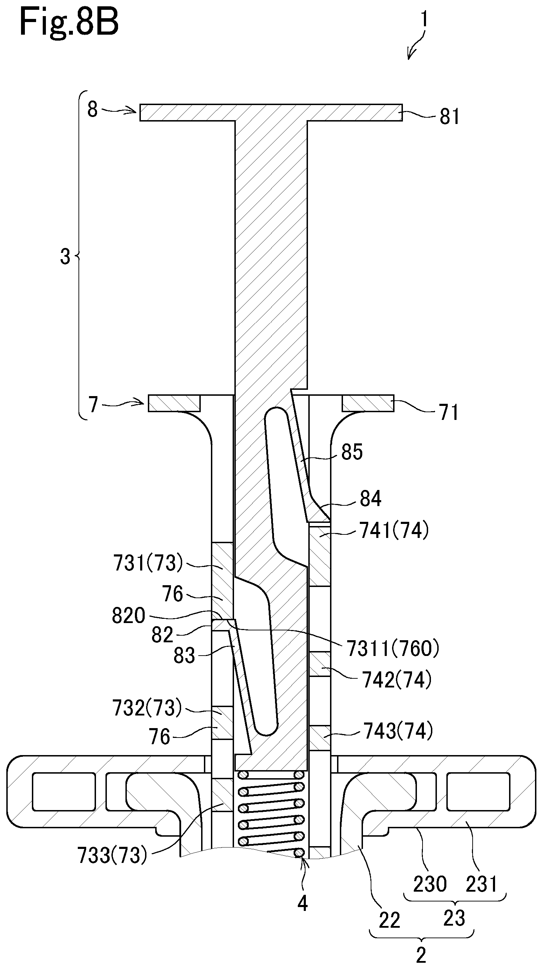

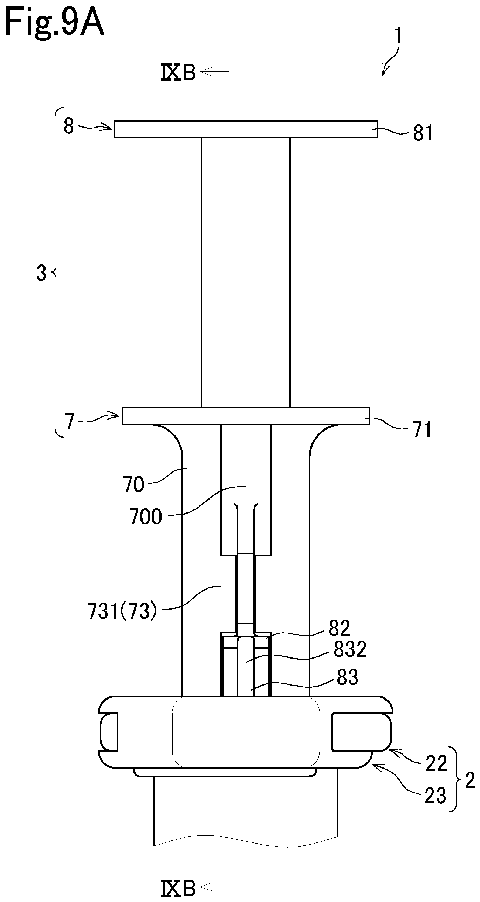

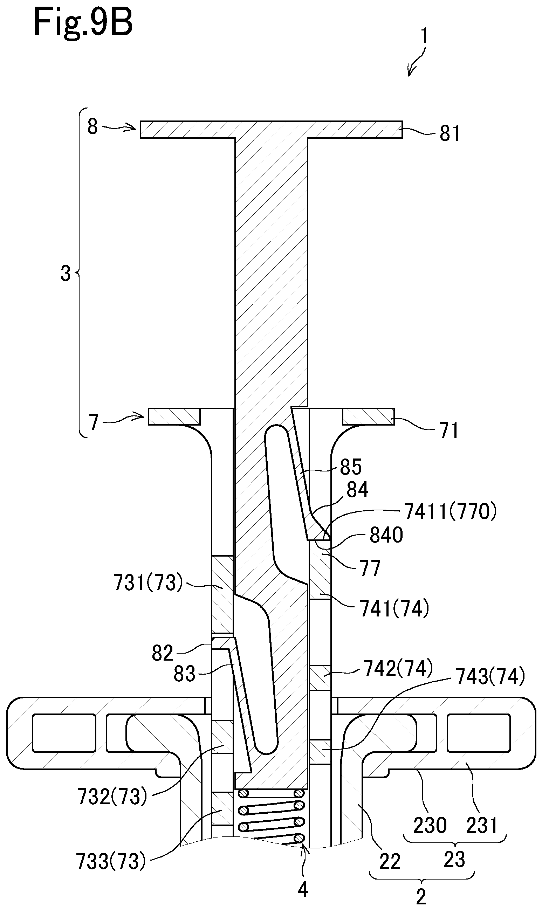

When using the syringe 1, the plunger 3 is pushed toward the distal end from the initial state (Fig. 1A to Fig. 1C) to perform a first discharge of the medical liquid (Fig. 3A to Fig. 3C). Next, after proceeding through the state immediately before a second discharge of the medical liquid (Fig. 4A and Fig. 4B), the plunger 3 is pushed toward the distal end to perform the second discharge of the medical liquid (Fig. 5A to Fig. 5C). After proceeding through the state immediately before a third discharge of the medical liquid (Fig. 6A and Fig. 6B), the plunger 3 is pushed toward the distal end to perform the third discharge of the medical liquid (Fig. 7A to Fig. 7C). After proceeding through the state immediately before a fourth discharge of the medical liquid (Fig. 8A and Fig. 8B), the plunger 3 is pushed toward the distal end to perform the fourth discharge of the medical liquid (Fig. 9A and Fig. 9B).

A description will be hereinbelow given on the components of the syringe 1.

The barrel 2 is a member that contains the medical liquid inside (see Fig. 1A). In the syringe 1 of this embodiment, the barrel 2 includes a barrel body 22 and a finger grip 23 disposed on the barrel body 22.

The barrel body 22 is a cylindrical body that contains the medical liquid. In this barrel 2, a body distal end part 220 that is the distal end part of the barrel body 22 forms the distal end part 20 of the barrel 2, and a body proximal end part 221 that is the proximal end part of the barrel body 22 forms the proximal end part 21 of the barrel 2 (see Fig. 2A). The barrel body 22 includes the body distal end part 220 and the body proximal end part 221, as well as a body connecting part 222 having a cylindrical shape that connects the body distal end part 220 and the body proximal end part 221. The barrel body 22 includes a flange part 223 that extends outward (outward in the radial direction of the barrel body 22) from the entire outer circumference of one end in the cylindrical axis direction of the body proximal end part 221.

The outer diameter of the body distal end part 220 is smaller than the outer diameter of the body connecting part 222. The outer diameter of the body connecting part 222 is substantially constant at all portions along the cylinder axis direction. The flange part 223 has an annular shape (for example, ring shape) when viewed from the cylinder axis direction.

The barrel body 22 is made of a material that is, for example, transparent and can withstand the internal pressure applied when administering the medical liquid. Specifically, the material of the barrel body 22 is, for example, glass or a resin containing cyclic olefins such as norbornene as repeating units. More specifically, the material of the barrel 2 is a transparent resin such as COP (cycloolefin polymer), which is a homopolymer of cyclic olefins, or COC (cycloolefin copolymer), which is a copolymer of cyclic olefins or ethylene, or glass.

The finger grip 23 is a part for placement of the fingers of a user when using the syringe 1. The finger grip 23 is fitted onto the body proximal end part 221 of the barrel body 22 and the flange part 223. In this embodiment, the finger grip 23 includes a grip body part 230 that covers the flange part 223, and a pair of grip extensions 231 that extend radially outward from two opposing locations of the grip body part 230.

The grip body part 230 is formed in an annular shape that surrounds the plunger 3. In this embodiment, the grip body part 230 includes a body proximal end part 232 that overlaps the proximal end side of the flange part 223 of the barrel body 22, and a body distal end part 233 that overlaps a distal end side of the flange part 223.

The finger grip 23 includes a discharge completion locking part 24 that locks to the plunger 3 when each of the first, second, and third discharges of the syringe 1 is completed. In this embodiment, a proximal end surface 2320, which is a surface facing the proximal end side of the finger grip 23, specifically a surface facing the proximal end side of the body proximal end part 232, forms the discharge completion locking part 24. A pair of the discharge completion locking parts 24 are disposed at positions opposite each other with a central axis of the finger grip 23 therebetween. Specifically, the discharge completion locking parts 24 are located opposite each other at positions 180° apart in the circumferential direction of the finger grip 23.

The plunger 3 is a member that can be pushed into the barrel 2 from the proximal end part 21 toward the distal end part 20. The plunger 3 includes a first shaft 7 and a second shaft 8 that is inserted into the first shaft 7 and is movable in the axial direction relative to the first shaft 7. In this embodiment, the plunger 3 is provided with a piston 30. In this plunger 3, the biasing member 4 is disposed at the distal end part of the second shaft 8, and the piston 30 is disposed at the distal end part of the first shaft 7. The plunger 3 is inserted into the barrel 2 with a posture at a position where the biasing member 4 is located on the proximal end side and the piston 30 is located on the distal end side.

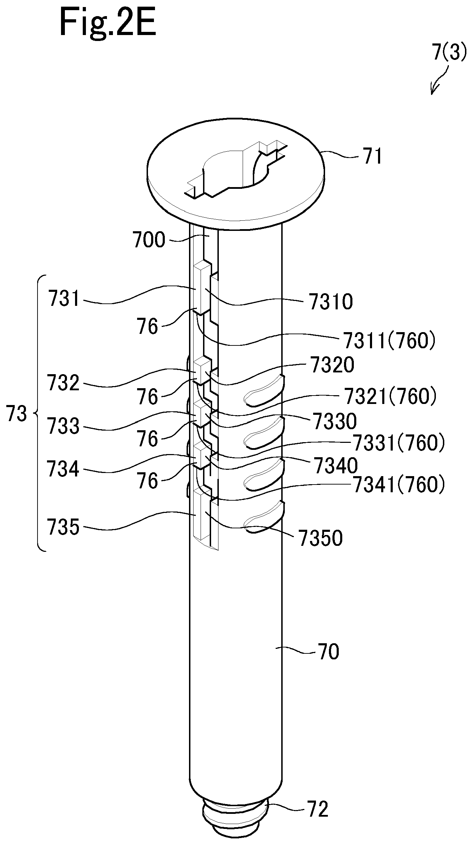

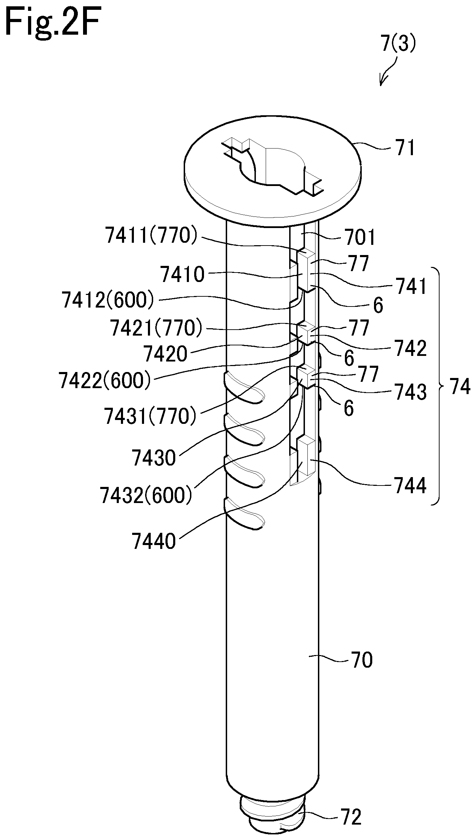

The first shaft 7 is a shaft that moves together with the second shaft 8 when discharging the medical liquid (see Fig. 1A). The first shaft 7 is a member that surrounds the second shaft 8. As shown in Fig. 2E and Fig. 2F, the first shaft 7 has a tubular shape, specifically, a substantially cylindrical shape. This first shaft 7 has an axially asymmetrical shape. The material of the first shaft 7 is a resin such as POM (polyacetal).

In this embodiment, the first shaft 7 includes a first main shaft 70 that extends in the axial direction, a first proximal end part 71 disposed at the proximal end part of the first main shaft 70, and a first distal end part 72 disposed at the distal end part of the first main shaft 70 (see Fig. 2A). The first shaft 7 includes a plurality of one-side protrusions 73 disposed on one side, which is a position in the circumferential direction of the first main shaft 70, and a plurality of other-side protrusions 74 disposed on another side, which is another position in the circumferential direction of the first main shaft 70 (see Fig. 2E and Fig. 2F). This first main shaft 70 is integrally molded with the first proximal end part 71, the first distal end part 72, and the protrusions 73 and 74. The first main shaft 70 can be molded with at least one of the first proximal end part 71, the first distal end part 72, and the protrusions 73 and 74 molded separate therefrom, or can be molded by combining a separate member.

The first main shaft 70 is a part that surrounds the second shaft 8. In this embodiment, the first main shaft 70 has a substantially cylindrical shape with a uniform diameter at all portions along the axial direction. A one-side slit 700 is formed on one side of the first main shaft 70, and an other-side slit 701 is formed on an other side of the first main shaft 70. The slits 700 and 701 are spaced apart from each other in the circumferential direction, and in this embodiment, located opposite each other with the central axis of the first main shaft 70 therebetween. Specifically, the slits 700 and 701 are located opposite each other at positions 180° apart in the circumferential direction of the first main shaft 70.

At positions adjacent to the slits 700 and 701 in the circumferential direction of the first main shaft 70, protrusions and recesses are formed on the outer surface to accumulate pressure when in the discharge of the medical liquid, that is, to create a force that generates the spray of the medical liquid. In this embodiment, the first main shaft 70 has a plurality of protrusions that extend in the circumferential direction and are arranged in the axial direction. The first main shaft 70 does not necessarily have such protrusions and recesses.

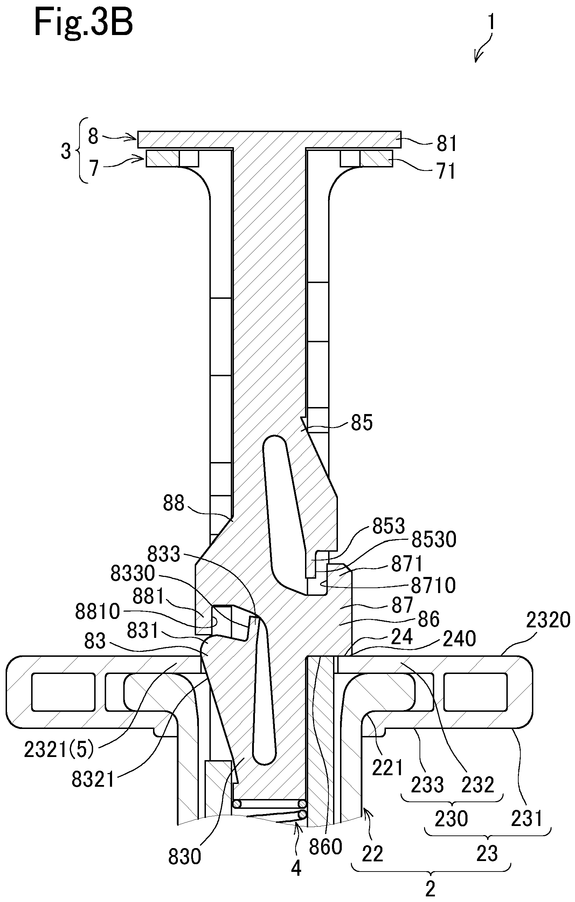

The slits 700 and 701 each extend linearly along the axial direction. As shown in Fig. 3B, a portion of the second shaft 8 protrudes radially outward from the first main shaft 70 through the slits 700 and 701.

Five sets of the one-side protrusions 73 are arranged at intervals in the axial direction of the first main shaft 70, while those of each set are located with the one-side slit 700 therebetween (see Fig. 2E). Each of the protrusions 731, 732, 733, 734, and 735 is provided in pairs, with the one-side slit 700 therebetween. The one-side protrusion 73 guides a portion of the second shaft 8 that protrudes radially outward through the one-side slit 700. The pair of the one-side protrusions 73 that have the one-side slit 700 therebetween protrude from the first main shaft 70 by the same dimension along the circumferential direction.

A description will be hereinbelow given on the detailed configuration of each of the one-side protrusions 73. Each of the one-side protrusions 73 guides the axial movement of the second shaft 8 relative to the first shaft 7 when the second shaft 8 moves relative to the first shaft 7 by the biasing force of the biasing member 4. The one-side protrusion 73 restricts the circumferential movement of the second shaft 8 in the state where the movement of the second shaft 8 relative to the first shaft 7 by the biasing force of the biasing member 4 is restricted. Specifically, the protrusions 731, 732, 733, 734, and 735 respectively have guide surfaces 7310, 7320, 7330, 7340, and 7350 facing the one-side slit 700 side, which abut against a portion of the second shaft 8, thereby guiding the axial movement of the second shaft 8 and restricting the circumferential movement of the second shaft 8. The guide surfaces 7310, 7320, 7330, 7340, and 7350 of the protrusion 73 are flat surfaces.

In this embodiment, the protrusions 731 on the most proximal side and the protrusions 735 on the most distal side have larger axial dimensions than the protrusions 732, 733, and 734 located therebetween.

Of the one-side protrusions 73, the protrusions 731, 732, 733, and 734 each, excluding the protrusions 735 on the most distal side in the axial direction, include a restriction locking part 76 that restricts the movement of the second shaft 8 relative to the first shaft 7 by the biasing force of the biasing member 4. In this embodiment, the distal end parts of the protrusions 731, 732, 733, and 734 each form the restriction locking part 76. The distal end surfaces 7311, 7321, 7331, and 7341, which are the surfaces facing the distal end side of the protrusions 731, 732, 733, and 734, each form the distal end surface 760 of the restriction locking part 76. The restriction locking parts 76 restrict the movement of the second shaft 8 relative to the first shaft 7 by the biasing force, by having their distal end surfaces 760 abutting against a portion of the second shaft 8. The distal end surfaces 760 of the restriction locking parts 76 are flat surfaces.

A plurality of the restriction locking parts 76 are arranged at intervals in the axial direction, and in this embodiment, four sets are provided. Specifically, the restriction locking parts 76 are composed of a plurality of main-shaft restriction locking parts 76 disposed on the first main shaft 70 at intervals in the axial direction, and in this embodiment, they are composed of four sets of the main-shaft restriction locking parts 76 arranged in the axial direction.

Four sets of the other-side protrusions 74 are arranged at intervals in the axial direction of the first main shaft 70, while those of each set are located with the other-side slit 701 therebetween (see Fig. 2F). Specifically, protrusions 741, 742, 743, and 744 are each provided in pairs, with the other-side slit 701 therebetween. The other-side protrusions 74 guide a portion of the second shaft 8 that protrudes radially outward from the other-side slit 701. A pair of the other-side protrusions 74 that have the other-side slit 701 therebetween protrude from the first main shaft 70 by the same dimension along the circumferential direction.

The description will be hereinbelow given on the detailed configuration of each of the other-side protrusions 74. The other-side protrusions 74 each guide the axial movement of the second shaft 8 relative to the first shaft 7 when the second shaft 8 moves relative to the first shaft 7 by the biasing force of the biasing member 4. The other-side protrusions 74 restrict the circumferential movement of the second shaft 8 in the state where the movement of the second shaft 8 relative to the first shaft 7 by the biasing force of the biasing member 4 is restricted. Specifically, the protrusions 741, 742, 743, and 744 respectively having guide surfaces 7410, 7420, 7430, and 7440 facing the other-side slit 701 side abut against a portion of the second shaft 8, thereby guiding the axial movement of the second shaft 8 and restricting the circumferential movement of the second shaft 8. The guide surfaces 7410, 7420, 7430, and 7440 of the protrusions 74 are flat surfaces.

Of the other-side protrusions 74, the protrusions 741, 742, and 743 each, excluding the protrusions 744 on the most distal side, include a push-in locking part 77, to which the second shaft 8 is locked, when the plunger 3 is pushed into the barrel 2. In this embodiment, the proximal end parts of the protrusions 741, 742, and 743 each form the push-in locking part 77. Proximal end surfaces 7411, 7421, and 7431, which are surfaces of the protrusions 741, 742, and 743 facing the proximal end side, form proximal end surfaces 770 of the push-in locking parts 77. The proximal end surfaces 770 of the push-in locking parts 77 each abut against a portion of the second shaft 8, thereby restricting the axial movement of the second shaft 8 relative to the first shaft 7 toward the distal end, and enabling the plunger 3 to be pushed into the barrel 2. The proximal end surfaces 770 of the push-in locking parts 77 are flat surfaces.

In this embodiment, the protrusions 741 on the most proximal side and the protrusions 744 on the most distal side have larger axial dimensions than the protrusions 742 and 743 located therebetween.

A plurality of the push-in locking parts 77 are arranged at intervals in the axial direction, and in this embodiment, three sets are provided. The push-in locking parts 77 include a plurality of main-shaft push-in locking parts 77 disposed on the first main shaft 70 at intervals in the axial direction, and in this embodiment, they are composed of three sets of the main-shaft push-in locking parts 77 arranged in the axial direction.

In this embodiment, the protrusions 741, 742, and 743 each are provided with a push-in guide part 6 that guides a portion of the second shaft 8 from a push-in implementing position to a push-in withdrawn position between two axially adjacent ones of the main-shaft push-in locking parts 77. Distal end parts of the protrusions 741, 742, and 743 each form the push-in guide part 6. Distal end surfaces 7412, 7422, and 7432, which are surfaces facing the distal end sides of the protrusions 741, 742, and 743, form distal end surfaces 600 of the push-in guide parts 6.

The push-in guide parts 6 each guide a portion of the second shaft 8 from a push-in implementing position to a push-in withdrawn position by having its distal end surface 600 abutting against the portion of the second shaft 8. The push-in guide part 6 retains the push-in part 85 in the push-in withdrawn position when the push-in part 85 moves along the axial direction from the main-shaft push-in locking part 77 on the distal end side to the main-shaft push-in locking part 77 on the proximal end side, of the two main-shaft push-in locking parts 77. When the push-in part 85 is positioned on the proximal end side relative to the main-shaft push-in locking part 77 on the proximal end side, the push-in guide part 6 releases the retaining and allows the push-in part 85 to move to the push-in implementing position.

A plurality of the push-in guide parts 6 are arranged at intervals in the axial direction, and in this embodiment, three sets are provided. In this embodiment, the push-in guide part 6 includes a plurality of main-shaft push-in guide parts 6 disposed on the first main shaft 70 at intervals in the axial direction, and in this embodiment, the push-in guide part 6 is composed of three sets of the main-shaft press-in guide parts 6 arranged in the axial direction.

The first proximal end part 71 is a part that overlaps with a second proximal end part 81 of the second shaft 8. In this embodiment, the first proximal end part 71 extends radially outward from the entire outer circumference of the proximal end part of the first main shaft 70. The first proximal end part 71 has an annular shape (for example, ring shape) when viewed from the axial direction. In this syringe 1, when in the first discharge of the medical liquid, the first proximal end part 71 forms the push-in locking part 77 that locks to a portion of the second proximal end part 81.

The first distal end part 72 is a part to which the piston 30 is attached. In this embodiment, the first distal end part 72 has a substantially cylindrical shape with a male screw on its outer circumferential surface.

The second shaft 8 is a shaft that is operated when discharging the medical liquid (see Fig. 2A). The second shaft 8 is a member disposed within the first shaft 7. The second shaft 8 is biased toward the proximal end by the biasing force of the biasing member 4. This second shaft 8 has an asymmetrical shape. The material of the second shaft 8 is a resin such as POM (polyacetal). In this embodiment, the materials of the first shaft 7 and the second shaft 8 are the same as each other, but they can be different from each other.

In this embodiment, the second shaft 8, as shown in Fig. 2A to Fig. 2C, includes a second main shaft 80 that extends along the axial direction and a second proximal end part 81 that is disposed at the proximal end part of the second main shaft 80. The second shaft 8 includes a restriction part 83 having a starting end 830 on the distal end side and a free end 831 on the proximal end side. The restriction part 83 is configured to be pivotally movable about the starting end 830 between a restriction withdrawn position where the free end 831 is located radially inward and a restriction implementing position where the free end 831 is located radially outward. The second shaft 8 includes a push-in part 85 that includes a push-in locked part 84 that is locked to the push-in locking part 77 of the first shaft 7 when the plunger 3 is pushed in.

In this second shaft 8, a restriction part 83 is disposed on one side in the circumferential direction, and a push-in part 85 is disposed on another side in the circumferential direction. Specifically, in this second shaft 8, the restriction part 83 and the push-in part 85 are disposed in positions opposite each other with the central axis therebetween. More specifically, the restriction part 83 and the push-in part 85 are located opposite each other at positions 180° apart in the circumferential direction of the second shaft 8.

The second shaft 8 includes a discharge completion part 87 that includes a discharge completion locked part 86 that is locked to the discharge completion locking part 24 when the discharge of the medical liquid is completed. The second shaft 8 includes a projection part 88.

In this second shaft 8, the projection part 88 is disposed on one side in the circumferential direction, and the discharge completion part 87 is disposed on another side in the circumferential direction. In this second shaft 8, the projection part 88 is aligned with the restriction part 83 on the proximal end side in the axial direction. The discharge completion part 87 is aligned with the push-in part 85 on the distal end side in the axial direction. In this embodiment, the second main shaft 80 is integrally molded with the second proximal end part 81, the restriction part 83, the push-in part 85, the discharge completion part 87, and the projection part 88. At least one of these members can be molded separate therefrom, or can be molded by combining another separate member.

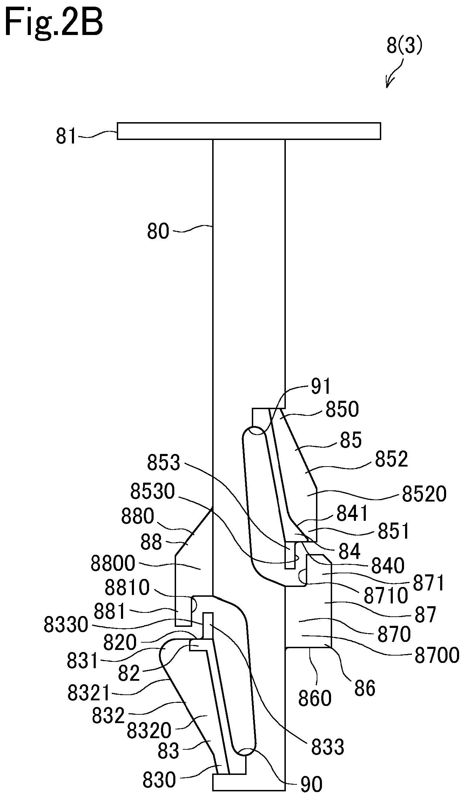

The second main shaft 80 is a part that is fitted within the first main shaft 70 (see Fig. 2A). In this embodiment, the second main shaft 80 has a substantially cylindrical shape and has a recess 90, in which the restriction part 83, when in the restriction withdrawn position, is fitted (see Fig. 2B). The second main shaft 80 is provided with a recess 91, in which the push-in part 85, when in the push-in withdrawn position, is fitted, in addition to the recess 90, in which the restriction part 83 is fitted. The distal end part of the second main shaft 80 abuts against the biasing member 4.

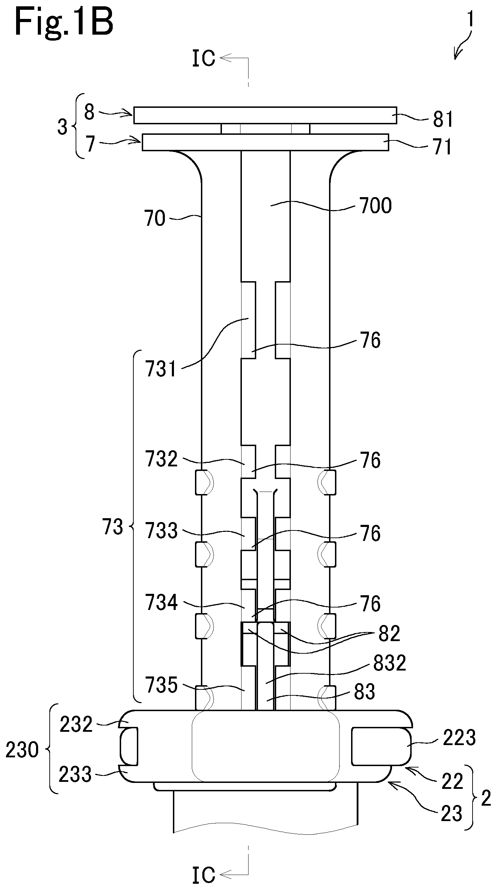

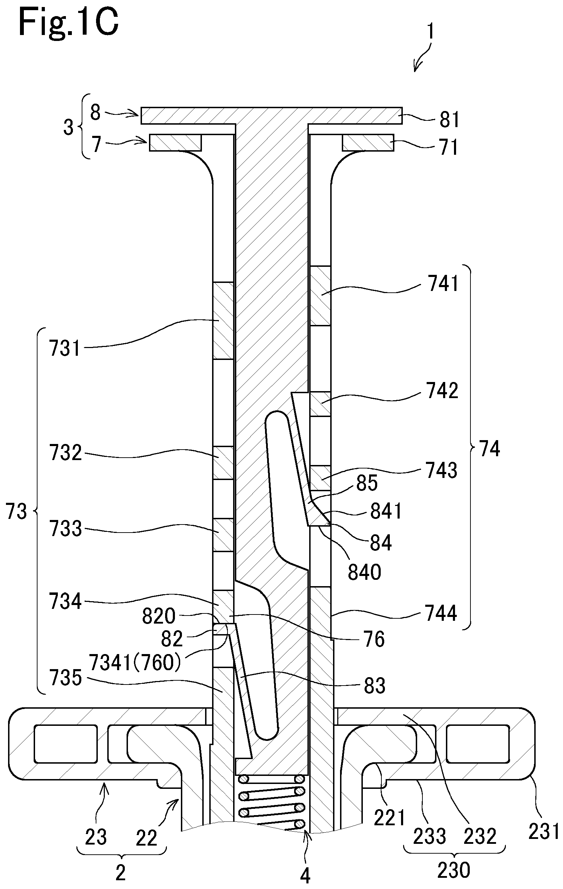

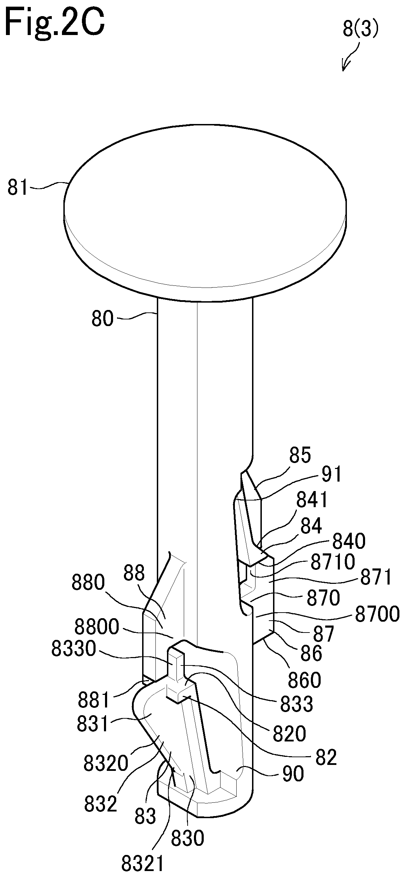

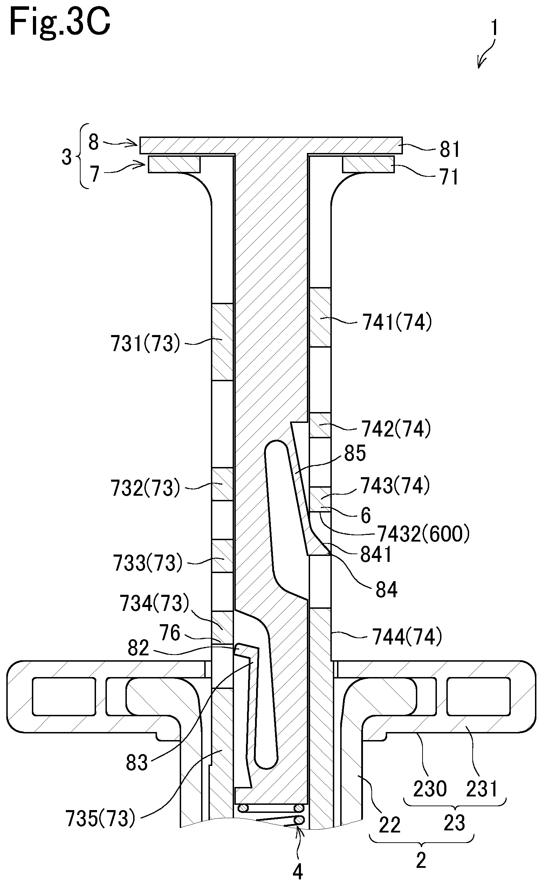

The restriction part 83 is a stopper that prevents the second shaft 8 from moving relative to the first shaft 7 by the biasing force of the biasing member 4. An end surface 8321 of the restriction part 83 on the radially outer side is an inclined surface that radially outwardly expands toward the proximal end to be located closer to the radially inner side as it advances toward the distal end, and to be located closer to the radially outer side as it advances toward the proximal end. This end surface 8321 is a surface that is pressed in by the restriction release part 5 at the position where one instance of the aforementioned pressing-in has been completed. The restriction part 83 includes a restriction locked part 82 at the free end 831 located on the proximal end side, which is locked to the restriction locking part 76 of the first shaft 7 by the biasing force of the biasing member 4 in the restriction implementing position. That is, the proximal end part of the restriction part 83 forms the restriction locked part 82 (see Fig. 2B and Fig. 2C). When the restriction part 83 is in the restriction implementing position, the restriction locked part 82 is locked to the restriction locking part 76 (see Fig. 1B and Fig. 1C). When the restriction part 83 is in the restriction withdrawn position, the restriction locked part 82 is not locked to the restriction locking part 76 (see Fig. 3A and Fig. 3C). The restriction part 83 is disposed on the second main shaft 80 (see Fig. 2B and Fig. 2C).

In this embodiment, a pair of the restriction locked parts 82 are provided with a gap therebetween in the circumferential direction of the second main shaft 80 (see Fig. 1B). The surface of the restriction part 83 facing the proximal end side, specifically the proximal end surface 820 of the restriction locked part 82, abuts against the distal end surface 760 of the restriction locking part 76 of the first shaft 7, thereby restricting the movement of the second shaft 8 relative to the first shaft 7 by a biasing force (see Fig. 1C). The proximal end surface 820 of the restriction locked part 82 is a flat surface.

The restriction part 83 includes a restriction guided part 832 disposed between the pair of the restriction locked parts 82 (see Fig. 1B). The restriction part 83 includes a restriction extension part 833 that extends further toward the proximal end from the end part on the radially inner side of the restriction locked part 82 (see Fig. 2B and Fig. 2C).

The restriction guided part 832 is a part that guides the axial movement of the second shaft 8 relative to the first shaft 7 when the second shaft 8 moves relative to the first shaft 7 by the biasing force of the biasing member 4. The restriction guided part 832 is a part that restricts the circumferential movement of the second shaft 8 in the state where the movement of the second shaft 8 relative to the first shaft 7 by the biasing force of the biasing member 4 is restricted.

In this embodiment, the restriction guided part 832 abuts against the one-side protrusions 732, 733, and 734 of the first shaft 7, thereby guiding the axial movement of the second shaft 8 and restricting the circumferential movement of the second shaft 8. Specifically, an outer surface 8320 of the restriction guided part 832, which faces in the circumferential direction of the second shaft 8, abuts against the guide surfaces 7320, 7330, and 7340 of the protrusions 732, 733, and 734, which face the one-side slit 700 side, thereby guiding the axial movement of the second shaft 8 and restricting the circumferential movement of the second shaft 8. The outer surface 8320 of the restriction guided part 832 is a flat surface. The end surface 8321 on the radially outer side of the restriction guided part 832 forms the end surface on the radially outer side of the restriction part 83. The end surface 8321 on the radially outer side of the restriction guided part 832 is a flat inclined surface.

The restriction extension part 833 is a stopper that prevents the restriction part 83 from opening radially outward when the second shaft 8 moves relative to the first shaft 7 by the biasing force of the biasing member 4, and when the movement of the second shaft 8 relative to the first shaft 7 is restricted. The restriction extension part 833 is located radially inward of a portion of the projection part 88. In this embodiment, the restriction extension part 833 is a rib that extends axially from the end part on the radially inner side of the restriction locked part 82 toward the proximal end. The outer surface 8330 located radially outward of the restriction extension part 833 is a flat surface.

The push-in part 85 is disposed on the second main shaft 80. The push-in part 85 has a starting end 850 on the proximal end side and a free end 851 on the distal end side. The push-in part 85 is configured to be pivotally movable about the starting end 850 as a starting point between a push-in withdrawn position where the free end 851 is located radially inward and a push-in implementing position where the free end 851 is located radially outward. The end surface on the radially outer side of the push-in part 85 is an inclined surface that is located closer to the radially inner side as it advances toward the proximal end and is located closer to the radially outer side as it advances toward the distal end.

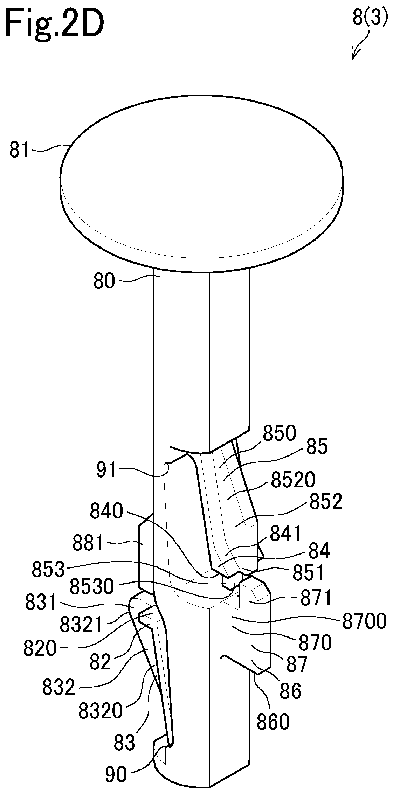

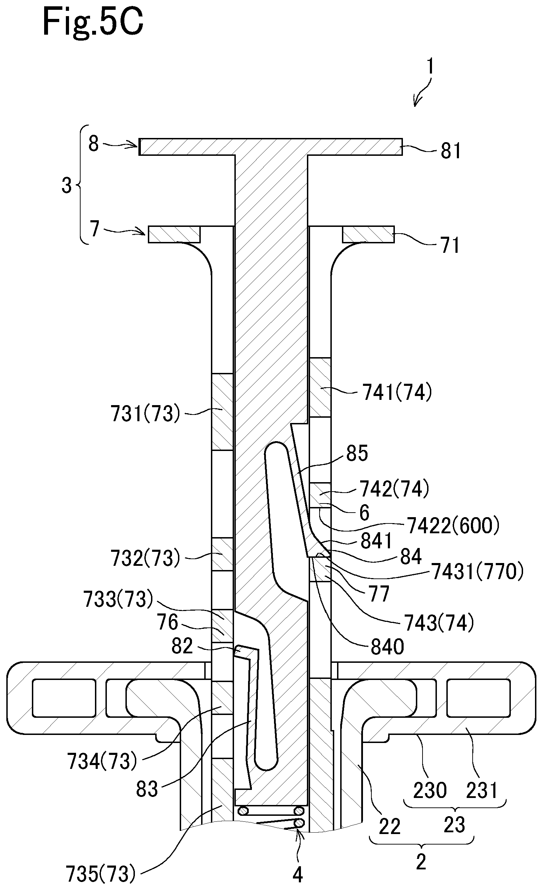

In this embodiment, the distal end part of the push-in part 85 forms the push-in locked part 84. The push-in locked part 84 is a main-shaft push-in locked part 84 disposed at the free end 851 of the push-in part 85, and is composed of a main-shaft push-in locked part 84 that is locked to the main-shaft push-in locking part 77 of the first shaft 7 when in the push-in in the push-in implementing position. A pair of the push-in locked parts 84 are disposed with a gap therebetween in the circumferential direction of the second main shaft 80 (see Fig. 2D). When the plunger 3 is pushed in, the surface of the push-in part 85 facing the distal end side, specifically the distal end surface 840 of the push-in locked part 84, abuts against the proximal end surface 770 of the push-in locking part 77 of the first shaft 7, so that the force causing the movement of the second shaft 8 toward the distal end is transmitted to the first shaft 7, causing the first shaft 7 to move together with the second shaft 8 (see Fig. 5C). The distal end surface 840 of the push-in locked part 84 is a flat surface.

The proximal end surface 841, which is a surface facing the proximal end side of the push-in locked part 84, is an inclined surface that is located closer to the radially inner side as it advances toward the proximal end (see Fig. 2B). When the second shaft 8 moves toward the proximal end relative to the first shaft 7 by the biasing force of the biasing member 4, the proximal end surface 841 of the push-in locked part 84 abuts against the distal end surfaces 7412, 7422, and 7432, which are surfaces facing the distal end sides of the other-side protrusions 741, 742, and 743 of the first shaft 7, so that the push-in part 85 moves from the push-in implementing position to the push-in withdrawn position (see Fig. 3C, Fig. 5C, and Fig. 7C). The proximal end surface 841 of the push-in locked part 84 is a flat inclined surface.

The push-in part 85 includes a push-in guided part 852 disposed between the pair of the push-in locked parts 84 (see Fig. 2B). The push-in part 85 includes a push-in extension part 853 that extends further toward the distal end from the end part on the radially inner side of the push-in locked part 84.

The push-in guided part 852 is a part that guides the axial movement of the second shaft 8 relative to the first shaft 7 when the second shaft 8 moves relative to the first shaft 7 by the biasing force of the biasing member 4. The push-in guided part 852 is a part that restricts the circumferential movement of the second shaft 8 in the state where the movement of the second shaft 8 relative to the first shaft 7 by the biasing force of the biasing member 4 is restricted.

In this embodiment, the push-in guided part 852 abuts against the other-side protrusions 741, 742, and 743 of the first shaft 7, so that the axial movement of the second shaft 8 is guided and the circumferential movement of the second shaft 8 is restricted. Specifically, the outer surface 8520 of the push-in guided part 852, which faces in the circumferential direction of the second shaft 8, abuts against the guide surfaces 7410, 7420, and 7430 of the protrusions 741, 742, and 743, which face the other-side slit 701 side, thereby guiding the axial movement of the second shaft 8 and restricting the circumferential movement of the second shaft 8. The outer surface 8520 of the push-in guided part 852 is a flat surface.

The push-in extension part 853 is a stopper that prevents the push-in part 85 from opening radially outward when the second shaft 8 moves relative to the first shaft 7 by the biasing force of the biasing member 4, and when the user of the syringe 1 moves the second shaft 8 toward the distal end. In this embodiment, the push-in extension part 853 is a rib that extends axially toward the distal end from the end part on the radially inner side of the push-in locked part 84. The push-in extension part 853 is located radially inward of a portion of the discharge completion part 87. A surface 8530 located radially outward of the push-in extension part 853 is a flat surface.

The discharge completion part 87 is a stopper that restricts the plunger 3, which has moved a distance corresponding to one discharge, from moving by being locked to the discharge completion locking part 24 of the barrel 2 (see Fig. 2B to Fig. 2D). In this embodiment, the discharge completion part 87 is locked to the discharge completion locking part 24 when each discharge of the medical liquid, excluding the last discharge, is completed. The discharge completion part 87 is disposed on the second main shaft 80.

In this embodiment, the distal end part of the discharge completion part 87 forms the discharge completion locked part 86. One discharge completion locked part 86 is disposed on the second main shaft 80. When a surface of the discharge completion part 87, which faces the distal end side, specifically a distal end surface 860 of the discharge completion locked part 86, abuts against a proximal end surface 240 of the discharge completion locking part 24 of the barrel 2, the movement of the second shaft 8 toward the distal end by the user of the syringe 1, that is, one discharge of the medical liquid is completed. The distal end surface 860 of the discharge locked part 86 is a flat surface.

The discharge completion part 87 includes a discharge guided part 870, which is an end part on the radially outer side (see Fig. 2B to Fig. 2C). The discharge completion part 87 includes a discharge extension part 871 that extends as an end part on the proximal side of the discharge guided part 870 (see Fig. 2B).

The discharge guided part 870 is a part that guides the axial movement of the second shaft 8 relative to the first shaft 7 when the second shaft 8 moves relative to the first shaft 7 by the biasing force of the biasing member 4. The discharge guided part 870 is a part that restricts the circumferential movement of the second shaft 8 in the state where the movement of the second shaft 8 relative to the first shaft 7 by the biasing force of the biasing member 4 is restricted.

In this embodiment, the discharge guided part 870 abuts against the other-side protrusions 742, 743, and 744 of the first shaft 7, thereby guiding the axial movement of the second shaft 8 and restricting the circumferential movement of the second shaft 8. Specifically, an outer surface 8700 of the discharge guided part 870, which faces in the circumferential direction of the second shaft 8, abuts against the guide surfaces 7420, 7430, and 7440 of the protrusions 742, 743, and 744, which face the other-side slit 701 side, thereby guiding the axial movement of the second shaft 8 and restricting the circumferential movement of the second shaft 8. The outer surface 8700 of the discharge guided part 870 is a flat surface.

The discharge extension part 871 is a stopper retaining part that presses the push-in extension part 853 of the push-in part 85 in order to prevent the push-in part 85 from opening radially outward when the second shaft 8 moves relative to the first shaft 7 by the biasing force of the biasing member 4, and when the user of the syringe 1 moves the second shaft 8 toward the distal end side. The discharge extension part 871 is located radially outward of the push-in extension part 853. In this embodiment, the discharge extension part 871 is a rib that extends axially toward the proximal end as an end part on the proximal side of the discharge guided part 870. An inner surface 8710 on the radially inner side of the discharge extension part 871 is a flat surface. The inner surface 8710 of the discharge extension part 871 abuts against the outer surface 8530 on the radially outer side of the push-in extension part 853, thereby preventing the push-in part 85 from opening radially outward (see Fig. 3B, Fig. 5B, and Fig. 7B).

The projection part 88 is disposed on the second main shaft 80. The projection part 88 is located closer to the proximal end than the restriction part 83 (see Fig. 2B). The projection part 88 includes a projection guided part 880, which is an end part on the radially outer side. The projection part 88 includes a projection extension part 881 that extends as an end part on the distal side of the projection guided part 880.

The projection guided part 880 is a part that guides the axial movement of the second shaft 8 relative to the first shaft 7 when the second shaft 8 moves relative to the first shaft 7 by the biasing force of the biasing member 4. The projection guided part 880 is a part that restricts the circumferential movement of the second shaft 8 in the state where the movement of the second shaft 8 relative to the first shaft 7 by the biasing force of the biasing member 4 is restricted.

In this embodiment, the projection guided part 880 abuts against the protrusions 731, 732, and 733 of the first shaft 7, thereby guiding the axial movement of the second shaft 8 and restricting the circumferential movement of the second shaft 8. Specifically, an outer surface 8800 of the projection guided part 880, which faces in the circumferential direction of the second shaft 8, abuts against the guide surfaces 7310, 7320, and 7330 of the protrusions 731, 732, and 733, which face the one-side slit 700 side, thereby guiding the axial movement of the second shaft 8 and restricting the circumferential movement of the second shaft 8. The outer surface 8800 is a flat surface.

The projection extension part 881 is a stopper retaining part that presses the restriction extension part 833 of the restriction part 83 in order to prevent the restriction part 83 from opening radially outward when the second shaft 8 moves relative to the first shaft 7 by the biasing force of the biasing member 4, and when the movement of the second shaft 8 relative to the first shaft 7 is restricted. The projection extension part 881 is located radially outward of the restriction extension part 833. In this embodiment, the projection extension part 881 is a rib that extends axially toward the distal end as an end part on the distal side of the projection guided part 880. An inner surface 8810 on the radially inner side of the projection extension part 881 is a flat surface. The inner surface 8810 of the projection extension part 881 abuts against the outer surface 8330 on the radially outer side of the restriction extension part 833, thereby preventing the restriction part 83 from opening radially outward (see Fig. 3B, Fig. 5B, and Fig. 7B).

The second proximal end part 81 is a part that overlaps with the first proximal end part 71 of the first shaft 7. In this embodiment, the second proximal end part 81 extends radially outward from the entire outer circumference of the proximal end part of the second main shaft 80. The second proximal end part 81 has a plate shape (for example, a disc shape) when viewed from the axial direction. In this syringe 1, when in the first discharge of the medical liquid, the second proximal end part 81 forms a push-in locked part 84 that is locked to the first proximal end part 71.

The biasing member 4 is an elastic member that is disposed inside the first shaft 7 together with the distal end part of the second shaft 8. The biasing member 4 biases the second shaft 8 toward the proximal end side relative to the first shaft 7.

In this embodiment, the biasing member 4 is, for example, a spring (specifically, a metal spring) (see Fig. 2A). Specifically, the biasing member 4 is a compression spring.

The biasing member 4 can be a material other than a metal spring, such as a resin spring. If the syringe 1 is equipped with a resin spring as the biasing member 4, the resin spring and the first shaft 7 can be integrally molded, or the resin spring and the second shaft 8 can be integrally molded.

When the pushing-in for one separate portion is completed, the restriction release part 5 moves the restriction part 83 from the restriction implementing position to the restriction withdrawn position, thereby releasing the restriction locked part 82 from the locking engagement with the restriction locking part 76 (see Fig. 3B, Fig. 5B, and Fig. 7B). In this embodiment, the restriction release part 5 is disposed on the barrel 2 and, specifically, is composed of a portion of the finger grip 23. More specifically, the restriction release part 5 is composed of an end part 2321 on the radially inner side of the grip body part 230 of the finger grip 23, for example, the end part 2321 on the radially inner side of the body proximal end part 232 of the grip body part 230. This end part 2321 abuts against the end surface 8321 on the radially outer side of the restriction part 83, moving the restriction part 83 from the restriction implementing position (a position on the radially outer side) to the restriction withdrawn position (a position on the radially inner side). The restriction release part 5 can be formed by a part of the barrel body 22.

The push-in guide part 6 guides the push-in part 85 of the second shaft 8 from the push-in implementing position to the push-in withdrawn position between the two main-shaft push-in locking parts 77 that are adjacent to each other in the axial direction of the first shaft 7. In this embodiment, as described above, the push-in guide part 6 is disposed on the first main shaft 70 of the first shaft 7, and specifically, the push-in guide part 6 is the distal end part of each of the protrusions 741, 742, and 743 of the first shaft 7 (see Fig. 3C, Fig. 5C, and Fig. 7C).

The discharge nozzle 9 is a part that brings out the medical liquid (see Fig. 1A). The discharge nozzle 9 of this embodiment is configured to discharge the medical liquid in a mist form. The discharge nozzle 9 is attached to the distal end part 20 of the barrel body 22. Specifically, the discharge nozzle 9 is attached to the body distal end part 220 of the barrel body 22 by press-fitting.

A detailed description will be hereinbelow made for the operation and various conditions in using the syringe 1.

The initial state is, as shown in Fig. 1A to Fig. 1C, the state where the syringe 1 has not yet discharged the medical liquid. In the initial state, the second shaft 8 is biased toward the proximal end relative to the first shaft 7 by the biasing force of the biasing member 4, and the restriction part 83 is in the restriction implementing position. The restriction locked part 82 of the restriction part 83 is locked to the restriction locking part 76 of the fourth one-side protrusion 734 from the proximal end side of the first shaft 7 by the biasing force of the biasing member 4 (see Fig. 1C). Specifically, the proximal end surface 820 of the restriction locked part 82 of the restriction part 83 is locked to the distal end surface 7341 of the fourth one-side protrusion 734 from the proximal end side. This locking restricts the movement of the second shaft 8 toward the proximal end relative to the first shaft 7. In the initial state, the second proximal end part 81 of the second shaft 8 is disposed with an axial gap between itself and the first proximal end part 71 of the first shaft 7.

From the initial state, when the user of the syringe 1 pushes the second shaft 8 (specifically, the second proximal end part 81) toward the distal end, the first shaft 7 is pushed toward the distal end together with the second shaft 8, as shown in Fig. 3A to Fig. 3C, and thereby the first discharge of the medical liquid is performed. Specifically, when the user pushes the second proximal end part 81 toward the distal end, the second proximal end part 81 moves toward the distal end until it abuts against the first proximal end part 71. Further, when the user pushes the second proximal end part 81 toward the distal end, the distal end surface 810 of the second proximal end part 81, which faces toward the distal end, abuts against the proximal end surface 710 of the first proximal end part 71, which faces toward the proximal end side (see Fig. 3A). Subsequently, when the user pushes the second proximal end part 81 toward the distal end, the distal end surface 810 of the second proximal end part 81 is pressed against the proximal end surface 710 of the first proximal end part 71, and in this pushed state, the first shaft 7 is pushed toward the distal end together with the second shaft 8, and thereby the first discharge of the medical liquid is performed. That is, when in the first discharge of the medical liquid, the distal end surface 810 of the second proximal end part 81 is a locked surface of the push-in locked part 84, and the proximal end surface 710 of the first proximal end part 71 is a locking surface of the push-in locking part 77. From the initial state until immediately after the first discharge, the second shaft 8 is biased toward the proximal end relative to the first shaft 7 by the biasing force of the biasing member 4.

The first discharge of the medical liquid is completed at a position at which the discharge completion locked part 86 of the discharge completion part 87 of the second shaft 8 is locked to the discharge completion locking part 24 of the barrel 2 (see Fig. 3B). Specifically, the first discharge of the medical liquid is completed at a position at which the distal end surface 860 of the discharge completion locked part 86 is locked to the proximal end surface 240 of the discharge completion locking part 24.

When in the first discharge of the medical liquid, the restriction part 83 of the second shaft 8 is moved from the restriction implementing position to the restriction withdrawn position by the restriction release part 5. Specifically, the restriction part 83 bends radially inward due to abutting of the end surface 8321 on the radially outer side of the restriction part 83 against the restriction release part 5 disposed on the barrel 2, specifically in this embodiment, the end part 2321 on the radially inner side of the body proximal end part 232 of the finger grip 23, and thereby the restriction part 83 is moved from the restriction implementing position to the restriction withdrawn position. Thereby, the restriction locked part 82 of the second shaft 8 also moves radially inward, and the locking of the restriction locked part 82 to the restriction locking part 76 of the first shaft 7 is released. Then, the second shaft 8 moves toward the proximal end relative to the first shaft 7 by the biasing force, coming into the state immediately before the second discharge shown in Fig. 4A and Fig. 4B.

When in the first discharge, the restriction part 83 is moved to the restriction withdrawn position by the restriction release part 5, and then moves to the restriction implementing position between the restriction locking part 76, to which the restriction part 83 has locked (previously locked) for the administration of one separate portion of the content and the (next) restriction locking part 76 that is located adjacent to this restriction locking part 76 on the proximal end side in the axial direction (see Fig. 4B). Specifically, after the restriction part 83 is moved to the restriction withdrawn position by the restriction release part 5, the restriction part 83 moves to the restriction implementing position between the restriction locking part 76 of the fourth one-side protrusion 734 from the proximal end of the first shaft 7, which was locked in the initial state, and the restriction locking part 76 of the third one-side protrusion 73 from the proximal end side of the first shaft 7.

In the state immediately after the first discharge, the push-in part 85 of the second shaft 8 is located closer to the distal end than the third other-side protrusion 743 from the proximal end side (see Fig. 3C). When the second shaft 8 moves toward the proximal end relative to the first shaft 7 by the biasing force of the biasing member 4, and comes into the state immediately before the second discharge, the proximal end surface 841 of the push-in locked part 84 of the push-in part 85 abuts against the distal end surface 7432 of the third other-side protrusion 743 from the proximal end side of the first shaft 7 (see Fig. 4B). Thereby, the push-in part 85 is pushed to the radially inner side and comes into the push-in withdrawn position, passing the third other-side protrusion 743 from the proximal end side toward the proximal end, and moving to a position between the second other-side protrusion 742 from the proximal end side and the third other-side protrusion 743 from the proximal end side. Thus, the push-in part 85 is moved to the push-in withdrawn position by the distal end surface 7432 of the third other-side protrusion 743, and then comes into the push-in implementing position at a position closer to the proximal end than the third other-side protrusion 743 from the proximal end side. In this manner, when the push-in part 85 is positioned on the proximal end side relative to the main-shaft push-in locking part 77 on the proximal end side, the push-in guide part 6 releases the retaining of the push-in part 85 in the push-in withdrawn position.

When the second shaft 8 moves toward the proximal end relative to the first shaft 7 by the biasing force of the biasing member 4 to cause shifting from the state immediately after the first discharge to the state immediately before the second discharge, the restriction guided part 832 moves while being guided by the fourth one-side protrusion 734 from the proximal end side. Specifically, the restriction guided part 832 is guided by the guide surface 7340 facing the one-side slit 700 side of the one-side protrusion 734, so that the second shaft 8 moves along the axial direction.

When in this movement, the push-in guided part 852 moves while being guided by the third other-side protrusion 743 from the proximal end side. Specifically, the push-in guided part 852 is guided by the guide surface 7430 facing the other-side slit 701 side of the other-side protrusion 743, so that the second shaft 8 moves along the axial direction. When in this movement, the discharge guided part 870 moves while being guided and moved by the fourth other-side protrusion 744 from the proximal end side. Specifically, the discharge guided part 870 is guided by the guide surface 7440 facing the other-side slit 701 side of the other-side protrusion 744, so that the second shaft 8 moves along the axial direction. When in this movement, the projection guided part 880 moves while being guided by the third one-side protrusion 733 from the proximal end side. Specifically, the projection guided part 880 is guided by the guide surface 7330 facing the one-side slit 700 side of the one-side projection 733, so that the second shaft 8 moves along the axial direction.

When in this movement, the inner surface 8810 of the projection extension part 881 abuts against the outer surface 8330 on the radially outer side of the restriction extension part 833, thereby preventing the restriction part 83 from opening radially outward (see Fig. 3B). When in this movement, the inner surface 8710 of the discharge extension part 871 abuts against the outer surface 8530 on the radially outer side of the push-in extension part 853, thereby preventing the push-in part 85 from opening radially outward.

In the state immediately before the second discharge, the restriction locked part 82 of the restriction part 83 of the second shaft 8 is locked to the restriction locking part 76 of the third one-side protrusion 733 from the proximal end side of the first shaft 7 by the biasing force of the biasing member 4 (see Fig. 4B). Specifically, the proximal end surface 820 of the restriction locked part 82 of the restriction part 83 is locked to the distal end surface 7331 of the third one-side protrusion 733 from the proximal end side. This locking restricts the movement of the second shaft 8 relative to the first shaft 7. In this initial state, the second proximal end part 81 of the second shaft 8 is disposed with an axial gap between itself and the first proximal end part 71 of the first shaft 7.

From the state immediately before the second discharge (see Fig. 4A and Fig. 4B), when the user of the syringe 1 pushes the second shaft 8 (specifically, the second proximal end part 81) toward the distal end, the first shaft 7 is pushed toward the distal end together with the second shaft 8, as shown in Fig. 5A to Fig. 5C, and thereby the second discharge of the medical liquid is performed. Specifically, when the user pushes the second proximal end part 81 toward the distal end, the distal end surface 840 of the push-in locked part 84 abuts against the proximal end surface 770 of the push-in locking part 77 of the first shaft 7, and in this state, the first shaft 7 is pushed toward the distal end together with the second shaft 8, and thereby the second discharge of the medical liquid is performed (see Fig. 5C). Specifically, the distal end surface 840 abuts against the proximal end surface 7431 of the third other-side protrusion 743 from the proximal end side. From the state immediately before the second discharge until immediately after the second discharge, the second shaft 8 is biased toward the proximal end by the biasing force of the biasing member 4 relative to the first shaft 7.

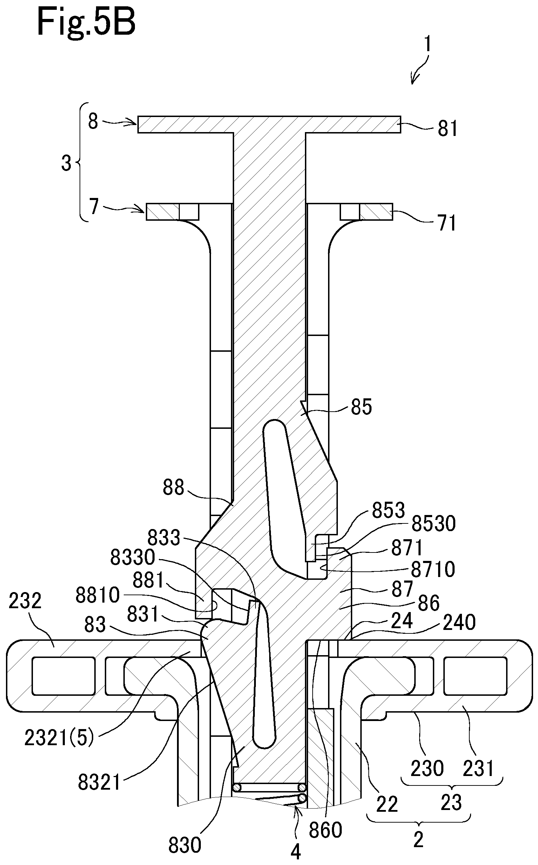

The second discharge of the medical liquid is completed at a position at which the distal end surface 860 of the discharge completion locked part 86 is locked to the proximal end surface 240 of the discharge completion locking part 24 (see Fig. 5B). When in the second discharge of the medical liquid, the restriction part 83 of the second shaft 8 bends radially inward due to abutting of the end surface 8321 on the radially outer side of the restriction part 83 against the end part 2321 on the radially inner side of the body proximal end part 232 of the finger grip 23, and thereby the restriction part 83 of the second shaft 8 is moved from the restriction implementing position to the restriction withdrawn position. Thereby, the restriction locked part 82 of the second shaft 8 also moves radially inward, and the locking of the restriction locked part 82 to the restriction locking part 76 of the first shaft 7 is released. Then, the second shaft 8 moves toward the proximal end relative to the first shaft 7 by the biasing force, coming into the state immediately before the third discharge, as shown in Fig. 6A and Fig. 6B.

When in the second discharge, the restriction part 83 is moved to the restriction withdrawn position by the restriction release part 5, and then moves to the restriction implementing position between the restriction locking part 76 of the third one-side protrusion 733 from the proximal end side of the first shaft 7, which was locked immediately before the second discharge, and the restriction locking part 76 of the second one-side protrusion 732 from the proximal end side.

When coming into immediately before the third discharge, the proximal end surface 841 of the push-in locked part 84 of the push-in part 85 abuts against the distal end surface 7422 of the second other-side protrusion 742 from the proximal end side of the first shaft 7, causing the push-in part 85 to be pushed radially inward into the push-in withdrawn position (see Fig. 6B). Thereby, the push-in part 85 passes the second other-side protrusion 742 from the proximal end side toward the proximal end, and moves to a position between the other-side protrusion 741 located on the most proximal side and the second other-side protrusion 742 from the proximal end side.

When the second shaft 8 moves toward the proximal end relative to the first shaft 7 by the biasing force of the biasing member 4 in order to cause shifting from the state immediately after the second discharge to the state immediately before the third discharge, the restriction guided part 832 is guided by the guide surface 7330 of the third one-side protrusion 733 from the proximal end side, and thereby the second shaft 8 moves along the axial direction. When in this movement, the push-in guided part 852 is guided by the guide surface 7420 of the second other-side protrusion 742 from the proximal end side, and thereby the second shaft 8 moves along the axial direction. When in this movement, the discharge guided part 870 is guided by the guide surface 7430 of the third other-side protrusion 743 from the proximal end side, and thereby the second shaft 8 moves along the axial direction. Furthermore, when in this movement, the projection guided part 880 is guided by the guide surface 7320 of the second one-side protrusion 732 from the proximal end side, and thereby the second shaft 8 moves along the axial direction.

When in this movement, the inner surface 8810 of the projection extension part 881 abuts against the outer surface 8330 on the radially outer side of the restriction extension part 833, thereby preventing the restriction part 83 from opening radially outward (see Fig. 5B). When in this movement, the inner surface 8710 of the discharge extension part 871 abuts against the outer surface 8530 on the radially outer side of the push-in extension part 853, thereby preventing the push-in part 85 from opening radially outward.

In the state immediately before the third discharge, the restriction locked part 82 of the restriction part 83 of the second shaft 8 is locked to the restriction locking part 76 of the second one-side protrusion 732 from the proximal end side of the first shaft 7 by the biasing force of the biasing member 4 (see Fig. 6B). Specifically, the proximal end surface 820 of the restriction locked part 82 of the restriction part 83 is locked to the distal end surface 7321 of the second one-side protrusion 732 from the proximal end side. This locking restricts the movement of the second shaft 8 relative to the first shaft 7. In this state, the second proximal end part 81 of the second shaft 8 is disposed with an axial gap between itself and the first proximal end part 71 of the first shaft 7.

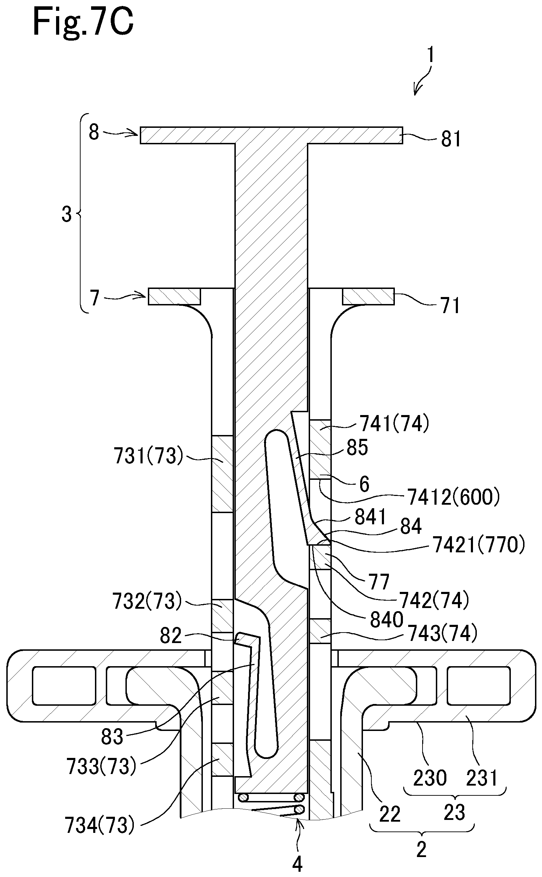

From the state immediately before the third discharge (see Fig. 6A and Fig. 6B), when the user of the syringe 1 pushes the second shaft 8 (specifically, the second proximal end part 81) toward the distal end, the first shaft 7 is pushed toward the distal end together with the second shaft 8, as shown in Fig. 7A to Fig. 7C, and thereby the third discharge of the medical liquid is performed. Specifically, when the user pushes the second proximal end part 81 toward the distal end, the distal end surface 840 of the push-in locked part 84 abuts against the proximal end surface 7421 of the second other-side protrusion 742 from the proximal end side, and in this state, the first shaft 7 is pushed toward the distal end together with the second shaft 8 (see Fig. 7C). From the state immediately before the third discharge until immediately after the third discharge, the second shaft 8 is biased toward the proximal end by the biasing force of the biasing member 4 relative to the first shaft 7.

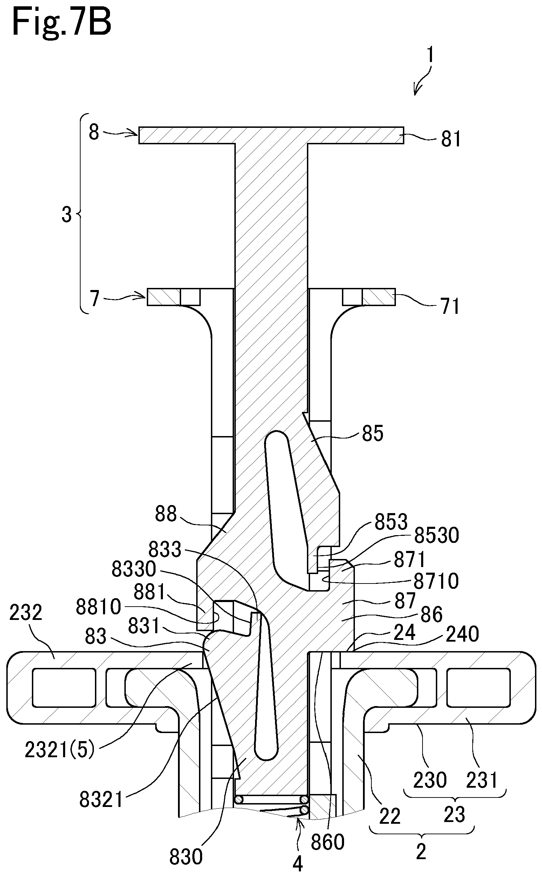

The third discharge of the medical liquid is completed at a position at which the distal end surface 860 of the discharge completion locked part 86 is locked to the proximal end surface 240 of the discharge completion locking part 24 (see Fig. 7B). When in the third discharge of the medical liquid, the restriction part 83 of the second shaft 8 bends radially inward due to abutting of the end surface 8321 on the radially outer side of the restriction part 83 against the end part 2321 on the radially inner side of the body proximal end part 232 of the finger grip 23, and thereby the restriction part 83 of the second shaft 8 is moved from the restriction implementing position to the restriction withdrawn position. Thereby, the restriction locked part 82 of the second shaft 8 also moves radially inward, and thereby the restriction locked part 82 is released from the locking engagement with the restriction locking part 76 of the first shaft 7. Thereby, the second shaft 8 moves toward the proximal end relative to the first shaft 7 by the biasing force, coming into the state immediately before the fourth discharge as shown in Fig. 8A and Fig. 8B.

When also in the third discharge, the restriction part 83 is moved to the restriction withdrawn position by the restriction release part 5, and then moves to the restriction implementing position between the restriction locking part 76 of the second one-side protrusion 732 from the proximal end side of the first shaft 7, which was locked immediately before the third discharge, and the restriction locking part 76 of the one-side protrusion 731 located on the most proximal side.

When coming into immediately before the fourth discharge, the proximal end surface 841 of the push-in locked part 84 of the push-in part 85 abuts against the distal end surface 7412 of the other-side protrusion 741 located on the most proximal side of the first shaft 7, causing the push-in part 85 to be pushed radially inward into the push-in withdrawn position (see Fig. 8B). Thereby, the push-in part 85 passes the other-side protrusion 741 located on the most proximal side toward the proximal end, and moves further toward the proximal end than the other-side protrusion 741 located on the most proximal side.

When the second shaft 8 moves toward the proximal end relative to the first shaft 7 by the biasing force of the biasing member 4 in order to cause shifting from the state immediately after the third discharge to the state immediately before the fourth discharge, the restriction guided part 832 is guided by the guide surface 7320 of the second one-side protrusion 732 from the proximal end side, and thereby the second shaft 8 moves along the axial direction. When in this movement, the push-in guided part 852 is guided by the guide surface 7410 of the other-side protrusion 741 located on the most proximal side, and thereby the second shaft 8 moves along the axial direction. When in this movement, the discharge guided part 870 is guided by the guide surface 7420 of the second other-side protrusion 742 from the proximal end side, and thereby the second shaft 8 moves along the axial direction. Furthermore, when in this movement, the projection guided part 880 is guided by the guide surface 7310 of the one-side protrusion 731 located on the most proximal side, so that the second shaft 8 moves along the axial direction.

When in this movement, the inner surface 8810 of the projection extension part 881 abuts against the outer surface 8330 on the radially outer side of the restriction extension part 833, thereby preventing the restriction part 83 from opening radially outward (see Fig. 7B). When in this movement, the inner surface 8710 of the discharge extension part 871 abuts against the outer surface 8530 on the radially outer side of the push-in extension part 853, thereby preventing the push-in part 85 from opening radially outward.

In the state immediately before the fourth discharge, the restriction locked part 82 of the restriction part 83 of the second shaft 8 is locked to the restriction locking part 76 of the one-side protrusion 731 located on the most proximal side of the first shaft 7 by the biasing force of the biasing member 4 (see Fig. 8B). Specifically, the proximal end surface 820 of the restriction locked part 82 of the restriction part 83 is locked to the distal end surface 7311 of the one-side protrusion 731 located on the most proximal side. This locking restricts the movement of the second shaft 8 relative to the first shaft 7. In this state, the second proximal end part 81 of the second shaft 8 is disposed with an axial gap between itself and the first proximal end part 71 of the first shaft 7.

From the state immediately before the fourth discharge (see Fig. 8A and Fig. 8B), when the user of the syringe 1 pushes the second shaft 8 (specifically, the second proximal end part 81) toward the distal end, the first shaft 7 is pushed toward the distal end together with the second shaft 8, as shown in Fig. 9A and Fig. 9B, and thereby the fourth discharge of the medical liquid is performed. Specifically, when the user pushes the second proximal end part 81 toward the distal end, the distal end surface 840 of the push-in locked part 84 abuts against the proximal end surface 7411 of the other-side protrusion 741 located on the most proximal side, and in this state, the first shaft 7 is pushed toward the distal end together with the second shaft 8 (see Fig. 9B). From the state immediately before the fourth discharge until immediately after the fourth discharge, the second shaft 8 is biased toward the proximal end by the biasing force of the biasing member 4 relative to the first shaft 7.

The fourth discharge of the medical liquid is completed in the state where the piston 30 disposed on the first distal end part 72 of the first shaft 7 abuts against the distal end part of the body connecting part 222 of the barrel body 22.

According to the syringe 1 described above, when the user pushes in the plunger 3 to perform the second, third, and fourth discharges while the movement of the second shaft 8 toward the proximal end relative to the first shaft 7 by the biasing force of the biasing member 4 is restricted by the locking of the restriction locked part 82 to the restriction locking part 76, the locking of the push-in locked part 84 to the push-in locking part 77 ensures that the discharge can be performed with the entire plunger 3 pushed in. At a position at which the pushing-in (discharge) for one separation portion of the content is completed, the restriction release part 5 releases the locking of the restriction locked part 82 to the restriction locking part 76. Thereby, the second shaft 8 moves toward the proximal end relative to the first shaft 7 by the biasing force of the biasing member 4, and the restriction locked part 82 forms the next locking (restriction) with the next restriction locking part 76 located on the proximal end side in the axial direction relative to the restriction locking part 76, to which the restriction locked part 82 has locked (previously locked) for the administration of one separate portion of the content, and the push-in locking part 77 and the push-in locked part 84 then form the next locking (push-in). In this way, the syringe 1 securely switches to prepare for the next discharge, ensuring that a plurality of discharges can be performed reliably.

In the syringe 1 of this embodiment, after the discharge of one separate portion of the content has been completed and the restriction part 83 of the second shaft 8 has been moved to the restriction withdrawn position by the restriction release part 5, the restriction part 83 switches to the restriction implementing position on the distal end side of the (next) restriction locking part 76 located on the proximal end side in the axial direction relative to the restriction locking part 76, to which the restriction part 83 has locked for the administration of one separate portion of the content, so that the restriction locked part 82 is securely locked to the next restriction locking part 76.

In the syringe 1 of this embodiment, the push-in part 85 of the second shaft 8 pivotally moves between the push-in implementing position and the push-in withdrawn position, thereby switching locking and unlocking between the main-shaft push-in locking part 77 of the first shaft 7 and the main-shaft push-in locked part 84 of the second shaft 8. Therefore, after discharge of one separate portion of the content is completed, the main-shaft push-in locked part 84 is securely locked to the next main-shaft push-in locking part 77, which is located on the proximal end side in the axial direction relative to the main-shaft push-in locking part 77 that was previously locked.

In the syringe 1 of this embodiment, the push-in part 85 switches to the push-in implementing position at a position on the proximal end side in the axial direction of the next main-shaft push-in locking part 77. Therefore, after discharge of one separate portion of the content is completed, the main-shaft push-in locked part 84 is securely locked to the next main-shaft push-in locking part 77.

It should be noted that the syringe of the present invention is not limited to the embodiments described above, and various modifications can be made without departing from the spirit of the present invention. For example, the configuration of one embodiment can be combined with the configuration of another embodiment, and a part of the configuration of one embodiment can be replaced with the configuration of another embodiment. A part of the configuration of a certain embodiment can be removed.

For example, the configuration of each component of the syringe 1 can differ from the configuration described above. In the above embodiment, the first shaft 7 and the second shaft 8 of the plunger 3 each have an asymmetrical shape, but they can also have an axially symmetrical shape.

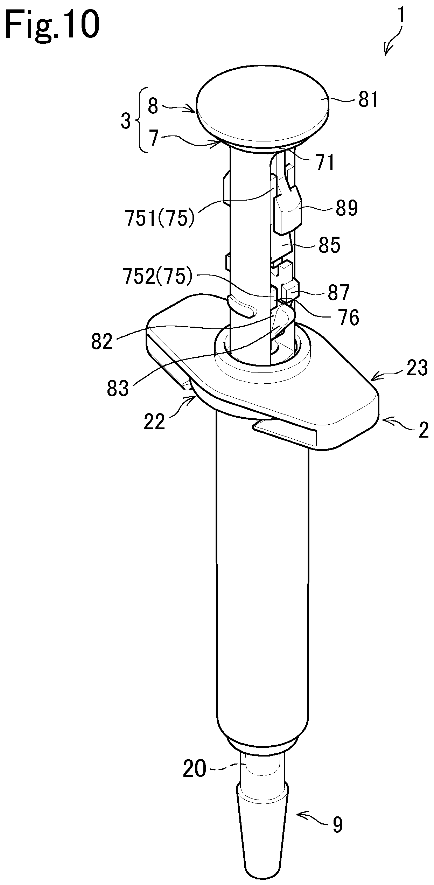

A description will be hereinbelow given for the configuration in which the first shaft 7 and the second shaft 8 each have an axially symmetrical shape, and the syringe 1 administers the medical liquid in two separate portions, with reference to Fig. 10 to Fig. 15B.

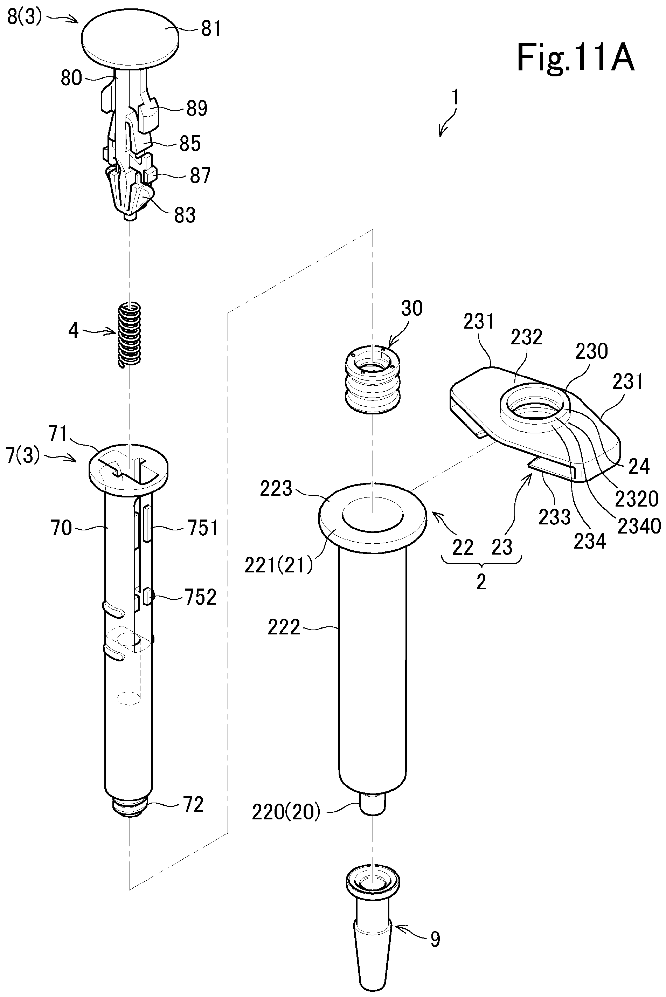

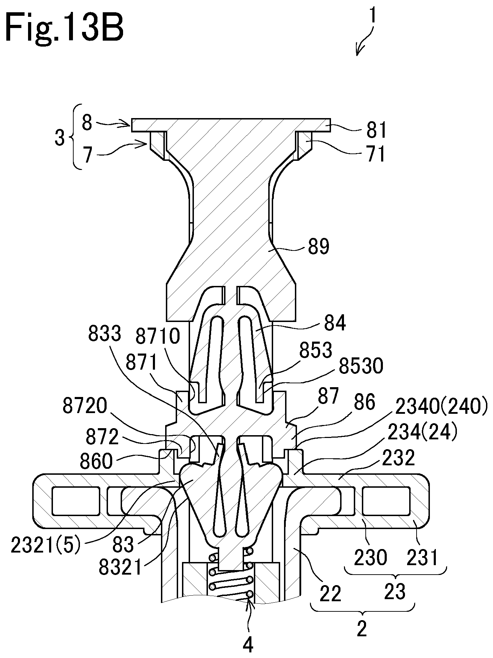

In this configuration, the finger grip 23 includes the grip body part 230 and the pair of grip extension parts 231, as well as a grip proximal end side extension part 234 having an annular shape that extends from an end part on the radially inner side of the grip body part 230 toward the proximal end and surrounds the plunger 3 (see Fig. 10 and Fig. 11A). The finger grip 23 includes the grip proximal end side extension part 234, which serves as a discharge completion locking part 24 that locks to the plunger 3 at the completion of the first discharge of the syringe 1 (see Fig. 13B). The proximal end surface 240 of the discharge completion locking part 24 is composed of a proximal end surface 2340 that is the surface facing the proximal end side of the grip proximal end side extension part 234. Specifically, two locations of the proximal end surface 2340, which are located opposite each other with a center line of the grip body part 230 therebetween, are the locations to which the plunger 3 is locked. In this embodiment, the proximal end surface 240 of the discharge completion locking part 24 faces the finger grip 23 at a position 180° apart in the circumferential direction.

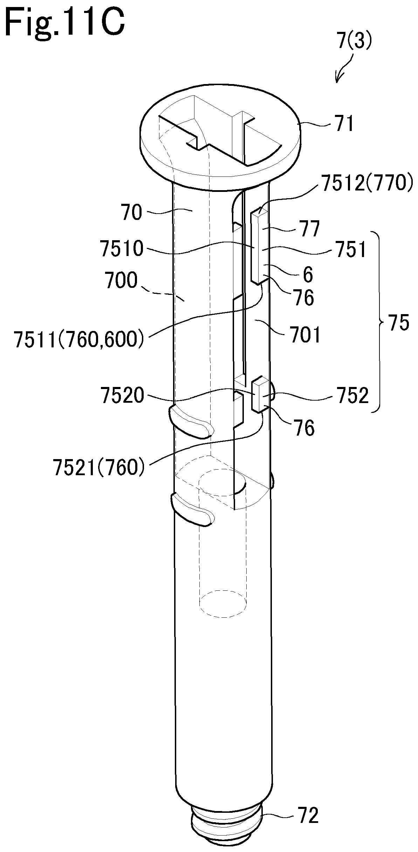

As shown in Fig. 11C, the first shaft 7 includes the first main shaft 70, the first proximal end part 71, and the first distal end part 72, as well as a plurality of protrusions 75 disposed on one side and the other side of the first main shaft 70. Two sets of the protrusions 75 are respectively arranged at intervals in the axial direction of the first main shaft 70, while those of each set are located with the slit 700 or 701 therebetween. The protruding dimensions of the protrusions 75 relative to the slits 700 and 701 are all the same. The protrusion 751 disposed on the proximal end side has a larger axial dimension than the protrusion 752 disposed on the distal end side.

Each of the protrusions 75 guides the axial movement of the second shaft 8 relative to the first shaft 7 when the second shaft 8 moves relative to the first shaft 7 by the biasing force of the biasing member 4. Each of the protrusions 75 restricts the circumferential movement of the second shaft 8 in the state where the movement of the second shaft 8 relative to the first shaft 7 by the biasing force of the biasing member 4 is restricted. Each of the protrusions 751 and 752 has guide surfaces 7510 and 7520 facing the slits 700 and 701 sides, which abut against a portion of the second shaft 8, thereby guiding the axial movement of the second shaft 8 and restricting the circumferential movement of the second shaft 8. The guide surfaces 7510 and 7520 of the protrusions 75 are flat surfaces.

Each of the protrusions 751 and 752 includes a restriction locking part 76 that restricts the movement of the second shaft 8 relative to the first shaft 7 by the biasing force of the biasing member 4. In this embodiment, the distal end parts of the protrusions 751 and 752 each form the restriction locking part 76. Distal end surfaces 7511 and 7521, which are surfaces facing the distal end sides of the protrusions 751 and 752, each form the distal end surface 760 of the restriction locking part 76. The restriction locking parts 76 restrict the movement of the second shaft 8 relative to the first shaft 7 by the biasing force, by having their distal end surfaces 760 abutting against a portion of the second shaft 8. The distal end surface 760 is a flat surface.

Two sets of the restriction locking parts 76 are disposed with a distance from each other in the axial direction, while those of each set are located with the central axis of the first shaft 7 therebetween. Specifically, the restriction locking part 76 is composed of two sets of the main shaft restriction locking parts 76 aligned in the axial direction, while those of each set are located with the central axis of the first shaft 7 therebetween. In this embodiment, the restriction locking parts 76 are located opposite each other at positions 180° apart in the circumferential direction of the first shaft 7.

The protrusion 751 located on the proximal end side includes a push-in locking part 77 to which the second shaft 8 is locked, when the plunger 3 is pushed into the barrel 2. One set of the push-in locking parts 77 are disposed in the axial direction. In this embodiment, the proximal end part of the protrusion 751 forms the push-in locking part 77. The proximal end surface 7512, which is a surface facing the proximal end side of the protrusion 751, forms the proximal end surface 770 of the push-in locking part 77. The proximal end surface 770 of the push-in locking part 77 abuts against a portion of the second shaft 8, thereby restricting the axial movement of the second shaft 8 relative to the first shaft 7 toward the distal end, and enabling the plunger 3 to be pushed into the barrel 2. The proximal end surface 770 of the push-in locking part 77 is a flat surface.

One set of the push-in locking parts 77 are disposed with the central axis of the first shaft 7 therebetween. Specifically, the push-in locking parts 77 are located opposite each other at positions 180° apart in the circumferential direction of the first shaft 7. In this embodiment, the push-in locking part 77 is composed of one set of the main shaft push-in locking parts 77.

In this embodiment, the protrusion 751 located on the proximal end side includes a push-in guide part 6 that guides a portion of the second shaft 8 from the push-in implementing position to the push-in withdrawn position. The distal end part of the protrusion 751 forms the push-in guide part 6. The distal end surface 7511, which is a surface facing the distal end side of the protrusion 751, forms the distal end surface 600 of the push-in guide part 6. The push-in guide part 6 guides a portion of the second shaft 8 from the push-in implementing position to the push-in withdrawn position by having the distal end surface 600 abutting against the portion of the second shaft 8.

One set of the push-in guide parts 6 are disposed with the central axis of the first shaft 7 therebetween. Specifically, the push-in guide parts 6 are located opposite each other at positions 180° apart in the circumferential direction of the first shaft 7. In this embodiment, the push-in guide parts 6 are configured such that each set of the main-shaft push-in guide parts 6 are located with the central axis of the first shaft 7 therebetween.

In this syringe 1, when in the first discharge of the medical liquid, the first proximal end part 71 forms a push-in locking part 77 that locks to a portion of the second proximal end part 81.

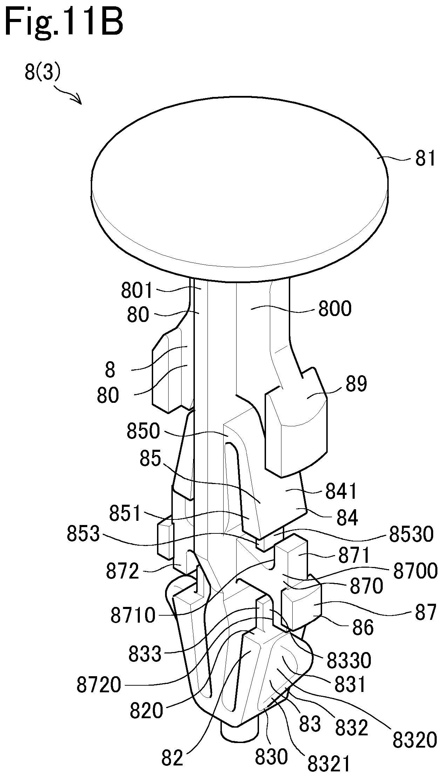

In this embodiment, as shown in Fig. 11B, the second shaft 8 includes the second main shaft 80 and the second proximal end part 81, as well as a restriction part 83, a distal end of which is a starting end 830 and a proximal end part of which is a free end 831. The restriction part 83 is configured to be pivotally movable about the starting end 830 as a starting point between a restriction withdrawn position where the free end 831 is located radially inward and a restriction implementing position where the free end 831 is located radially outward. The second shaft 8 includes a push-in part 85 that includes a push-in locked part 84 that is locked to the push-in locking part 77 of the first shaft 7 when the plunger 3 is pushed in. The second shaft 8 includes a projection part 89, and a discharge completion part 87 that includes a discharge completion locked part 86 that is locked to the discharge completion locking part 24 when the discharge of the medical liquid is completed.

In this second shaft 8, a pair of the restriction parts 83 are disposed at positions opposite each other with the central axis in the circumferential direction of the second main shaft 80 therebetween. Specifically, the restriction parts 83 are located opposite each other at positions 180° apart in the circumferential direction of the second main shaft 80. Similarly, the push-in part 85, the discharge completion part 87, and the projection parts 88 and 89 are each disposed in pairs at positions opposite each other with the central axis in the circumferential direction of the second main shaft 80 therebetween. Specifically, at least one of the push-in part 85, the discharge completion part 87, and the projection parts 88 and 89, and in this embodiment, all of these are located opposite each other at positions 180° apart in the circumferential direction of the second main shaft 80. In this second shaft 8, the projection part 89, the push-in part 85, the discharge completion part 87, and the restriction part 83 are arranged in order from the proximal end side toward the distal end side in the axial direction.

In this embodiment, the second main shaft 80 includes a second main wall part 800 having a substantially rectangular plate shape and extending along the axial direction, and a second sub-wall part 801 extending orthogonal to the second main wall part 800 through the center in the width direction of the second main wall part 800. The second main wall part 800 has a partially cut-out shape, and the remaining portions respectively form the projection part 89, the push-in part 85, the discharge completion part 87, and the restriction part 83. The second sub-wall part 801 fits into the first shaft 7. This fitting between them restricts the circumferential movement of the second shaft 8 in the state where the movement of the second shaft 8 relative to the first shaft 7 by the biasing force of the biasing member 4 is restricted. This fitting also restricts the circumferential movement of the second shaft 8 when the plunger 3 is pushed in or when the second shaft 8 moves in the axial direction relative to the first shaft 7 by the biasing force.

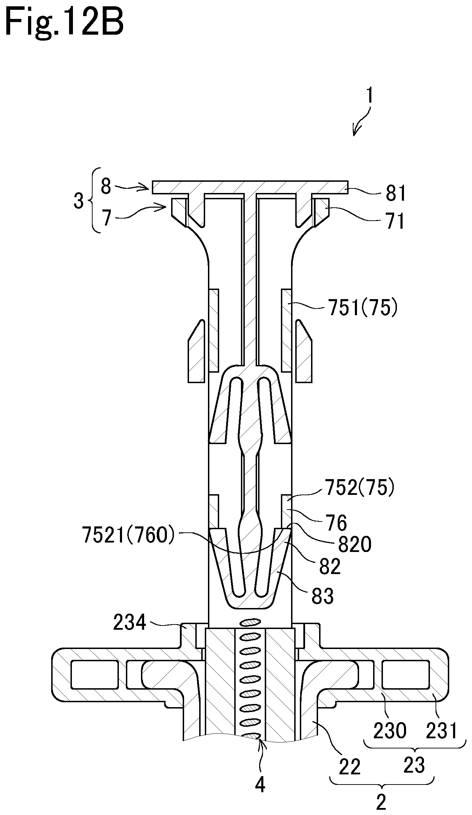

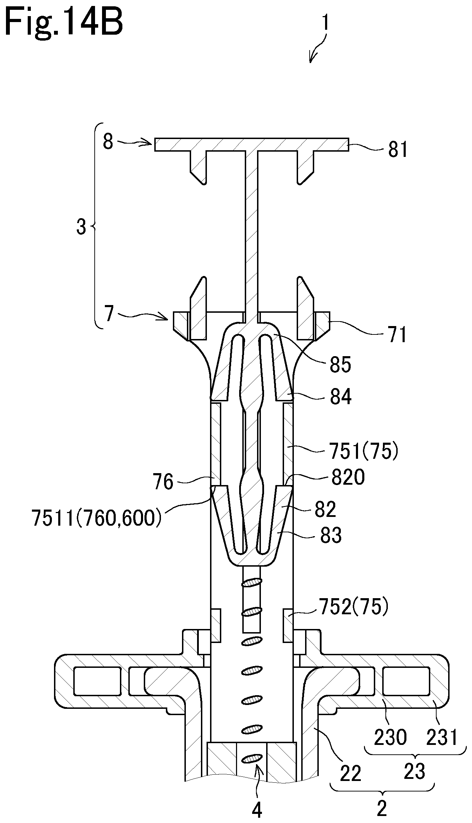

The restriction part 83 includes a restriction locked part 82 at the free end 831 that, at the restriction implementing position, is locked to the restriction locking part 76 of the first shaft 7 by the biasing force of the biasing member 4. In this embodiment, the proximal end part of the restriction part 83 forms the restriction locked part 82. When the restriction part 83 is in the restriction implementing position, the restriction locked part 82 is locked to the restriction locking part 76 (see Fig. 12B and Fig. 14B). When the restriction part 83 is in the restriction withdrawn position, the restriction locked part 82 is not locked to the restriction locking part 76.

In this embodiment, one set of the restriction locked parts 82 are disposed on the restriction part 83 (see Fig. 11B). The surface of the restriction part 83 facing the proximal end side, specifically the proximal end surface 820 of the restriction locked part 82, abuts against the distal end surface 760 of the restriction locking part 76 of the first shaft 7, thereby restricting the movement of the second shaft 8 relative to the first shaft 7 by the biasing force (see Fig. 12B and Fig. 14B). The proximal end surface 820 of each of the restriction locked parts 82 is a flat surface.

The restriction part 83 includes the restriction guided parts 832 disposed on the radially outer sides of the pair of the restriction locked parts 82 (see Fig. 11B). The restriction part 83 includes the restriction extension parts 833 that each extend further toward the proximal end from the end part on the radially inner side of each of the restriction locked parts 82.

The restriction guided part 832 is a part that guides the axial movement of the second shaft 8 relative to the first shaft 7 when the second shaft 8 moves relative to the first shaft 7 by the biasing force of the biasing member 4.

In this embodiment, the restriction guided part 832 abuts against the protrusion 752 of the first shaft 7, thereby guiding the axial movement of the second shaft 8. Specifically, the outer surface 8320 of the restriction guided part 832, which faces in the circumferential direction of the second shaft 8, abuts against the guide surface 7520 of the protrusion 752, which faces the slits 700 and 701, thereby guiding the axial movement of the second shaft 8. The outer surface 8320 of the restriction guided part 832 is a flat surface.

The restriction extension part 833 is a stopper that prevents the restriction part 83 from opening radially outward when the second shaft 8 moves relative to the first shaft 7 by the biasing force of the biasing member 4, and when the movement of the second shaft 8 relative to the first shaft 7 is restricted. The restriction extension part 833 is located radially inward of a portion of the discharge completion part 87. In this embodiment, the restriction extension part 833 is a rib that extends axially from the end part on the radially inner side of the restriction locked part 82 toward the proximal end. The outer surface 8330 located radially outward of the restriction extension part 833 is a flat surface.

The push-in part 85 has a starting end 850 on the proximal end side and a free end 851 on the distal end side, and is configured to be pivotally movable around the starting end 850 between a push-in withdrawn position where the free end 851 is located radially inward and a push-in implementing position where the free end 851 is located radially outward. In this embodiment, the distal end part of the push-in part 85 forms the push-in locked part 84. The push-in locked part 84 is a main-shaft push-in locked part 84 disposed at the free end 851 of the push-in part 85, and is composed of the main-shaft push-in locked part 84 that is locked to the main-shaft push-in locking part 77 of the first shaft 7 in the push-in implementing position.

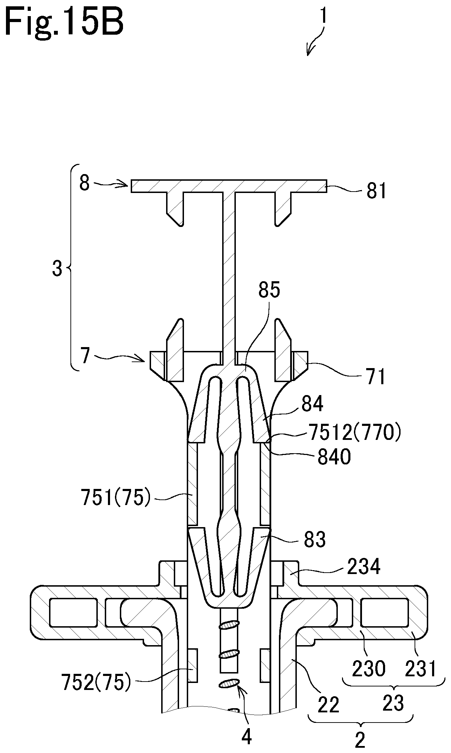

One push-in locked part 84 is disposed for each of the push-in parts 85.
When the plunger 3 is pushed in, the surface of the push-in part 85 facing the distal end side, specifically the distal end surface 840 of the push-in locked part 84, abuts against the proximal end surface 770 of the push-in locking part 77 of the first shaft 7. Thereby, a force that moves the second shaft 8 toward the distal end is transmitted to the first shaft 7, and the first shaft 7 moves together with the second shaft 8 (see Fig. 15B). The distal end surface 840 of the push-in locked part 84 is a flat surface.

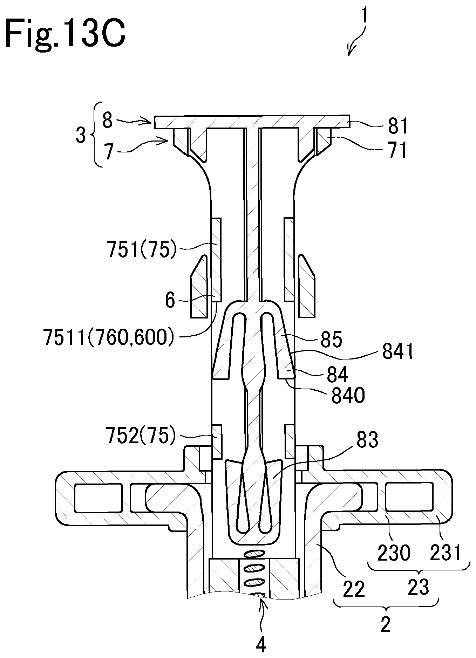

The proximal end surface 841, which is a surface facing the proximal end side of the push-in locked part 84, is an inclined surface that is located more radially inward toward the proximal end. When the second shaft 8 moves toward the proximal end relative to the first shaft 7 by the biasing force of the biasing member 4, the proximal end surface 841 of the push-in locked part 84 abuts against the distal end surface 7511, which is a surface facing the distal end side of the protrusion 751 of the first shaft 7. Thereby, the push-in part 85 moves from the push-in implementing position to the push-in withdrawn position (see Fig. 13C). The proximal end surface 841 of the push-in locked part 84 is a flat surface.

The push-in part 85 includes the push-in extension part 853 that extends further toward the distal end from the end part on the radially inner side of the push-in locked part 84 (see Fig. 11B).

The push-in extension part 853 is a stopper that prevents the push-in part 85 from opening radially outward when the second shaft 8 moves relative to the first shaft 7 by the biasing force of the biasing member 4, and when the user of the syringe 1 moves the second shaft 8 toward the distal end. In this embodiment, the push-in extension part 853 is a rib that extends axially toward the distal end from the end part on the radially inner side of the push-in locked part 84 . The push-in extension part 853 is located radially inward of a portion of the discharge completion part 87. The surface 8530 on the radially outer side of the push-in extension part 853 is a flat surface.

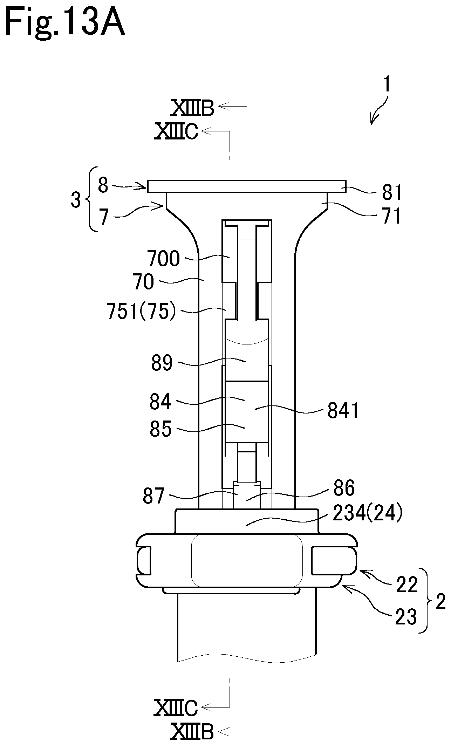

The discharge completion part 87 is a stopper that restricts the plunger 3, which has moved a distance corresponding to the discharge of one separate portion, from moving by being locked to the discharge completion locking part 24 of the barrel 2 (see Fig. 13A and Fig. 13B). In this embodiment, the distal end part of the discharge completion part 87
forms the discharge completion locked part 86. One set of the discharge completion locked parts 86 is disposed on the second main shaft 80. The distal-end-facing surface of the discharge completion part 87, specifically the distal end surface 860 of the discharge completion locked part 86, abuts against the proximal end surface 240 of the discharge completion locking part 24 of the barrel 2, thereby completing the movement of the plunger 3 toward the distal end, that is, the first discharge of the medical liquid by the user of the syringe 1 (see Fig. 13B). The distal end surface 860 of the discharge completion locked part 86 is a flat surface.

The discharge completion part 87 includes a discharge guided part 870, which is an end part on the radially outer side (see Fig. 11B). The discharge completion part 87 includes discharge extension parts 871 and 872 that extend as end parts on the proximal end side and the distal end side, respectively, of the discharge guided part 870.

The discharge guided part 870 is a part that guides the axial movement of the second shaft 8 relative to the first shaft 7 when the second shaft 8 moves relative to the first shaft 7 by the biasing force of the biasing member 4. The discharge guided part 870 is a part that restricts the circumferential movement of the second shaft 8 in the state where the movement of the second shaft 8 relative to the first shaft 7 by the biasing force of the biasing member 4 is restricted.

In this embodiment, the discharge guided part 870 abuts against the protrusion 752 on the distal end side of the first shaft 7, thereby guiding the axial movement of the second shaft 8, and restricting the circumferential movement of the second shaft 8. Specifically, the outer surface 8700 of the discharge guided part 870, which faces in the circumferential direction of the second shaft 8, abuts against the guide surface 7520 of the protrusion 752, which faces the slits 700 and 701 sides, thereby guiding the axial movement of the second shaft 8 and restricting the circumferential movement of the second shaft 8. The outer surface 8700 of the discharge guided part 870 is a flat surface.

The discharge extension part 871 is a stopper retaining part that presses the push-in extension part 853 of the push-in part 85 in order to prevent the push-in part 85 from opening radially outward when the second shaft 8 moves relative to the first shaft 7 by the biasing force of the biasing member 4, and when the user of the syringe 1 moves the second shaft 8 toward the distal end side (see Fig. 11B). The discharge extension part 871 is located radially outward of the push-in extension part 853. In this embodiment, the discharge extension part 871 is a rib that extends axially toward the proximal end as an end part on the proximal side of the discharge guided part 870. An inner surface 8710 on the radially inner side of the discharge extension part 871 is a flat surface. The inner surface 8710 of the discharge extension part 871 abuts against the surface 8530 on the radially outer side of the push-in extension part 853, thereby preventing the push-in part 85 from opening radially outward.

The discharge extension part 872 is a stopper retaining portion that presses the restriction extension part 833 of the restriction part 83 in order to prevent the restriction part 83 from opening radially outward when the second shaft 8 moves relative to the first shaft 7 by the biasing force of the biasing member 4, and in the state where the movement of the second shaft 8 relative to the first shaft 7 is restricted. The discharge extension part 872 is located radially outward of the restriction extension part 833. In this embodiment, the discharge extension part 872 is a rib that axially extends toward the distal end as an end part on the distal side of the discharge guided part 870. The inner surface 8720 on the radially inner side of the discharge extension part 872 is a flat surface. The inner surface 8720 of the discharge extension part 872 abuts against the outer surface 8330 on the radially outer side of the restriction extension part 833, thereby preventing the restriction part 83 from opening radially outward.

The projection part 89 is disposed on the second main shaft 80. The projection part 89 includes a projection guided part 890, which is an end part on the radially outer side.

The projection guided part 890 is a part that guides the axial movement of the second shaft 8 relative to the first shaft 7 when the second shaft 8 moves relative to the first shaft 7 by the biasing force of the biasing member 4. The projection guided part 890 is a part that restricts the circumferential movement of the second shaft 8 in the state where the movement of the second shaft 8 relative to the first shaft by the biasing force of the biasing member 4 is restricted.

In this embodiment, the projection guided part 890 abuts against the protrusion 751 on the proximal end side of the first shaft 7, thereby guiding the axial movement of the second shaft 8 and restricting the circumferential movement of the second shaft 8. Specifically, an outer surface 8900 of the projection guided part 890, which faces in the circumferential direction of the second shaft 8, abuts against the guide surface 7510 of the protrusion 751, which faces the slits 700 and 701, thereby guiding the axial movement of the second shaft 8 and restricting the circumferential movement of the second shaft 8. The outer surface 8900 is a flat surface.

The restriction release part 5 causes the restriction part 83 to move from the restriction implementing position to the restriction withdrawn position at a position at which the pushing-in for one separate portion of the content is completed, and thereby releases the restriction locked part 82 from the locking engagement with the restriction locking part 76 (see Fig. 13B). In this embodiment, the restriction release part 5 is composed of the end part 2321 on the radially inner side of the grip body part 230 of the finger grip 23, for example, the end part 2321 on the radially inner side of the body proximal end part 232 of the grip body part 230.

The push-in guide part 6 guides the push-in part 85 of the second shaft 8 from the push-in implementing position to the push-in withdrawn position when the push-in part 85 rides over the protrusion 751 on the proximal end side from the distal end side to the proximal end side. In this embodiment, as described above, the push-in guide part 6 is disposed on the main shaft 70 of the first shaft 7, and specifically, the push-in guide part 6 is the distal end surface 7511 of the protrusion 751 of the first shaft 7 (see Fig. 13C).

The following provides a detailed explanation of the operation and various conditions when using the syringe 1.

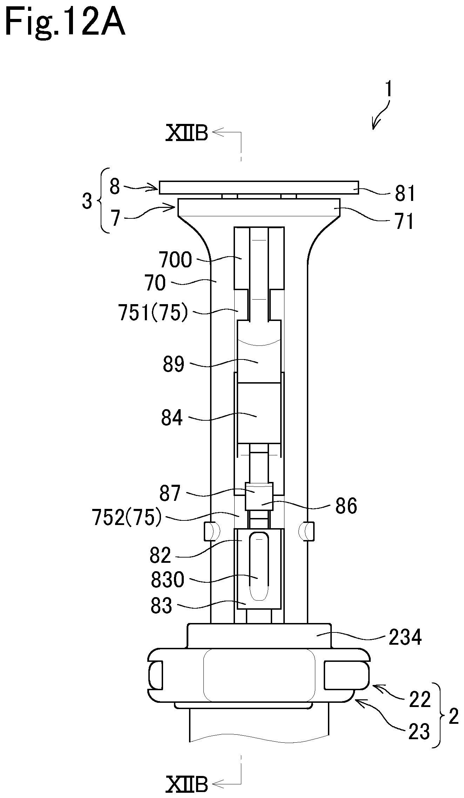

The initial state is as shown in Fig. 10, Fig. 12A, and Fig. 12B, the state where the syringe 1 has not yet discharged the medical liquid. In the initial state, the restriction locked part 82 of the restriction part 83 of the second shaft 8 is locked to the restriction locking part 76 of the protrusion 752 on the distal end side of the first shaft 7 by the biasing force of the biasing member 4. Specifically, the proximal end surface 820 of the restriction locked part 82 of the restriction part 83 is locked to the distal end surface 7521 of the protrusion 752 on the distal end side (see Fig. 12B). This locking restricts the movement of the second shaft 8 relative to the first shaft 7.

When the user of the syringe 1 pushes the second proximal end part 81 toward the distal end from the initial state, the first shaft 7 is pushed toward the distal end together with the second shaft 8, as shown in Fig. 13A to Fig. 13C, and thereby the first discharge of the medical liquid is performed.

The first discharge of the medical liquid is completed at a position at which the discharge completion locked part 86 of the discharge completion part 87 is locked to the discharge completion locking part 24 of the barrel 2. Specifically, the first discharge of the medical liquid is completed at a position at which the distal end surface 860 of the discharge completion locked part 86 is locked to the proximal end surface 240 of the discharge completion locking part 24 (see Fig. 13B).

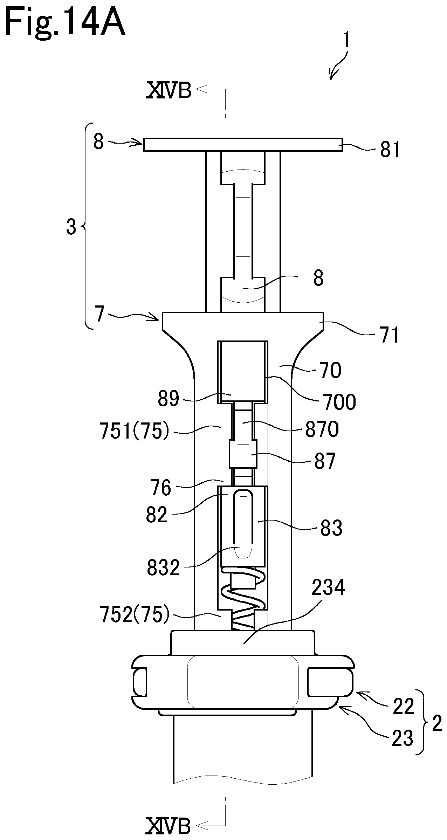

When in the first discharge of the medical liquid, the restriction part 83 of the second shaft 8 is moved from the restriction implementing position to the restriction withdrawn position by the restriction release part 5. Specifically, the restriction part 83 bends due to abutting of the end surface 8321 on the radially outer side of the restriction part 83 against the end part 2321 on the radially inner side of the body proximal end part 232 of the finger grip 23, so that the restriction part 83 is moved from the restriction implementing position to the restriction withdrawn position. Thereby, the restriction locked part 82 of the second shaft 8 also moves radially inward, and thereby the restriction locked part 82 is released from the locking engagement with the restriction locking part 76 of the first shaft 7. Then, the second shaft 8 moves toward the proximal end relative to the first shaft 7 by the biasing force of the biasing member 4, coming into the state immediately before the second discharge shown in Fig. 14A and Fig. 14B.

When in the first discharge, the restriction part 83 is moved to the restriction withdrawn position by the restriction release part 5, and then moves to the restriction implementing position between the restriction locking part 76 of the protrusion 752 on the distal end side of the first shaft 7, which was locked in the initial state, and the restriction locking part 76 of the protrusion 751 on the proximal end side.

In the state immediately after the first discharge, the push-in part 85 of the second shaft 8 is located closer to the distal end than the protrusion 751 on the proximal end side (see Fig. 13C). When coming into the state immediately before the second discharge after the second shaft 8 moves toward the proximal end relative to the first shaft 7 by the biasing force of the biasing member 4, the proximal end surface 841 of the push-in locked part 84 of the push-in part 85 abuts against the distal end surface 7511 of the protrusion 751 on the proximal end side. Thereby, the push-in part 85 is pushed radially inward, coming into the push-in withdrawn position, so that the push-in part 85 passes the protrusion 751 on the proximal end side toward the proximal end and moves to a position closer to the proximal end than the protrusion 751 on the proximal end side (see Fig. 15B). Thus, the push-in part 85 is moved to the push-in withdrawn position by the protrusion 751 on the proximal end, and then comes into the push-in implementing position at a position closer to the proximal end than the protrusion 751 on the proximal end side.

When the second shaft 8 moves toward the proximal end relative to the first shaft 7 by the biasing force of the biasing member 4 in order to cause shifting from the state immediately after the first discharge to the state immediately before the second discharge, the restriction guided part 832 moves while being guided by the protrusion 752 on the distal end side (see Fig. 14A). When in this movement, the discharge guided part 870 moves while being guided by the protrusion 752 on the distal end side. When in this movement, the projection guided part 890 moves while being guided by the protrusion 751 on the proximal end side.

When in this movement, the restriction part 83 is prevented from opening radially outward, and the push-in part 85 is prevented from opening radially outward (see Fig. 13B).

Immediately before the second discharge, the second shaft 8 is held in the state where the restriction locked part 82 of the restriction part 83 is locked to the restriction locking part 76 of the protrusion 751 on the proximal end side of the first shaft 7 by the biasing force of the biasing member 4 (see Fig. 14B). Specifically, the proximal end surface 820 of the restriction locked part 82 of the restriction part 83 is locked to the distal end surface 7511 of the protrusion 751 on the proximal end side. This locking restricts the movement of the second shaft 8 relative to the first shaft 7.

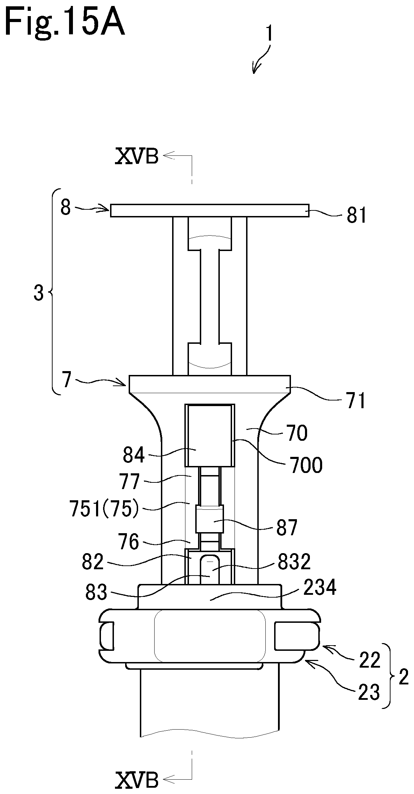

From the state immediately before the second discharge, when the user of the syringe 1 pushes the second shaft 8 (specifically, the second proximal end part 81) toward the distal end, as shown in Fig. 15A and Fig. 15B, the first shaft 7 is pushed toward the distal end together with the second shaft 8, and thereby the second discharge of the medical liquid is performed.

The second discharge of the medical liquid is completed in the state where the piston 30, disposed at the first distal end part 72 of the first shaft 7, abuts against the inner surface of the distal end part of the body connecting part 222 of the barrel body 22.

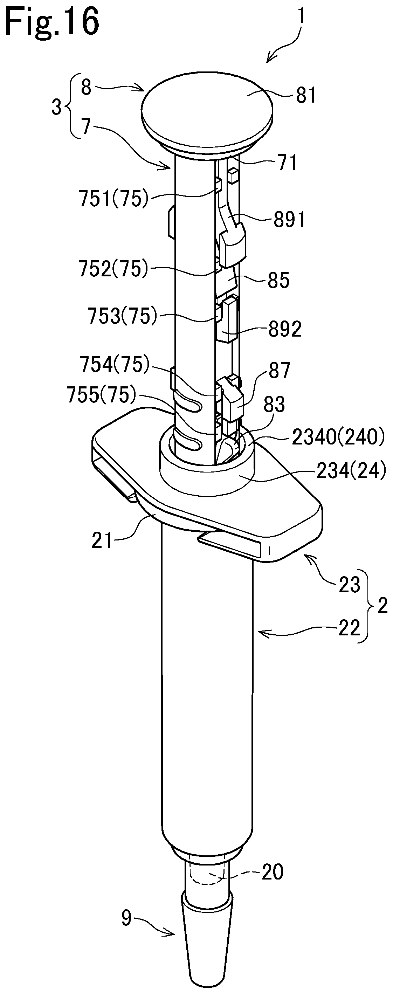

A description will be hereinbelow given for the configuration in which the first shaft 7 and the second shaft 8 each have an axially symmetrical shape, and the syringe 1 administers the medical liquid in three separate portions, with reference to Fig. 16 to Fig. 23C.

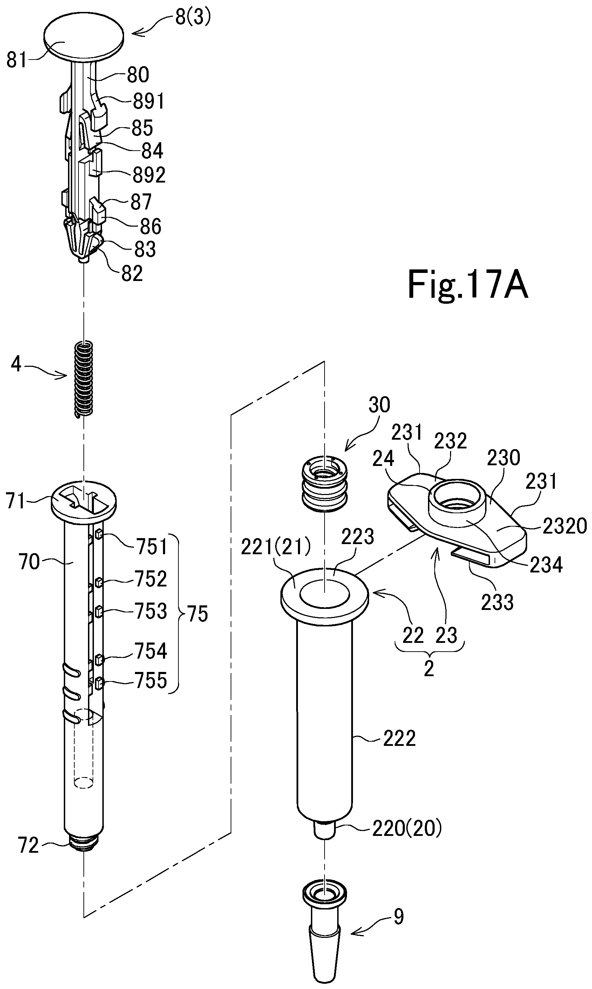

In this embodiment as well, as shown in Fig. 16 and Fig. 17A, the finger grip 23 includes the grip proximal end side extension part 234 as the discharge completion locking part 24 that locks to the plunger 3 when the first discharge and the second discharge of the syringe 1 are completed. The proximal end surface 240 of the discharge completion locking part 24 is composed of a proximal end surface 2340 which is a surface facing the proximal end side of the grip proximal end side extension part 234.

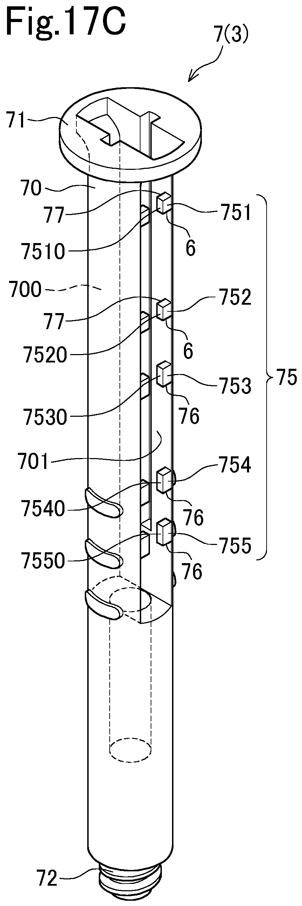

In this embodiment, as shown in Fig. 17C, the first shaft 7 includes the first main shaft 70, the first proximal end part 71, and the first distal end part 72, as well as a plurality of protrusions 75 disposed on one side and the other side of the first main shaft 70. Five sets of the protrusions 75 are disposed in the first main shaft 70 at intervals in the axial direction with the slits 700 and 701 located between each set of the protrusions 75. Of the protrusions 75, the axial dimensions of the first and second protrusions 751 and 752 from the proximal end side are smaller than those of the third, fourth, and fifth protrusions 753, 754, and 755 in the axial direction from the proximal end side.

Each of the protrusions 75 guides the axial movement of the second shaft 8 relative to the first shaft 7 when the second shaft 8 moves relative to the first shaft 7 by the biasing force of the biasing member 4. Each of the protrusions 75 restricts the circumferential movement of the second shaft 8 in the state where the movement of the second shaft 8 relative to the first shaft 7 by the biasing force of the biasing member 4 is restricted. The protrusions 751, 752, 753, 754, and 755 respectively have guide surfaces 7510, 7520, 7530, 7540, and 7550 facing the slits 700 and 701 sides, which abut against a portion of the second shaft 8, thereby guiding the axial movement of the second shaft 8 and restricting the circumferential movement of the second shaft 8. The guide surfaces 7510, 7520, 7530, 7540, and 7550 of the protrusions 75 are flat surfaces.

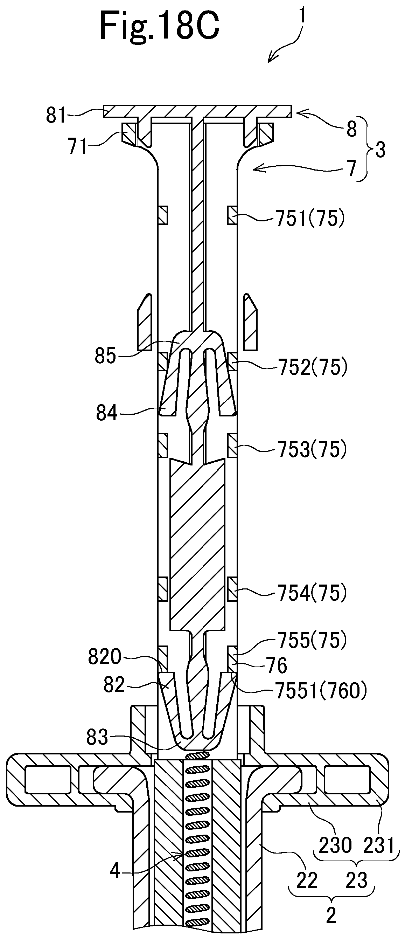

The third, fourth, and fifth protrusions 753, 754, and 755 from the proximal end side in the axial direction of the protrusions 75 include the restriction locking parts 76 that restrict the movement of the second shaft 8 relative to the first shaft 7 by the biasing force of the biasing member 4. In this embodiment, the distal end parts of the protrusions 753, 754, and 755 each form the restriction locking part 76. The distal end surfaces 7531, 7541, and 7551, which are the surfaces facing the distal end sides of the protrusions 753, 754, and 755, each form the distal end surface 760 of the restriction locking part 76 (see Fig. 18C, Fig. 20C, and Fig. 22C). The restriction locking parts 76 restrict the movement of the second shaft 8 relative to the first shaft 7 by the biasing force, by having their distal end surfaces 760 abutting against a portion of the second shaft 8. The distal end surface 760 is a flat surface.

A plurality of the restriction locking parts 76 are arranged at intervals in the axial direction, and in this embodiment, three sets are disposed (see Fig. 17C). Specifically, the restriction locking parts 76 are composed of three sets of the main-shaft restriction locking parts 76 that are arranged in the axial direction of the first main shaft 70 at intervals in the axial direction. In this embodiment, three sets of the restriction locking parts 76 are arranged at intervals in the axial direction, each set being located at positions 180° apart in the circumferential direction of the first shaft 7.

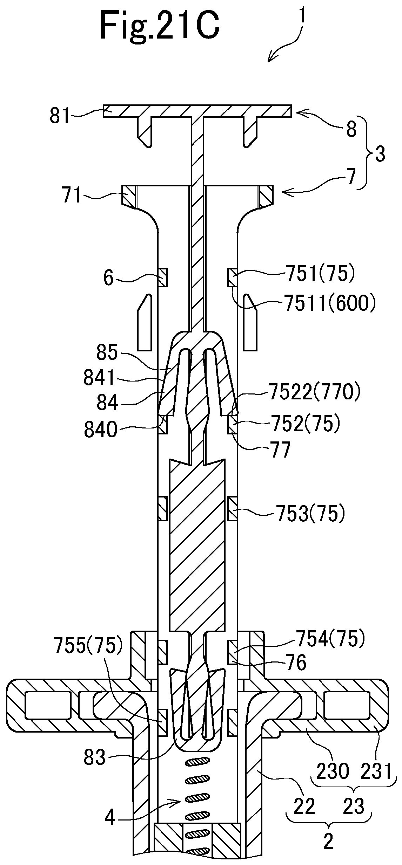

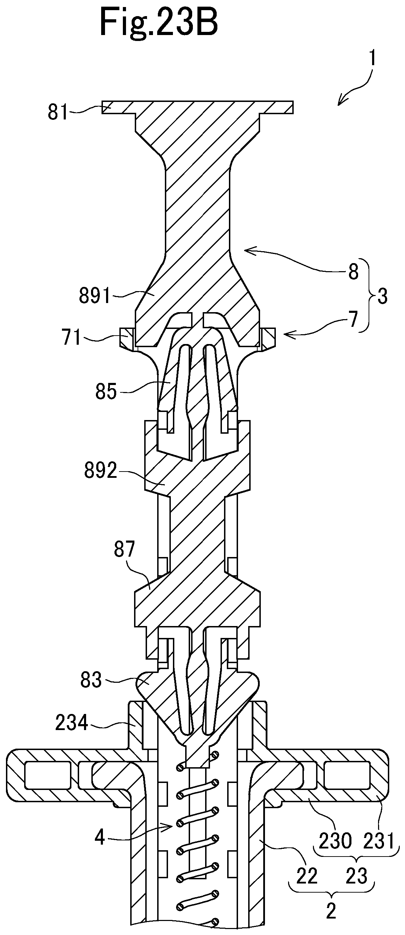

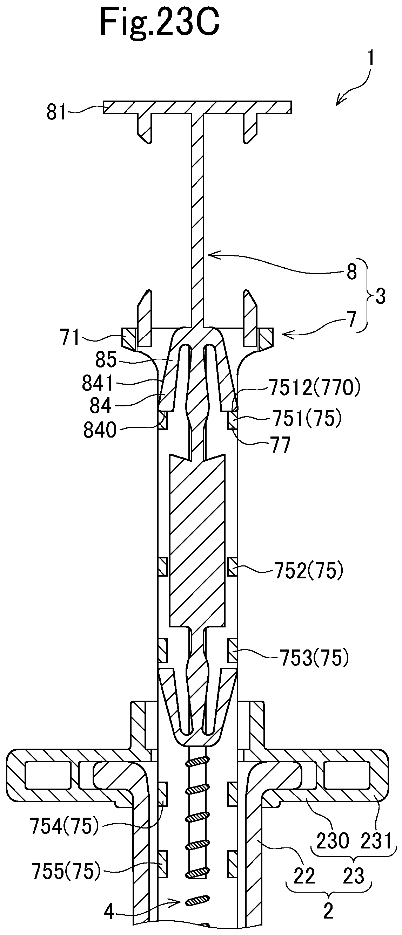

The first and second protrusions 751 and 752 from the proximal end side include the push-in locking parts 77, to which the second shaft 8 is locked, when the plunger 3 is pushed into the barrel 2. Two sets of the push-in locking parts 77 are disposed in the axial direction. In this embodiment, the proximal end parts of the protrusions 751 and 752 form the push-in locking parts 77. The proximal end surfaces 7512 and 7522, which are the surfaces facing the proximal end sides of the protrusions 751 and 752, each form the proximal end surface 770 of the push-in locking part 77 (Fig. 21C and Fig. 23C). The proximal end surfaces 770 of the push-in locking parts 77 lock to a portion of the second shaft 8, thereby restricting the axial movement of the second shaft 8 relative to the first shaft 7 toward the distal end, and enabling the plunger 3 to be pushed into the barrel 2. The proximal end surfaces 770 of the push-in locking parts 77 are flat surfaces.

Two sets of the push-in locking parts 77 are arranged at intervals in the axial direction of the first main shaft 70, while those of each set are located respectively on both sides with the central axis of the first main shaft 70 therebetween (see Fig. 17C). Specifically, two sets of the push-in locking parts 77 are arranged at intervals in the axial direction of the first main shaft 70, while those of each set are located at positions 180° apart in the circumferential direction of the first main shaft 70. In this embodiment, the push-in locking parts 77 in each set are located on both sides with the central axis of the first main shaft 70 therebetween, and the two sets of the main shaft push-in locking parts 77 are arranged in the axial direction.

In this syringe 1, when in the first discharge of the medical liquid, the first proximal end part 71 forms a push-in locking part 77 that locks to a portion of the second proximal end part 81.

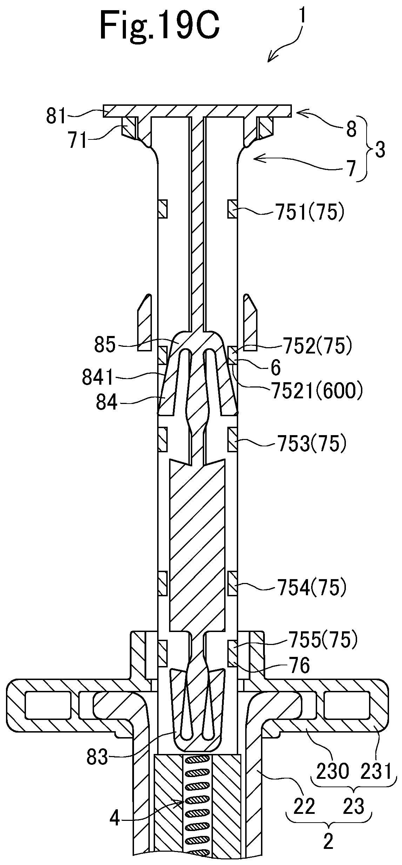

The first and second protrusions 751 and 752 from the proximal end side include the push-in guide parts 6. The distal end parts of the protrusions 751 and 752 each form the push-in guide part 6. The distal end surfaces 7511 and 7521, which are the surfaces facing the distal end sides of the protrusions 751 and 752, each form the distal end surface 600 of the push-in guide part 6 (see Fig. 19C and Fig. 21C). The push-in guide part 6 guides a portion of the second shaft 8 from the push-in implementing position to the push-in withdrawn position by having the distal end surface 600 abutting against the portion of the second shaft 8.

Two sets of the push-in guide parts 6 are arranged at intervals in the axial direction of the first main shaft 70, while those of each pair are located respectively on both sides with the central axis of the first main shaft 70 therebetween. Specifically, two pairs of the push-in guide parts 6 are arranged at intervals in the axial direction, while those of each pair are located at positions 180° apart in the circumferential direction of the first main shaft 70. In this embodiment, the push-in guide parts 6 are composed of two sets of the main-shaft push-in guide parts 6 arranged at intervals in the axial direction of the first main shaft 70, while those in each pair are located on both sides with the central axis of the first main shaft 70 therebetween.

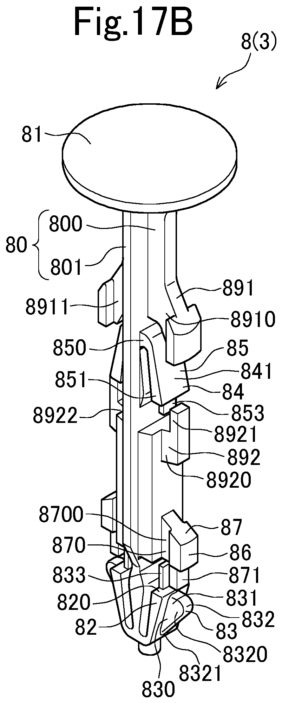

In this embodiment, as shown in Fig. 17B, the second shaft 8 includes the second main shaft 80 and the second proximal end part 81, as well as the restriction part 83, the distal end of which is the starting end 830 and the proximal end of which is the free end 831. The restriction part 83 is configured to be pivotally movable about the starting end 830 between a restriction withdrawn position where the free end 831 is located radially inward and a restriction implementing position where the free end 831 is located radially outward. The second shaft 8 includes a push-in part 85 that includes a push-in locked part 84 that is locked to the push-in locking part 77 of the first shaft 7 when the plunger 3 is pushed in. The second shaft 8 includes the discharge completion part 87 that includes the discharge completion locked part 86 that is locked to the discharge completion locking part 24 when the discharge of the medical liquid is completed, and the projection parts 891 and 892.

In this second shaft 8, a pair of the restriction parts 83 are disposed at positions opposite each other with the central axis in the circumferential direction of the second main shaft 80 therebetween. Similarly, the push-in part 85, the discharge completion part 87, and the projection parts 891 and 892 are each provided in pairs at positions opposite each other with the circumferential central axis of the second main shaft 80 therebetween. In this second shaft 8, the projection part 891, the push-in part 85, the projection part 892, the discharge completion part 87, and the restriction part 83 are arranged in order from the proximal end side toward the distal end side in the axial direction.

In this embodiment, the second main shaft 80 includes a second main wall part 800 having a substantially rectangular plate shape and extending along the axial direction, and a second sub-wall part 801 extending orthogonal to the second main wall part 800 through the center in the width direction of the second main wall part 800. The second main wall part 800 has a shape in which the end part on the radially outer side is partially cut out, and the remaining portion forms the projection part 891, the push-in part 85, the projection part 892, the discharge completion part 87, and the restriction part 83. The second sub-wall part 801 fits into the first shaft 7. This fitting restricts the circumferential movement of the second shaft 8 in the state where the movement of the second shaft 8 relative to the first shaft 7 by the biasing force of the biasing member 4 is restricted. This fitting restricts the circumferential movement of the second shaft 8 when the plunger 3 is pushed in or when the second shaft 8 moves in the axial direction relative to the first shaft 7 by the biasing force.

The restriction part 83 includes a restriction locked part 82 at the free end 831 that, in the restriction implementing position, is locked to the restriction locking part 76 of the first shaft 7 by the biasing force of the biasing member 4. The restriction part 83 is disposed on the second main shaft 80. The proximal end part of the restriction part 83 forms the restriction locked part 82. When the restriction part 83 is in the restriction implementing position, the restriction locked part 82 is locked to the restriction locking part 76 (see Fig. 18C, Fig. 20C, and Fig. 22C). When the restriction part 83 is in the restriction withdrawn position, the restriction locked part 82 is not locked to the restriction locking part 76.

In this embodiment, one set of the restriction locked parts 82 are disposed with the central axis of the second main shaft 80 therebetween (see Fig. 17B). The surfaces facing the proximal end side of the restriction part 83, specifically the proximal end surfaces 820 of the restriction locked parts 82, abut against the distal end surfaces 760 of the restriction locking part 76 of the first shaft 7, thereby restricting the movement of the second shaft 8 relative to the first shaft 7 by the biasing force (see Fig. 18C, Fig. 20C, and Fig. 22C). The proximal end surfaces 820 of the restriction locked parts 82 are flat surfaces.

The restriction part 83 includes a restriction guided part 832 disposed between a pair of the restriction locked parts 82 (see Fig. 17B). The restriction part 83 includes the restriction extension part 833 that extends further toward the proximal end from the end part on the radially inner side of each of the restriction locked parts 82.

The restriction guided part 832 is a part that guides the axial movement of the second shaft 8 relative to the first shaft 7 when the second shaft 8 moves relative to the first shaft 7 by the biasing force of the biasing member 4.

In this embodiment, the restriction guided part 832 abuts against the protrusions 754 and 755 of the first shaft 7, thereby guiding the axial movement of the second shaft 8. Specifically, the outer surface 8320 of the restriction guided part 832, which faces in the circumferential direction of the second shaft 8, abuts against the guide surfaces 7540 and 7550 of the protrusions 754 and 755, which face the slits 700 and 701 sides, thereby guiding the axial movement of the second shaft 8. The outer surface 8320 of the restriction guided part 832 is a flat surface.

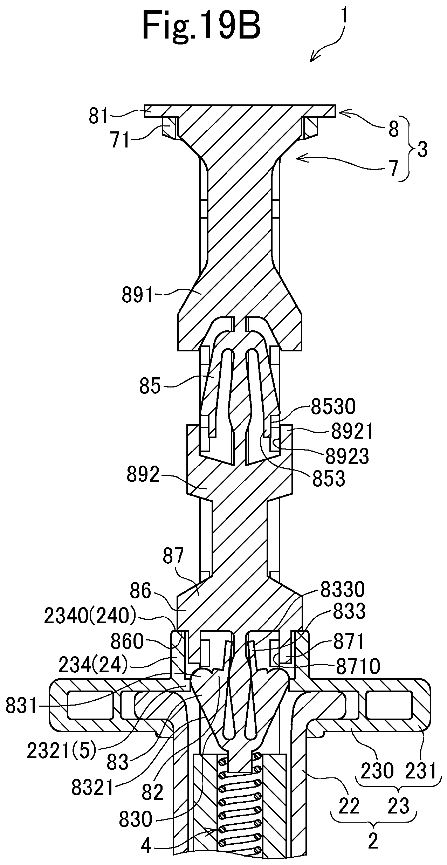

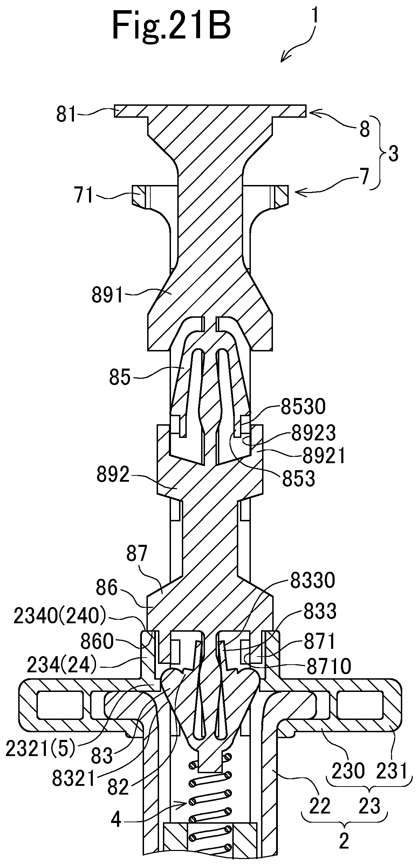

The restriction extension part 833 is a stopper that prevents the restriction part 83 from opening radially outward when the second shaft 8 moves relative to the first shaft 7 by the biasing force of the biasing member 4, and in the state where the movement of the second shaft 8 relative to the first shaft 7 is restricted. The restriction extension part 833 is located radially inward of a portion of the discharge completion part 87. In this embodiment, the restriction extension part 833 is a rib that extends axially from the end part on the radially inner side of the restriction locked part 82 toward the proximal end. The outer surface 8330 located radially outward of the restriction extension part 833 is a flat surface (see Fig. 19B and Fig. 21B).

The push-in part 85 has a starting end 850 at its proximal end and a free end 851 at its distal end, and is configured to be pivotally movable around the starting end 850 as a starting point between a push-in withdrawn position where the free end 851 is located radially inward and a push-in implementing position where the free end 851 is located radially outward (see Fig. 17B). In this embodiment, the distal end part of the push-in part 85 forms the push-in locked part 84. The push-in locked part 84 is a main-shaft push-in locked part 84 disposed at the free end 851 of the push-in part 85, and is composed of the main-shaft push-in locked part 84 that is locked to the main-shaft push-in locking part 77 of the first shaft 7 in the push-in implementing position.

A set of the push-in locked parts 84 are disposed respectively on both sides with the central axis of the second main shaft 80 therebetween. When the plunger 3 is pushed in, the surface of the push-in part 85 facing the distal end side, specifically the distal end surface 840 of the push-in locked part 84, abuts against the proximal end surface 770 of the push-in locking part 77 of the first shaft 7. Thereby, a force of moving the second shaft 8 toward the distal end is transmitted to the first shaft 7, causing the first shaft 7 to move together with the second shaft 8 (see Fig. 21C and Fig. 23C). The distal end surface 840 of the push-in locked part 84 is a flat surface.

The proximal end surface 841, which is a surface facing the proximal end side of the push-in locked part 84, is an inclined surface that is located more radially inward toward the proximal end. When the second shaft 8 moves toward the proximal end relative to the first shaft 7 by the biasing force of the biasing member 4, the proximal end surface 841 of the push-in locked part 84 abuts against the distal end surfaces 7511 and 7521, which are surfaces respectively facing the distal end sides of the first and second protrusions 751 and 752 from the proximal end side of the first shaft 7, causing the push-in part 85 to move from the push-in implementing position to the push-in withdrawn position (see Fig. 19C and Fig. 21C). The proximal end surface 841 of the push-in locked part 84 is a flat surface.

The push-in part 85 includes a push-in extension part 853 that extends further toward the distal end from the end part on the radially inner side of the push-in locked part 84 (see Fig. 17B).

The push-in extension part 853 is a stopper that prevents the push-in part 85 from opening radially outward when the second shaft 8 moves relative to the first shaft 7 by the biasing force of the biasing member 4, and when the user of the syringe 1 moves the second shaft 8 toward the distal end side. In this embodiment, the push-in extension part 853 is a rib that extends axially toward the distal end from the end part on the radially inner side of the push-in locked part 84. The push-in extension part 853 is located radially inward of a portion of the discharge completion part 87. The outer surface 8530 located radially outward of the push-in extension part 853 is a flat surface (see Fig. 19B and Fig. 21B).

The discharge completion part 87 is a stopper that restricts the plunger 3, which has moved a distance corresponding to the discharge for one separate portion, from further moving, by being locked to the discharge completion locking part 24 of the barrel 2. In this embodiment, the discharge completion part 87 is locked to the discharge completion locking part 24 at the time of completion of the first discharge and the second discharge of the medical liquid. The discharge completion part 87 is disposed on the second main shaft 80.

In this embodiment, the distal end part of the discharge completion part 87 forms the discharge completion locked part 86. A set of the discharge completion locked parts 86 are disposed with the central axis of the second main shaft 80 therebetween. The distal end surface 860 of the discharge completion locked part 86 abuts against the proximal end surface 240 of the discharge completion locking part 24 of the barrel 2, so that the movement of the second shaft 8 toward the distal end, that is, the discharge for one separate portion of the medical liquid by the user of the syringe 1, is completed. The distal end surface 860 of the discharge completion locked part 86 is a flat surface.

The discharge completion part 87 includes a discharge guided part 870, which is an end part on the radially outer side (see Fig. 17B). The discharge completion part 87 includes a discharge extension part 871 that extends as the distal end part on the distal end side of the discharge guided part 870.

The discharge guided part 870 is a part that guides the axial movement of the second shaft 8 relative to the first shaft 7 when the second shaft 8 moves relative to the first shaft 7 by the biasing force of the biasing member 4. The discharge guided part 870 is a part that restricts the circumferential movement of the second shaft 8 in the state where the movement of the second shaft 8 relative to the first shaft 7 by the biasing force of the biasing member 4 is restricted.

In this embodiment, the discharge guided part 870 abuts against the protrusions 753 and 754 of the first shaft 7, thereby guiding the axial movement of the second shaft 8 and restricting the circumferential movement of the second shaft 8. Specifically, the outer surface 8700 of the discharge guided part 870, which faces in the circumferential direction of the second shaft 8, abuts against the guide surfaces 7530 and 7540 of the protrusions 753 and 754, which face the slits 700 and 701 sides, thereby guiding the axial movement of the second shaft 8 and restricting the circumferential movement of the second shaft 8. The outer surface 8700 of the discharge guided part 870 is a flat surface.

The discharge extension part 871 is a stopper retaining part that presses the restriction extension part 833 of the restriction part 83 in order to prevent the restriction part 83 from opening radially outward when the second shaft 8 moves relative to the first shaft 7 by the biasing force of the biasing member 4, and when the movement of the second shaft 8 relative to the first shaft 7 is restricted. The discharge extension part 871 is located radially outward of the restriction extension part 833. In this embodiment, the discharge extension part 871 is a rib that extends axially toward the distal end as the end part on the distal side of the discharge guided part 870. An inner surface 8710 on the radially inner side of the discharge extension part 871 is a flat surface. The inner surface 8710 of the discharge extension part 871 abuts against the outer surface 8330 of the restriction extension part 833, thereby preventing the restriction part 83 from opening radially outward (see Fig. 19B and Fig. 21B).

The projection part 891 includes a projection guided part 8910 that is the end part on the radially outer side (see Fig. 17B).

The projection guided part 8910 is a part that guides the axial movement of the second shaft 8 relative to the first shaft 7 when the second shaft 8 moves relative to the first shaft 7 by the biasing force of the biasing member 4. The projection guided part 8910 is a part that restricts the circumferential movement of the second shaft 8 in the state where the movement of the second shaft 8 relative to the first shaft by the biasing force of the biasing member 4 is restricted.

In this embodiment, the projection guided part 8910 abuts against the protrusion 751 located on the most proximal side of the first shaft 7, thereby guiding the axial movement of the second shaft 8 and restricting the circumferential movement of the second shaft 8. Specifically, the outer surface 8911 of the projection guided part 8910, which faces in the circumferential direction of the second shaft 8, abuts against the guide surface 7510 of the protrusions 751, which faces the slits 700 and 701, thereby guiding the axial movement of the second shaft 8 and restricting the circumferential movement of the second shaft 8. The outer surface 8911 of the projection guide part 8910 is a flat surface.

The projection part 892 includes a projection guided part 8920, which is the end part on the radially outer side. The projection part 892 includes a projection extension part 8921 that extends as the end part on the distal side of the projection guided part 8920.

The projection guided part 8920 is a part that guides the axial movement of the second shaft 8 relative to the first shaft 7 when the second shaft 8 moves relative to the first shaft 7 by the biasing force of the biasing member 4. The projection guided part 8920 is a part that restricts the circumferential movement of the second shaft 8 in the state where the movement of the second shaft 8 relative to the first shaft by the biasing force of the biasing member 4 is restricted.

In this embodiment, the projection guided part 8920 abuts against the second and third protrusions 752 and 753 from the proximal end side of the first shaft 7, thereby guiding the axial movement of the second shaft 8 and restricting the circumferential movement of the second shaft 8. Specifically, an outer surface 8922 of the projection guided part 8920, which faces in the circumferential direction of the second shaft 8, abuts against the guide surfaces 7520 and 7530 of the protrusions 752 and 753, which face the one side slit 700 side, thereby guiding the axial movement of the second shaft 8 and restricting the circumferential movement of the second shaft 8. The outer surface 8922 of the projection guided part 8920 is a flat surface.

The projection extension part 8921 is a stopper retaining part that presses the push-in extension part 853 of the push-in part 85 in order to prevent the push-in part 85 from opening radially outward when the second shaft 8 moves relative to the first shaft 7 by the biasing force of the biasing member 4, and when the user of the syringe 1 moves the second shaft 8 toward the distal end. The projection extension part 8921 is located radially outward of the press-in extension part 853. In this embodiment, the projection extension part 8921 is a rib that extends axially toward the proximal end as the end part on the proximal side of the projection part 892. The inner surface 8923 on the radially inner side of the projection extension part 8921 is a flat surface. The inner surface 8923 of the projection extension part 8921 abuts against the outer surface 8530 on the radially outer side of the push-in extension part 853, thereby preventing the push-in part 85 from opening radially outward (see Fig. 19B and Fig. 21B).

In this syringe 1, when in the first discharge of the medical liquid, the second proximal end part 81 of the second shaft 8 forms the push-in locked part 84 that is locked to the first proximal end part 71 of the first shaft 7.

At a position at which the pushing-in for one separate portion is completed, the restriction release part 5 moves the restriction part 83 from the restriction implementing position to the restriction withdrawn position, thereby releasing the restriction locked part 82 from the locking engagement with the restriction locking part 76. In this embodiment, the restriction release part 5 is formed by the end part 2321 on the radially inner side of the body proximal end part 232 of the grip body part 230.

A detailed description will be hereinbelow made for the operation and various conditions in using the syringe 1.

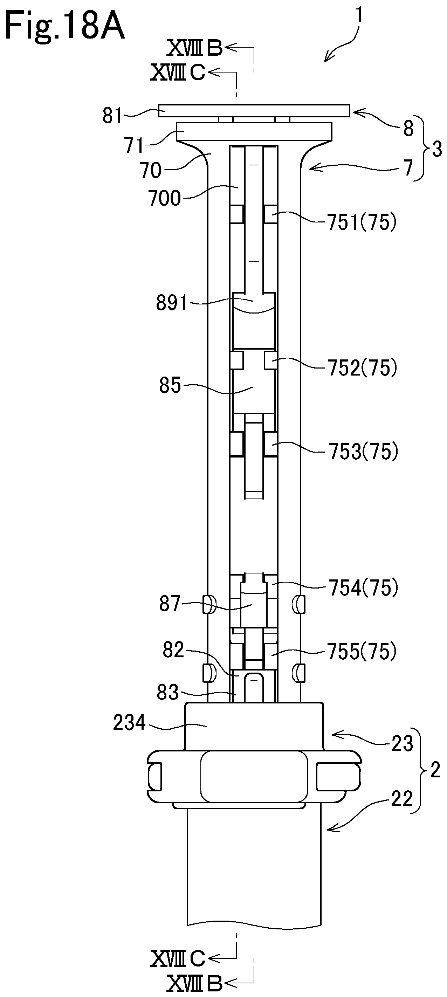

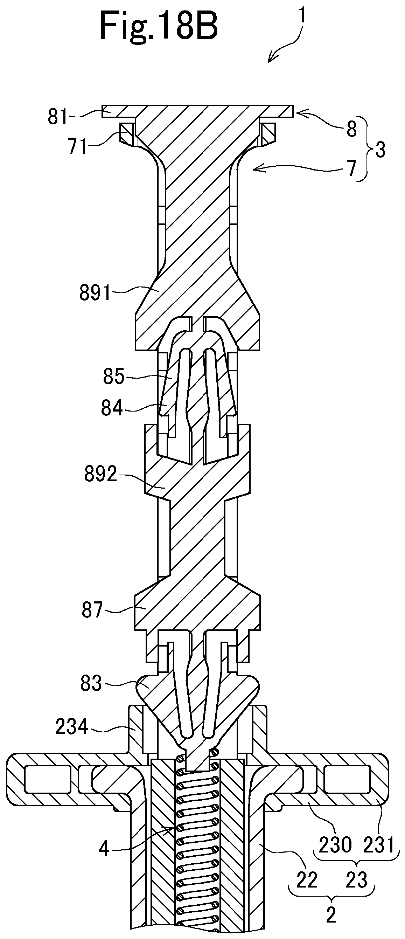

The initial state, as shown in Fig. 16 and Fig. 18A, is the state where the syringe 1 has not yet discharged a medical liquid. In the initial state, the second shaft 8 is biased toward the proximal end by the biasing force of the biasing member 4 relative to the first shaft 7, and the restriction part 83 is in the restriction implementing position (see Fig. 18B). The restriction locked part 82 of the restriction part 83 is locked to the restriction locking part 76 of the fifth protrusion 755 from the proximal end side of the first shaft 7 by the biasing force of the biasing member 4 (see Fig. 18C). Specifically, the proximal end surface 820 of the restriction locked part 82 of the restriction part 83 is locked to the distal end surface 7551 of the fifth protrusion 755 from the proximal end side. This locking restricts the movement of the second shaft 8 relative to the first shaft 7.

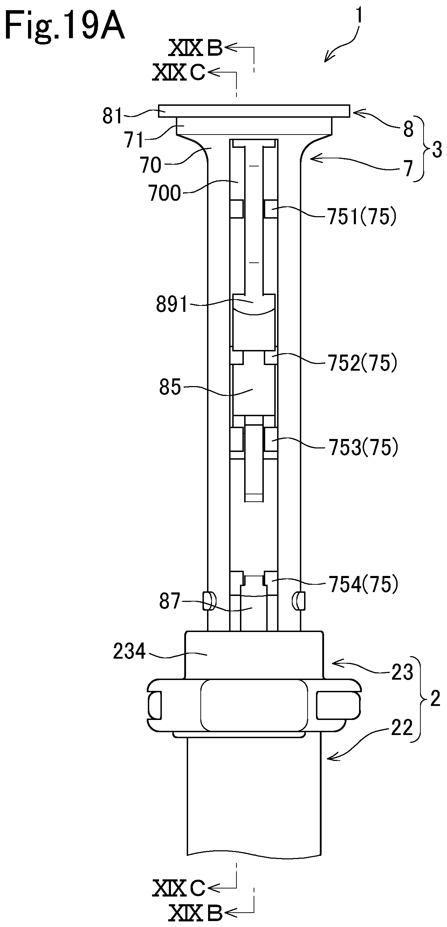

From the initial state, when the user of the syringe 1 pushes the second proximal end part 81 toward the distal end, the first shaft 7 is pushed toward the distal end together with the second shaft 8, as shown in Fig. 19A and Fig. 19B, and thereby the first discharge of the medical liquid is performed.

The first discharge of the medical liquid is completed at a position at which the discharge completion locked part 86 of the discharge completion part 87 is locked to the discharge completion locking part 24 of the barrel 2. Specifically, the first discharge of the medical liquid is completed at a position at which the distal end surface 860 of the discharge completion locked part 86 is locked to the proximal end surface 240 of the discharge completion locking part 24 (see Fig. 19B).

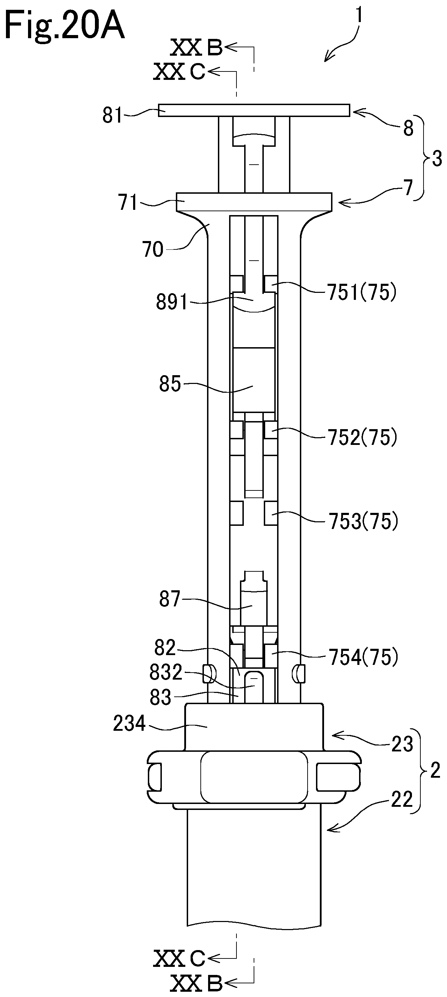

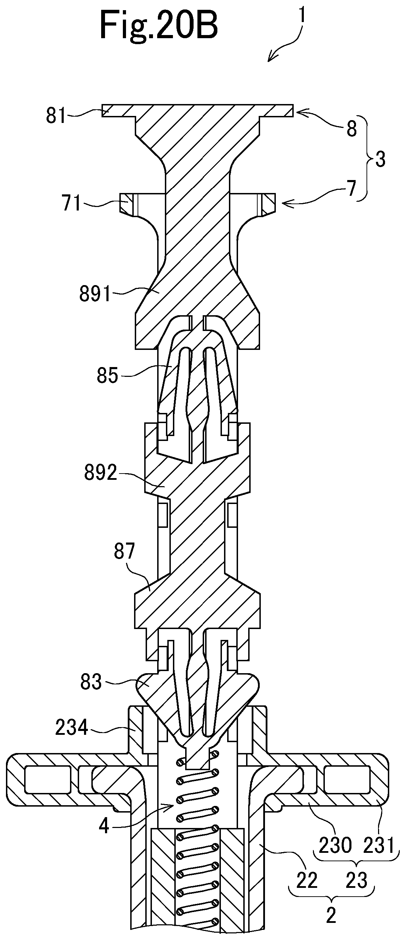

When in the first discharge of the medical liquid, the restriction part 83 of the second shaft 8 is moved from the restriction implementing position to the restriction withdrawn position by the restriction release part 5. Specifically, the restriction part 83 bends due to abutting of the end surface 8321 on the radially outer side of the restriction part 83 against the end part 2321 on the radially inner side of the body proximal end part 232 of the finger grip 23, so that the restriction part 83 is moved from the restriction implementing position to the restriction withdrawn position. Thereby, the restriction locked part 82 of the second shaft 8 also moves radially inward, and thereby the restriction locked part 82 is released from the locking engagement with the restriction locking part 76 of the first shaft 7. Then, the second shaft 8 moves toward the proximal end relative to the first shaft 7 by the biasing force of the biasing member 4, coming into the state immediately before the second discharge shown in Fig. 20A to Fig. 20C.

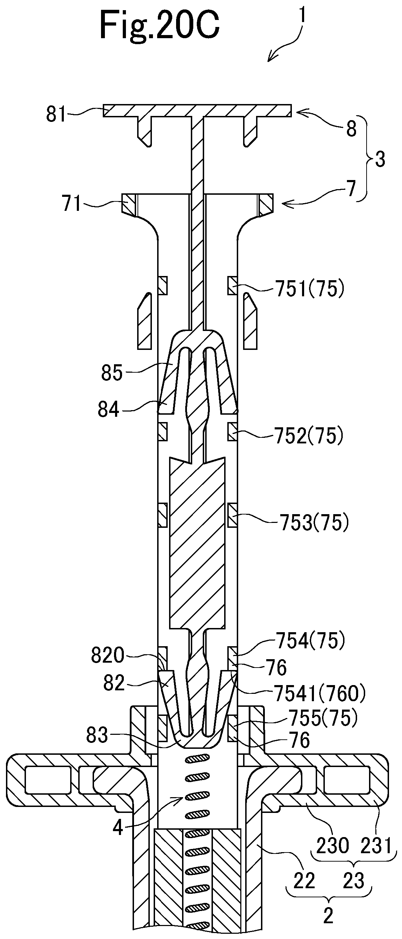

When in the first discharge, the restriction part 83 is moved to the restriction withdrawn position by the restriction release part 5, and then moves to the restriction implementing position between the restriction locking part 76 of the fifth protrusion 755 from the proximal end side of the first shaft 7, which was locked in the initial state, and the restriction locking part 76 of the fourth protrusion 754 from the proximal end side of the first shaft 7 (see Fig. 19C and Fig. 20C).

In the state immediately after the first discharge, the push-in part 85 of the second shaft 8 is located closer to the distal end than the second protrusion 752 from the proximal end side (see Fig. 19C). When coming into the state immediately before the second discharge after the second shaft 8 moves toward the proximal end relative to the first shaft 7 by the biasing force of the biasing member 4, the proximal end surface 841 of the push-in locked part 84 of the push-in part 85 abuts against the distal end surface 7521 of the second protrusion 752 from the proximal end side. Thereby, the push-in part 85 is pushed radially inward and comes into the push-in withdrawn position, passes the second protrusion 752 from the proximal end side toward the proximal end, and moves to a position between the first protrusion 751 from the proximal end side and the second protrusion 752 from the proximal end side (see Fig. 20C). Thus, the push-in part 85 is moved to the push-in withdrawn position by the distal end surface 7521 of the second protrusion 752 from the proximal end side, and then is located in the push-in implementing position at a position closer to the proximal end than the second protrusion 752 from the proximal end side.

When the second shaft 8 moves toward the proximal end relative to the first shaft 7 by the biasing force of the biasing member 4 in order to cause shifting from the state immediately after the first discharge to the state immediately before the second discharge, the restriction guided part 832 moves while being guided by the fifth protrusion 755 from the proximal end side. When in this movement, the projection guided part 8920 moves while being guided by the third protrusion 753 from the proximal end side.

When in this movement, the restriction part 83 is prevented from opening radially outward, and the push-in part 85 is prevented from opening radially outward (see Fig. 19B).

In the state immediately before the second discharge, the restriction locked part 82 of the restriction part 83 of the second shaft 8 is locked to the restriction locking part 76 of the fourth protrusion 754 from the proximal end side of the first shaft 7 by the biasing force of the biasing member 4 (see Fig. 20C). Specifically, the proximal end surface 820 of the restriction locked part 82 of the restriction part 83 locks to the distal end surface 7541 of the fourth protrusion 754 from the proximal end side. This locking restricts the movement of the second shaft 8 relative to the first shaft 7.

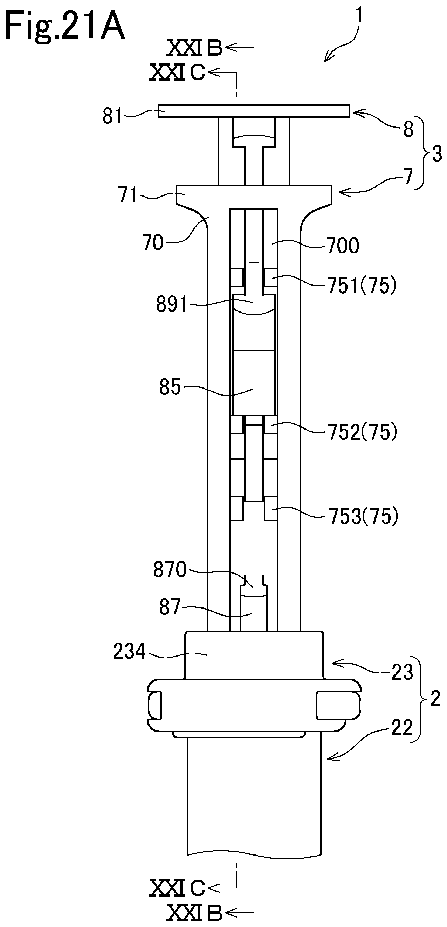

From the state immediately before the second discharge (see Fig. 20A to Fig. 20C), when the user of the syringe 1 pushes the second proximal end part 81 toward the distal end, as shown in Fig. 21A to Fig. 21C, the first shaft 7 is pushed toward the distal end together with the second shaft 8 in the state where the distal end surface 840 of the push-in locked part 84 abuts against the proximal end surface 770 of the push-in locking part 77 of the first shaft 7, and thereby the second discharge of the medical liquid is performed. Specifically, the distal end surface 840 abuts against the proximal end surface 7522 of the second protrusion 752 from the proximal end side (see Fig. 21C).

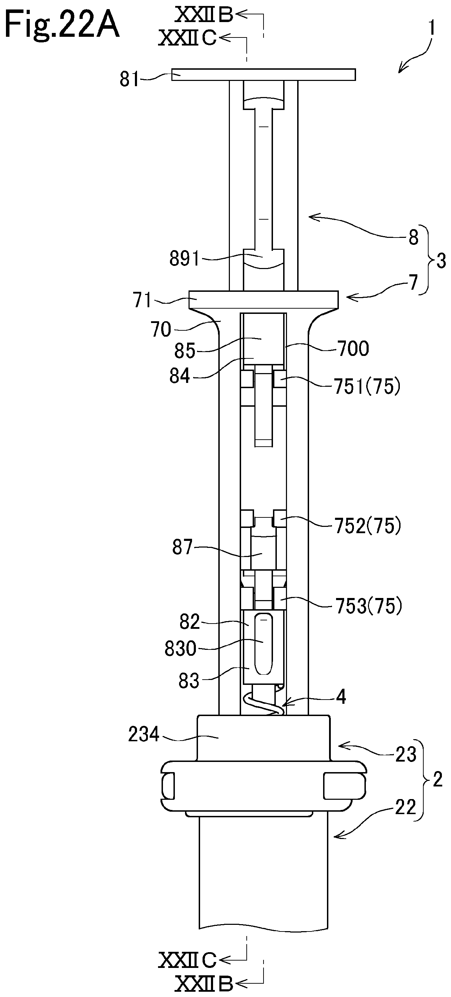

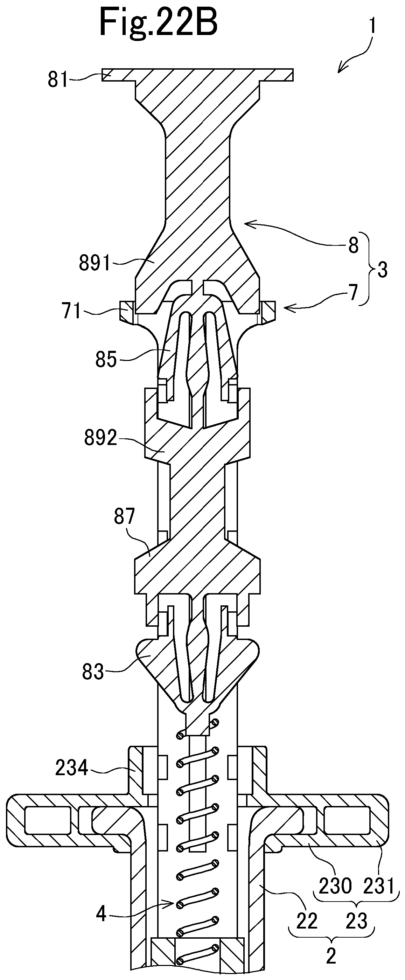

The second discharge of the medical liquid is completed at a position at which the distal end surface 860 of the discharge completion locked part 86 is locked to the proximal end surface 240 of the discharge completion locking part 24 (see Fig. 21B). When in the second discharge of the medical liquid, the restriction part 83 of the second shaft 8 bends due to abutting of the end surface 8321 on the radially outer side of the restriction part 83 against the end part 2321 on the radially inner side of the body proximal end part 232 of the finger grip 23, so that the restriction part 83 is moved from the restriction implementing position to the restriction withdrawn position. Thereby, the restriction locked part 82 of the second shaft 8 also moves radially inward, and thereby the restriction locked part 82 is released from the locking engagement with the restriction locking part 76 of the first shaft 7. Thereby, the second shaft 8 moves toward the proximal end relative to the first shaft 7 by the biasing force of the biasing member 4, coming into the state immediately before the third discharge shown in Fig. 22A to Fig. 22C.

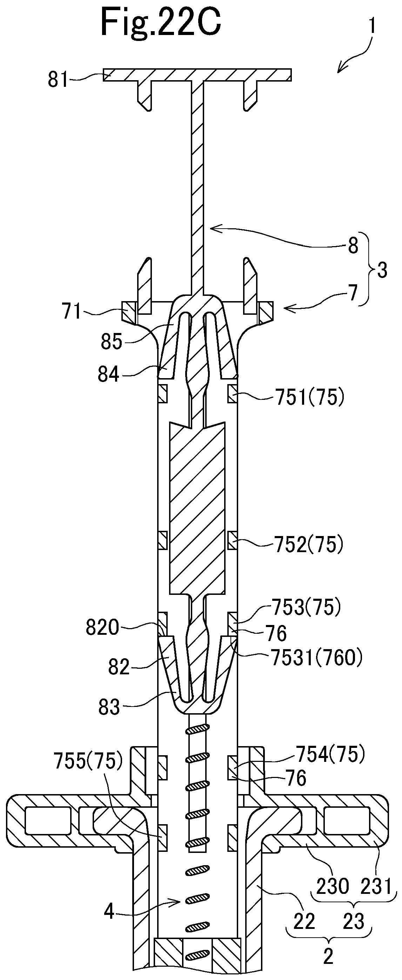

When in the second discharge, the restriction part 83 is moved to the restriction withdrawn position by the restriction release part 5, and then moves to the implementing position between the restriction locking part 76 of the fourth protrusion 754 from the proximal end side of the first shaft 7, which was locked immediately before the second discharge, and the restriction locking part 76 of the third protrusion 753 from the proximal end side (see Fig. 21C and Fig. 22C).

Further, when coming into the state immediately before the third discharge, the proximal end surface 841 of the push-in locked part 84 of the push-in part 85 abuts against the distal end surface 7511 of the protrusion 751 located on the most proximal side of the first shaft 7. Thereby, the push-in part 85 is pushed radially inward to the push-in withdrawn position, passes the protrusion 751 toward the proximal end, and moves toward the proximal end of the protrusion 751 located on the most proximal side.

When the second shaft 8 moves toward the proximal end relative to the first shaft 7 by the biasing force of the biasing member 4 in order to cause shifting from the state immediately after the second discharge to the state immediately before the third discharge, the restriction guided part 832 is guided by the fourth protrusion 754 from the proximal end side, and the second shaft 8 moves along the axial direction (see Fig. 17B and Fig. 21A). When in this movement, the discharge guided part 870 is guided by the third protrusion 753 from the proximal end side, and the second shaft 8 moves along the axial direction. When in this movement, the projection guided part 8910 is guided by the protrusion 751 located on the most proximal side, and thereby the second shaft 8 moves along the axial direction. When in this movement, the projection guided part 8920 is guided by the second protrusion 752 from the proximal end side, and the second shaft 8 moves along the axial direction.

Furthermore, when in this movement, the restriction part 83 is prevented from opening radially outward, and the push-in part 85 is prevented from opening radially outward (see Fig. 21B).

In the state immediately before the third discharge, the second shaft 8 is held in the state where the restriction locked part 82 of the restriction part 83 is locked by the biasing force of the biasing member 4 to the restriction locking part 76 of the third protrusion 753 from the proximal end side of the first shaft 7 (see Fig. 22C). Specifically, the proximal end surface 820 of the restriction locked part 82 of the restriction part 83 is locked to the distal end surface 7531 of the third protrusion 753 from the proximal end side. This locking restricts the movement of the second shaft 8 relative to the first shaft 7.

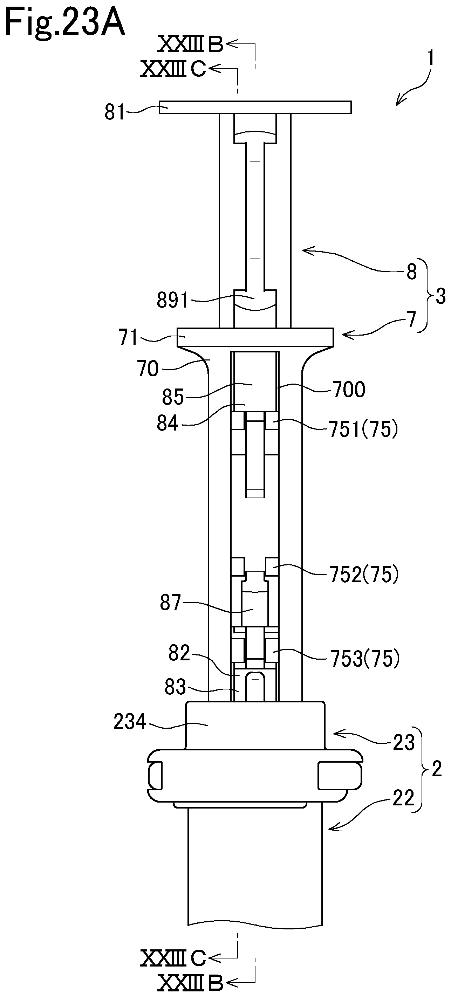

From the state immediately before the third discharge (see Fig. 22A to Fig. 22C), when the user of the syringe 1 pushes the second proximal end part 81 toward the distal end, as shown in Fig. 23A to Fig. 23C, the first shaft 7 is pushed toward the distal end together with the second shaft 8 in the state where the distal end surface 840 of the push-in locked part 84 abuts against the proximal end surface 7512 of the protrusion 751 located on the most proximal side, and thereby the third discharge of the medical liquid is performed.

The third discharge of the medical liquid is completed in the state where the piston 30 disposed at the first distal end part 72 of the first shaft 7 abuts against the distal end part of the body connecting part 222 of the barrel body 22.

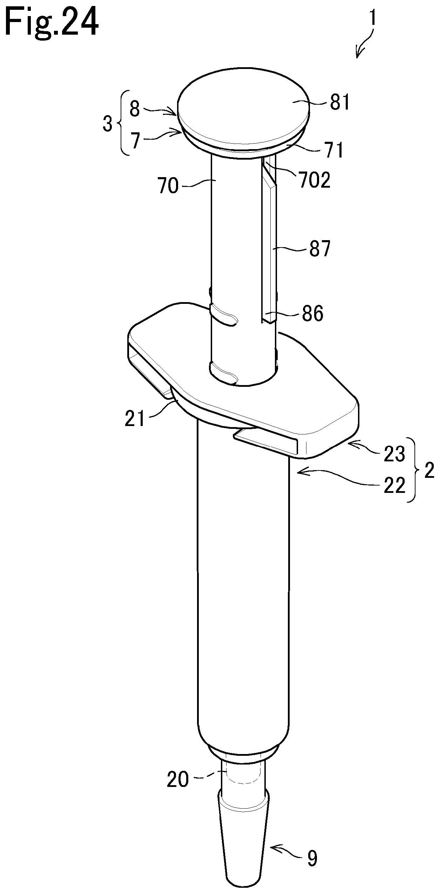

In the configuration shown in Fig. 1A to Fig. 9B, the second shaft 8 is configured such that the restriction part 83 is disposed on one side in the circumferential direction of the second shaft 8 and the push-in part 85 is disposed on the other side in the circumferential direction of the second shaft 8. However, both of the restriction part 83 and the push-in part 85 can be disposed on one side in the circumferential direction of the second shaft 8. A description will be hereinbelow given for the configuration in which the first shaft 7 and the second shaft 8 each have an axially asymmetrical shape, and the syringe 1 administers the medical liquid in two separate portions with reference to Fig. 24 to Fig. 29B.

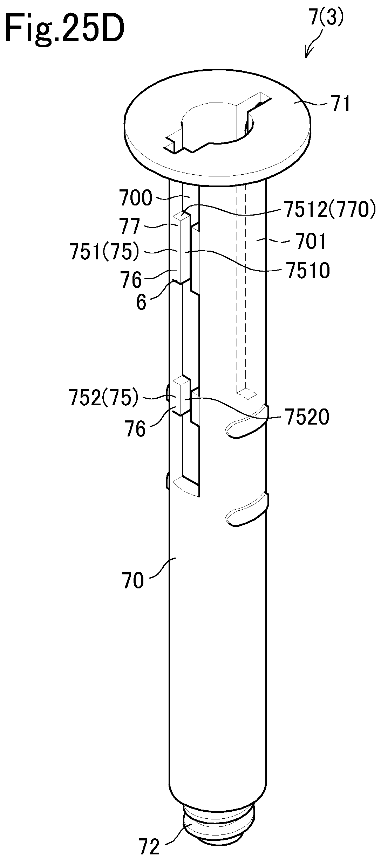

As shown in Fig. 25D, the first shaft 7 includes the first main shaft 70, the first proximal end part 71, and the first distal end part 72, as well as a plurality of protrusions 75 disposed on one side of the first main shaft 70. Two sets of the protrusions 75 are arranged at intervals in the axial direction of the first main shaft 70. One pair of the protrusions 75 are disposed on the first main shaft 70 with the one-side slit 700 therebetween. The projection dimensions of the protrusions 75 relative to the slit 700 are all the same as each other. The protrusion 751 disposed on the proximal end side has a larger axial dimension than the protrusion 752 disposed on the distal end side.

Two sets of the protrusions 75 each guide the axial movement of the second shaft 8 relative to the first shaft 7 when the second shaft 8 moves relative to the first shaft 7 by the biasing force of the biasing member 4. Two sets of the protrusions 75 restrict the circumferential movement of the second shaft 8 in the state where the movement of the second shaft 8 relative to the first shaft 7 by the biasing force of the biasing member 4 is restricted. The protrusions 751 and 752 respectively have guide surfaces 7510 and 7520 facing the slit 700 side, which abut against a portion of the second shaft 8, thereby guiding the axial movement of the second shaft 8 and restricting the circumferential movement of the second shaft 8. The guide surfaces 7510 and 7520 of the protrusions 751 and 752 are flat surfaces.

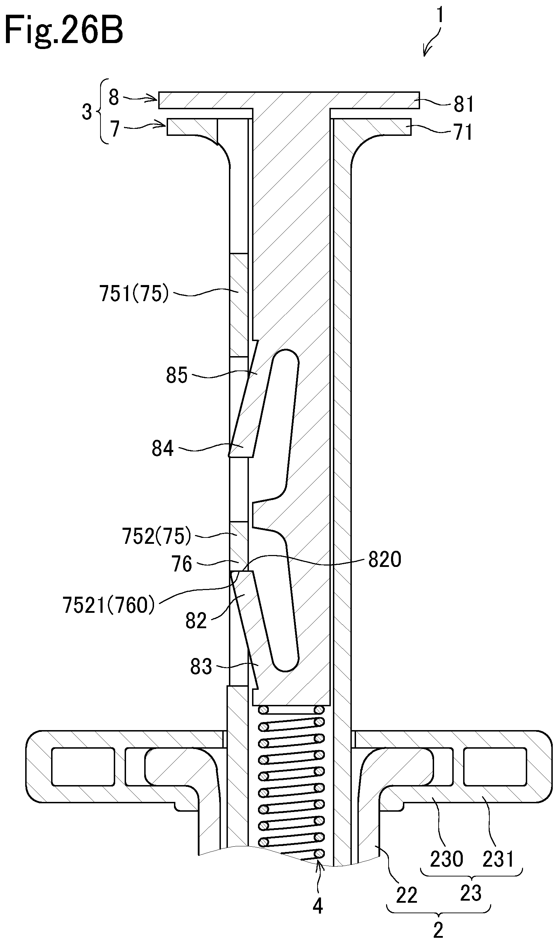

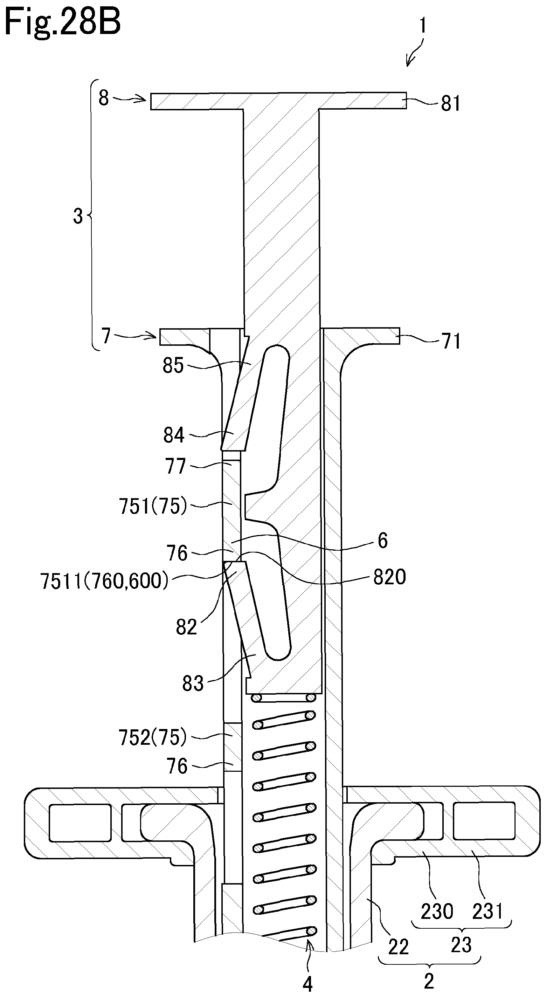

Each of the protrusions 751 and 752 includes a restriction locking part 76 that restricts the movement of the second shaft 8 relative to the first shaft 7 by the biasing force of the biasing member 4. In this embodiment, the distal end parts of the protrusions 751 and 752 each form the restriction locking part 76. The distal end surfaces 7511 and 7521, which are the surfaces facing the distal end sides of the protrusions 751 and 752, each form the distal end surface 760 of the restriction locking part 76 (see Fig. 26B and Fig. 28B). The restriction locking parts 76 restrict the movement of the second shaft 8 relative to the first shaft 7 by the biasing force, by having their distal end surfaces 760 abutting against a portion of the second shaft 8. The distal end surfaces 760 are flat surfaces. Two sets of the restriction locking parts 76 are arranged at intervals in the axial direction.

The protrusion 751 located on the proximal end side includes the push-in locking part 77, to which the second shaft 8 is locked, when the plunger 3 is pushed into the barrel 2 (see Fig. 25D). One set of the push-in locking parts 77 is provided. In this embodiment, the proximal end part of the protrusion 751 forms the push-in locking part 77. The proximal end surface 7512, which is a surface facing the proximal end side of the protrusion 751, forms the proximal end surface 770 of the push-in locking part 77. The proximal end surfaces 770 of the push-in locking parts 77 lock to a portion of the second shaft 8, thereby restricting the axial movement of the second shaft 8 relative to the first shaft 7 toward the distal end, and enabling the plunger 3 to be pushed into the barrel 2. The proximal end surfaces 770 of the push-in locking parts 77 are flat surfaces.

One set of the push-in locking parts 77 is provided. In this embodiment, the push-in locking part 77 is composed of a set of the main shaft push-in locking parts 77.

In this embodiment, the protrusion 751 located on the proximal end side includes a push-in guide part 6 that guides a portion of the second shaft 8 from the push-in implementing position to the push-in withdrawn position. The distal end part of the protrusion 751 forms the push-in guide part 6. The distal end surface 7511, which is a surface facing the distal end side of the protrusion 751, forms the distal end surface 600 of the push-in guide part 6 (see Fig. 28B). The push-in guide part 6 guides a portion of the second shaft 8 from the push-in implementing position to the push-in withdrawn position by having the distal end surface 600 abutting against the portion of the second shaft 8. The push-in guide part 6 is composed of a set of the main-shaft push-in guide parts 6 disposed on the first main shaft 70.

In this syringe 1, when in the first discharge of the medical liquid, the first proximal end part 71 forms a push-in locking part 77 that locks to a portion of the second proximal end part 81.

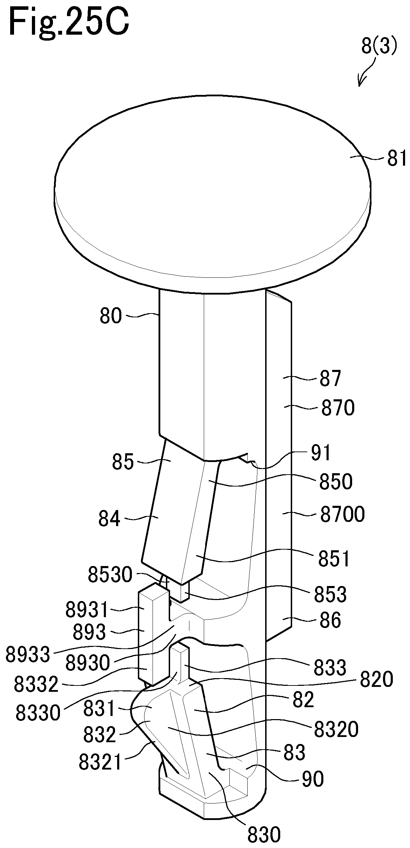

In this embodiment, the second shaft 8, as shown in Fig. 25C, includes the restriction part 83 that is pivotally movable about the starting end 830 as a starting point, and the push-in part 85 that includes the push-in locked part 84. The second shaft 8 includes the projection part 893, and the discharge completion part 87 that includes the discharge completion locked part 86. On one side of the second main shaft 80, the push-in part 85, the projection part 893, and the restriction part 83 are arranged in order from the proximal end side toward the distal end side in the axial direction. The discharge completion part 87 is disposed on the other side of the second main shaft 80. The second main shaft 80 is provided with the recess 90, in which the restriction part 83, when in the push-in withdrawn position, is fitted, and the recess 91, in which the push-in part 85, when in the push-in withdrawn position, is fitted.

The restriction part 83 includes a pair of the restriction locked parts 82 arranged in the circumferential direction, the restriction guided part 832, and the restriction extension part 833.

In this embodiment, the restriction guided part 832 abuts against the protrusion 752 on the distal end side of the first shaft 7, thereby guiding the axial movement of the second shaft 8 and restricting the circumferential movement of the second shaft 8. Specifically, the outer surface 8320 of the restriction guided part 832, which faces in the circumferential direction of the second shaft 8, abuts against the guide surface 7520 of the protrusion 752, which faces the one side slit 700 side, thereby guiding the axial movement of the second shaft 8 and restricting the circumferential movement of the second shaft 8. The outer surface 8320 of the restriction guided part 832 is a flat surface.

The restriction extension part 833 is a stopper that prevents the restriction part 83 from opening radially outward when the second shaft 8 moves relative to the first shaft 7 by the biasing force of the biasing member 4, and in the state where the movement of the second shaft 8 relative to the first shaft 7 is restricted. The restriction extension part 833 is located radially inward of a portion of the projection part 893. In this embodiment, the restriction extension part 833 is a rib that extends axially from the end part on the radially inner side of the restriction locked part 82 toward the proximal end. The outer surface 8330 located radially outward of the restriction extension part 833 is a flat surface.

The push-in part 85 has a starting end 850 on the proximal end side and a free end 851 on the distal end side, and is configured to be pivotally movable around the starting end 850 as a starting point between a push-in withdrawn position where the free end 851 is located radially inward and a push-in implementing position where the free end 851 is located radially outward.

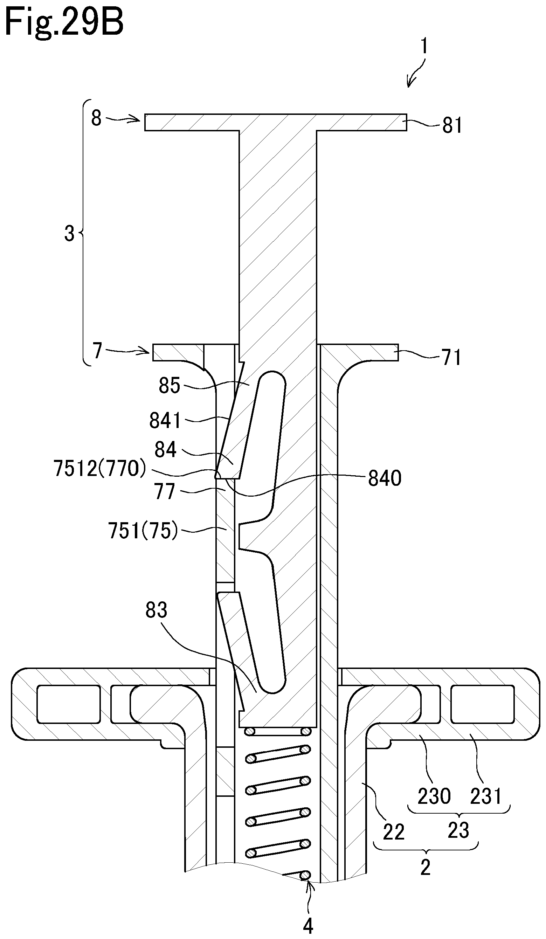

In this embodiment, the distal end part of the push-in part 85 forms the push-in locked part 84. The push-in locked part 84 is a main-shaft push-in locked part 84 disposed at the free end 851 of the push-in part 85, and is composed of the main-shaft push-in locked part 84 that is locked to the main-shaft push-in locking part 77 of the first shaft 7 when pushed in, in the push-in implementing position (see Fig. 29B). One push-in locked part 84 is disposed on the push-in part 85. When the plunger 3 is pushed in, the surface of the push-in part 85 facing the distal end side, specifically the distal end surface 840 of the push-in locked part 84, abuts against the proximal end surface 770 of the push-in locking part 77 of the first shaft 7, thereby transmitting a force, which is caused by the movement of the second shaft 8 toward the distal end, to the first shaft 7, and causing the first shaft 7 to move together with the second shaft 8. The distal end surface 840 of the push-in locked part 84 is a flat surface.

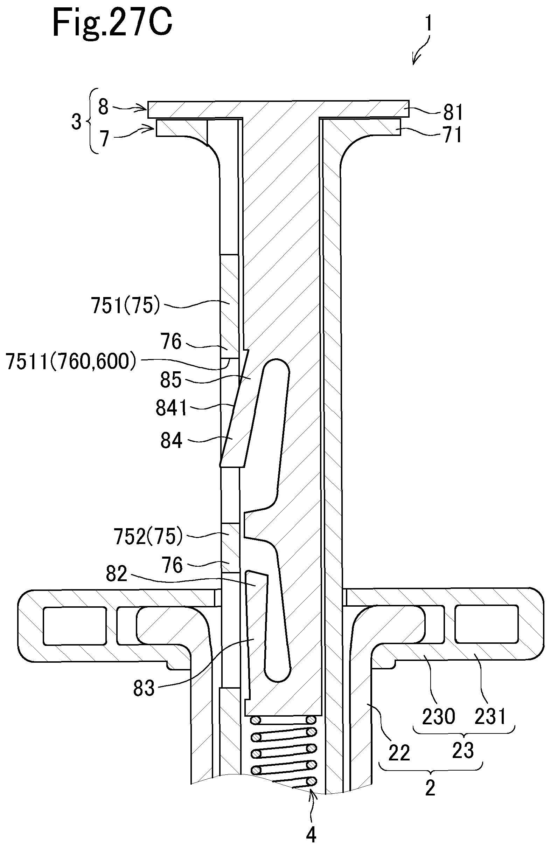

The proximal end surface 841, which is a surface facing the proximal end side of the push-in locked part 84, is an inclined surface that is located more radially inward toward the proximal end (see Fig. 27C). When the second shaft 8 moves toward the proximal end relative to the first shaft 7 by the biasing force of the biasing member 4, the proximal end surface 841 of the push-in locked part 84 abuts against the distal end surface 7511, which is a surface facing the distal end side of the protrusion 751 on the proximal end side of the first shaft 7, thereby causing the push-in part 85 to move from the push-in implementing position to the push-in withdrawn position. The proximal end surface 841 of the push-in locked part 84 is a flat surface.

In this syringe 1, when in the first discharge of the medical liquid, the second proximal end part 81 forms the push-in locked part 84 that is locked to the first proximal end part 71.

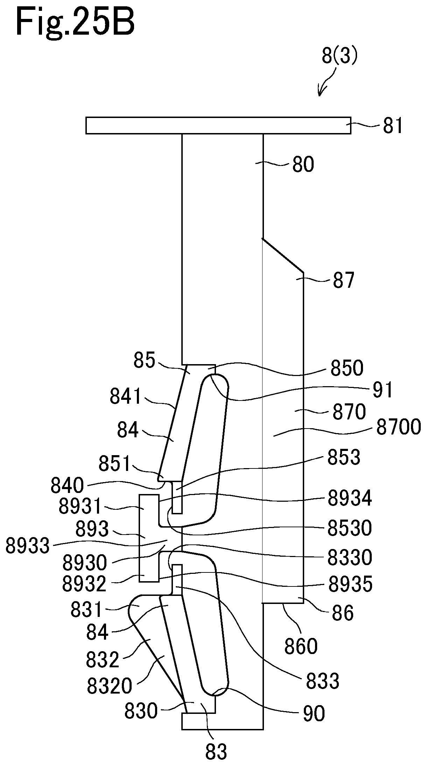

The push-in part 85 includes a push-in extension part 853 that extends further toward the distal end from the end part on the radially inner side of the push-in locked part 84 (see Fig. 25B and Fig. 25C).

The push-in extension part 853 is a stopper that prevents the push-in part 85 from opening radially outward when the second shaft 8 moves relative to the first shaft 7 by the biasing force of the biasing member 4, and when the user of the syringe 1 moves the second shaft 8 toward the distal end. In this embodiment, the push-in extension part 853 is a rib that extends axially toward the distal end from the end part on the radially inner side of the push-in locked part 84. The push-in extension part 853 is located radially inward of a portion of the projection part 893. The surface 8530 on the radially outer side of the push-in extension part 853 is a flat surface.

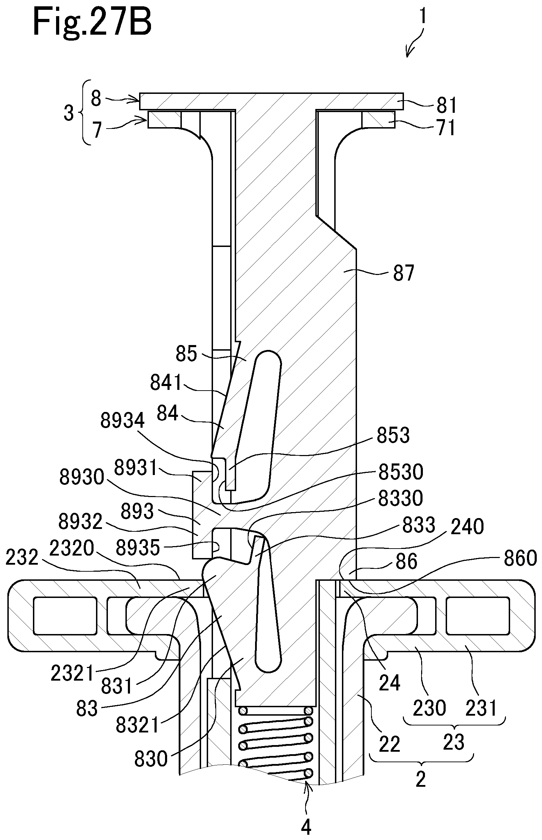

The discharge completion part 87 is a stopper that restricts the plunger 3 by being locked to the discharge completion locking part 24 of the barrel 2 when in the first discharge. In this embodiment, when the distal end surface 860 of the discharge completion part 87 abuts against the proximal end surface 240 of the discharge completion locking part 24 of the barrel 2, the movement of the second shaft 8 toward the distal end of the second shaft 8, that is, the first discharge of the medical liquid by the user of the syringe 1 is completed (see Fig. 27B). The distal end surface 860 of the discharge completion locked part 86 is a flat surface. The discharge completion locking part 24 is composed of the body proximal end part 232, and the proximal end surface 240 of the discharge completion locking part 24 is composed of the proximal end surface 2320, which is a surface facing the proximal end side of the body proximal end part 232.

The discharge completion part 87 includes the discharge guided part 870 that is the end part on the radially outer side (see Fig. 25B and Fig. 25C).

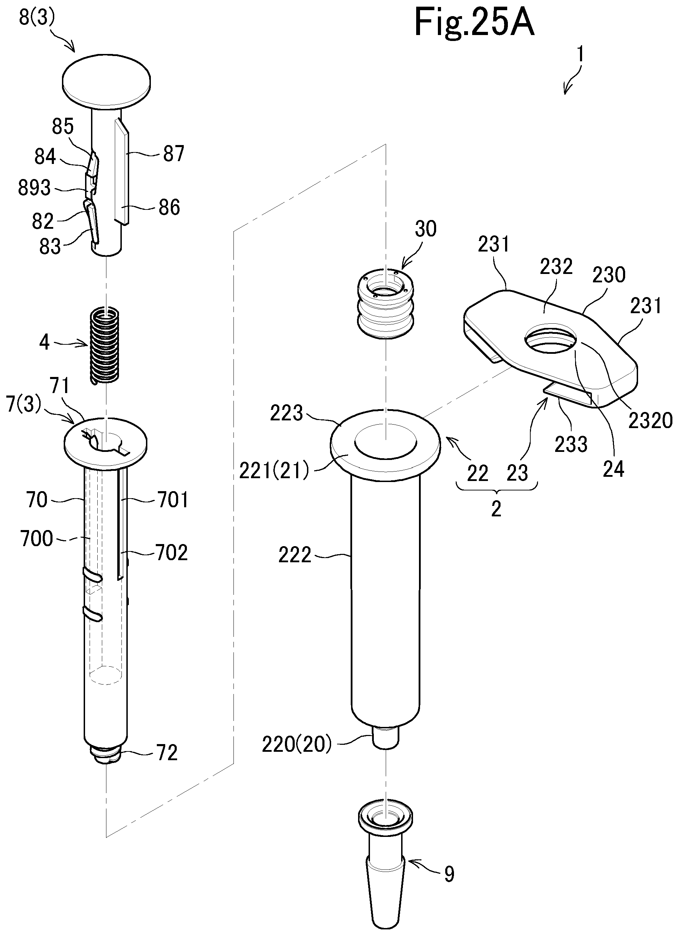

The discharge guided part 870 is a part that guides the axial movement of the second shaft 8 relative to the first shaft 7 when the second shaft 8 moves relative to the first shaft 7 by the biasing force of the biasing member 4. The discharge guided part 870 is a part that restricts the circumferential movement of the second shaft 8 in the state where the movement of the second shaft 8 relative to the first shaft 7 by the biasing force of the biasing member 4 is restricted. In this embodiment, the outer surface 8700 of the discharge guided part 870, which faces in the circumferential direction of the second shaft 8, abuts against the guide surface 702 (see Fig. 25A) that defines the other-side slit 701 of the first shaft 7, thereby guiding the axial movement of the second shaft 8 and restricting the circumferential movement of the second shaft 8.

The projection part 893 includes the projection guided part 8930 (see Fig. 25C). The projection part 893 includes a proximal-end-side projection extension part 8931 that extends as the end part on the proximal side of the projection guided part 8930, and a distal-end-side projection extension part 8932 that extends as the end part on the distal side of the projection guided part 8930.

The projection guided part 8930 is a part that guides the axial movement of the second shaft 8 relative to the first shaft 7 when the second shaft 8 moves relative to the first shaft 7 by the biasing force of the biasing member 4. The projection guided part 8930 is a part that restricts the circumferential movement of the second shaft 8 in the state where the movement of the second shaft 8 relative to the first shaft by the biasing force of the biasing member 4 is restricted.

In this embodiment, the projection guided part 8930 abuts against the protrusion 752 on the distal end side of the first shaft 7, thereby guiding the axial movement of the second shaft 8 and restricting the circumferential movement of the second shaft 8. Specifically, the outer surface 8933 of the projection guided part 8930, which faces in the circumferential direction of the second shaft 8, abuts against the guide surface 7520 of the protrusion 752, which faces the slit 700 side, thereby guiding the axial movement of the second shaft 8 and restricting the circumferential movement of the second shaft 8. The outer surface 8933 of the projection guided part 8930 is a flat surface.

The proximal-end-side projection extension part 8931 is a stopper retaining part that presses the push-in extension part 853 of the push-in part 85 in order to prevent the push-in part 85 from opening radially outward. The proximal-end-side projection extension part 8931 is located radially outward of the push-in extension part 853. In this embodiment, the proximal-end-side projection extension part 8931 is a rib that extends axially toward the proximal end as the end part on the proximal side of the projection guided part 8930. The inner surface 8934 on the radially inner side of the proximal-end-side projection extension part 8931 is a flat surface. The inner surface 8934 of the proximal-end-side projection extension part 8931 abuts against the outer surface 8530 on the radially outer side of the push-in extension part 853, thereby preventing the push-in part 85 from opening radially outward (see Fig. 27B).

The distal-end-side projection extension part 8932 is a stopper retaining part that presses the restriction extension part 833 of the restriction part 83 in order to prevent the restriction part 83 from opening radially outward. The distal-end-side projection extension part 8932 is located radially outward of the restriction extension part 833. In this embodiment, the distal-end-side projection extension part 8932 is a rib that extends axially toward the distal end as the end part on the distal side of the projection guided part 8930. The inner surface 8935 on the radially inner side of the distal-end-side projection extension part 8932 is a flat surface. The inner surface 8935 of the distal-end-side projection extension part 8932 abuts against the outer surface 8330 on the radially outer side of the restriction extension part 833, thereby preventing the restriction part 83 from opening radially outward.

The restriction release part 5 is composed of the end part 2321 on the radially inner side of the grip body part 230 of the finger grip 23, for example, the end part 2321 on the radially inner side of the body proximal end part 232 of the grip body part 230.

A detailed description will be hereinbelow given for the operation and various conditions in using the syringe 1.

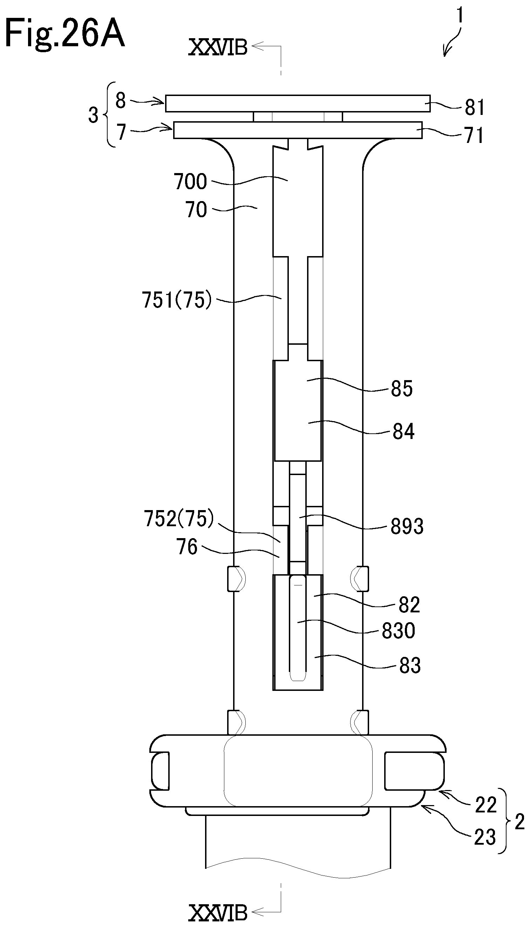

The initial state, as shown in Fig. 24 and Fig. 26A, is the state where the syringe 1 has not yet discharged the medical liquid. In the initial state, the restriction locked part 82 of the restriction part 83 of the second shaft 8 is locked to the restriction locking part 76 of the protrusion 752 on the distal end side of the first shaft 7 by the biasing force of the biasing member 4. Specifically, the proximal end surface 820 of the restriction locked part 82 of the restriction part 83 is locked to the distal end surface 7521 of the protrusion 752 on the distal end side (see Fig. 26B). This locking restricts the movement of the second shaft 8 relative to the first shaft 7.

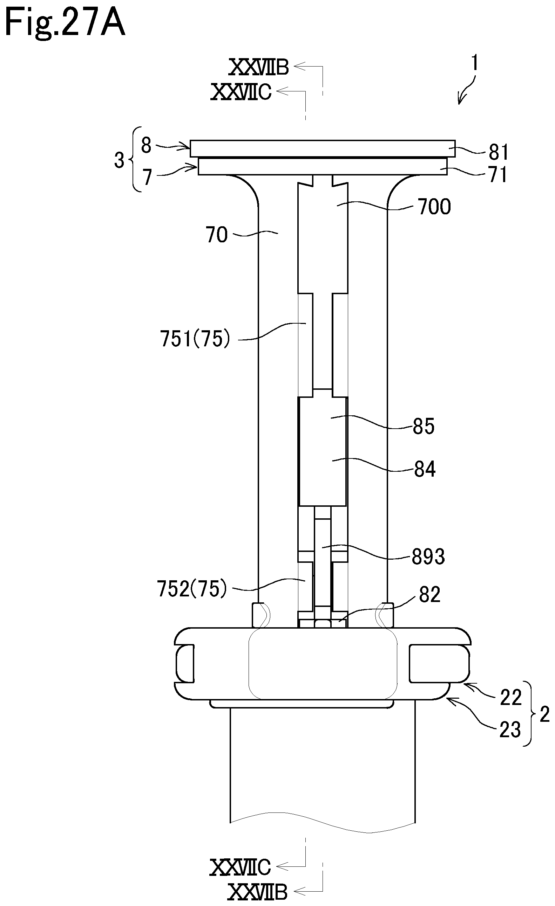

From the initial state, when the user of the syringe 1 pushes the second shaft 8 (specifically, the second proximal end part 81) toward the distal end, the first shaft 7 is pushed toward the distal end together with the second shaft 8, as shown in Fig. 27A to Fig. 27C, and thereby the first discharge of the medical liquid is performed.

The first discharge of the medical liquid is completed at a position at which the discharge completion locked part 86 of the discharge completion part 87 is locked to the discharge completion locking part 24 of the barrel 2 (see Fig. 27B). Specifically, the first discharge of the medical liquid is completed at a position at which the distal end surface 860 of the discharge completion locked part 86 is locked to the proximal end surface 240 of the discharge completion locking part 24.

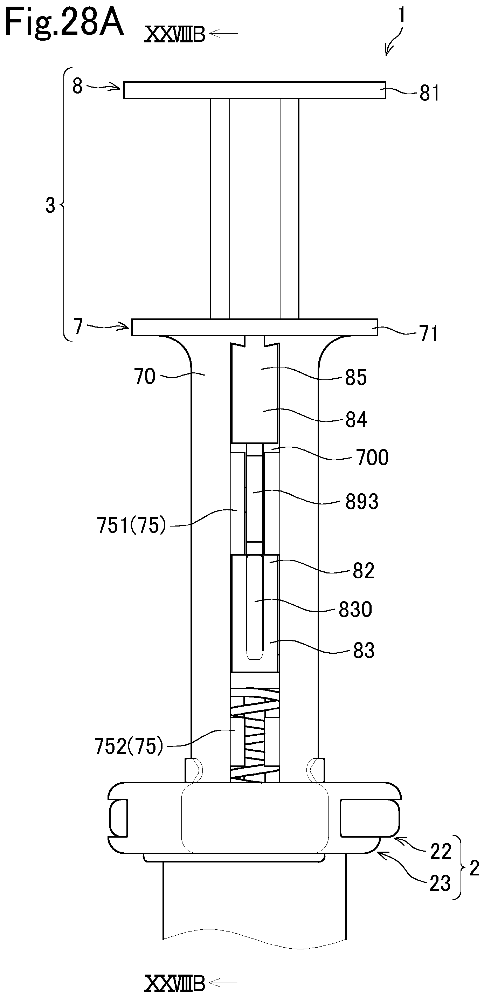

When in the first discharge of the medical liquid, the restriction part 83 of the second shaft 8 is moved from the restriction implementing position to the restriction withdrawn position by the restriction release part 5. Specifically, the restriction part 83 bends due to abutting of the end surface 8321 on the radially outer side of the restriction part 83 against the end part 2321 on the radially inner side of the body proximal end part 232 of the finger grip 23, so that the restriction part 83 is moved from the restriction implementing position to the restriction withdrawn position. Thereby, the restriction locked part 82 of the second shaft 8 also moves radially inward, and thereby the restriction locked part 82 is released from the locking engagement with the restriction locking part 76 of the first shaft 7. Then, the second shaft 8 moves toward the proximal end relative to the first shaft 7 by the biasing force of the biasing member 4, coming into the state immediately before the second discharge shown in Fig. 28A and Fig. 28B.

When in the first discharge, the restriction part 83 is moved to the restriction withdrawn position by the restriction release part 5, and then moves to the restriction implementing position between the restriction locking part 76 of the protrusion 752 on the distal end side of the first shaft 7, which was locked in the initial state, and the restriction locking part 76 of the protrusion 751 on the proximal end side (see Fig. 27C and Fig. 28B).

Immediately after the first discharge, the push-in part 85 of the second shaft 8 is located closer to the distal end than the protrusion 751 on the proximal end side (see Fig. 27C). When coming into the state immediately before the second discharge after the second shaft 8 moves toward the proximal end relative to the first shaft 7 by the biasing force of the biasing member 4, the proximal end surface 841 of the push-in locked part 84 of the push-in part 85 abuts against the distal end surface 7511 of the protrusion 751 on the proximal end side. Thereby, the push-in part 85 is pushed radially inward, coming into the press-in withdrawn position, so that the push-in part 85 passes the protrusion 751 on the proximal end side toward the proximal end and moves to a position closer to the proximal end than the protrusion 751 on the proximal end side (see Fig. 28B). Thus, the push-in part 85 is moved to the push-in withdrawn position by the protrusion 751 on the proximal end side, and then comes into the push-in implementing position at a position closer to the proximal end than the protrusion 751 on the proximal end side.

When the second shaft 8 moves toward the proximal end relative to the first shaft 7 by the biasing force of the biasing member 4 in order to cause shifting from the state immediately after the first discharge to the state immediately before the second discharge, the restriction guided part 832 moves while being guided by the protrusion 752 on the distal end side. When in this movement, the discharge guided part 870 moves while being guided by the guide surface 702 that defines the other-side slit 701 of the first main shaft 70. When in this movement, the projection guided part 8930 moves while being guided by the protrusion 752 on the distal end side. Further, when in this movement, the restriction part 83 is prevented from opening radially outward, and the push-in part 85 is prevented from opening radially outward (see Fig. 27B).

In the state immediately before the second discharge, the second shaft 8 is held in the state where the restriction locked part 82 of the restriction part 83 is locked to the restriction locking part 76 of the protrusion 751 on the proximal end side of the first shaft 7 by the biasing force of the biasing member 4 (see Fig. 28B). Specifically, the proximal end surface 820 of the restriction locked part 82 of the restriction part 83 is locked to the distal end surface 7511 of the protrusion 751 on the proximal end side. This locking restricts the movement of the second shaft 8 relative to the first shaft 7.

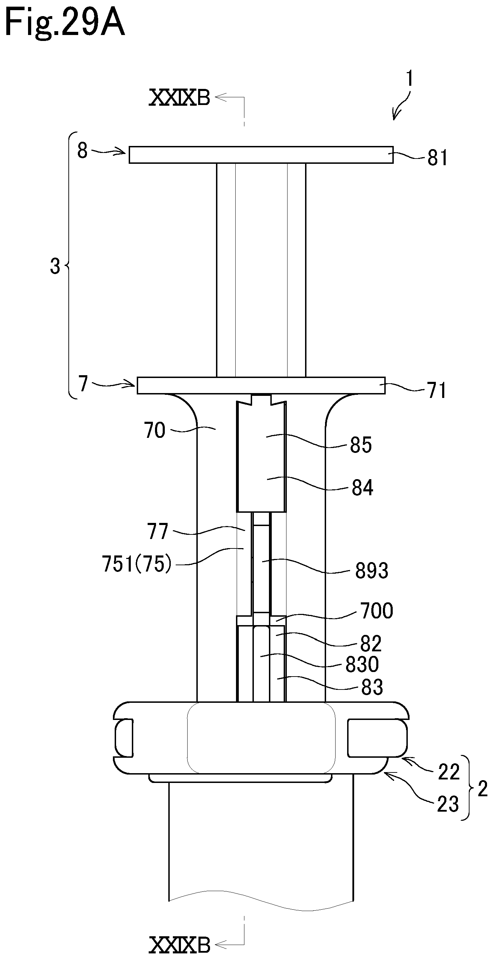

From the state immediately before the second discharge, when the user of the syringe 1 pushes the second proximal end part 81 toward the distal end, as shown in Fig. 29A and Fig. 29B, the first shaft 7 is pushed toward the distal end together with the second shaft 8, and thereby the second discharge of the medical liquid is performed.

The second discharge of the medical liquid is completed in the state where the piston 30, disposed at the first distal end part 72 of the first shaft 7, abuts against the distal end part of the body connecting part 222 of the barrel body 22.

In the above embodiment, the syringe 1 includes the barrel 2, the plunger 3, the biasing member 4, the restriction release part 5, the push-in guide part 6, and the discharge nozzle 9. However, it is sufficient for the syringe 1 to include at least the barrel 2, the plunger 3, the biasing member 4, and the restriction release part 5. For example, the syringe 1 may not include at least one of the push-in guide part 6 and the discharge nozzle 9. When the syringe 1 does not include the discharge nozzle 9, it is conceivable that the contents (e.g., medical liquid) may be discharged from the distal end part 20 of the barrel 2 in a linear fashion rather than in a mist.

In the above embodiment, the push-in locking part 77 of the first shaft 7 includes a plurality of the main-shaft push-in locking parts 77 disposed on the first main shaft 70, but they can be disposed at a different location on the first shaft 7. Although the push-in part 85 of the second shaft 8 is disposed on the second main shaft 80, it can be disposed at a different location on the second shaft 8.

In the above embodiment, when the user pushes the second proximal end part 81 toward the distal end at the time of discharging the medical liquid except when in the first discharge, the first shaft 7 is pushed toward the distal end together with the second shaft 8 in the state where the distal end surface 840 of the push-in locked part 84 abuts against the proximal end surface 770 of the push-in locking part 77 of the first shaft 7. However, at the times of all the discharges of the medical liquid, the first shaft 7 can be pushed toward the distal end together with the second shaft 8 in the state where the distal end surface 840 of the push-in locked part 84 abuts against the proximal end surface 770 of the push-in locking part 77 of the first shaft 7.

In the above embodiment, the discharge completion locking part 24 is disposed on the finger grip 23, but it can be disposed on any locations other than the finger grip 23. For example, the discharge completion locking part 24 can be disposed on the barrel body 22.

In the above embodiment, the discharge completion part 87 is locked to the discharge completion locking part 24 when the discharge of the medical liquid is completed, except for the last discharge. However, the discharge completion part 87 can be locked to the discharge completion locking part 24 at the times of all the completions of the discharges of the medical liquid.

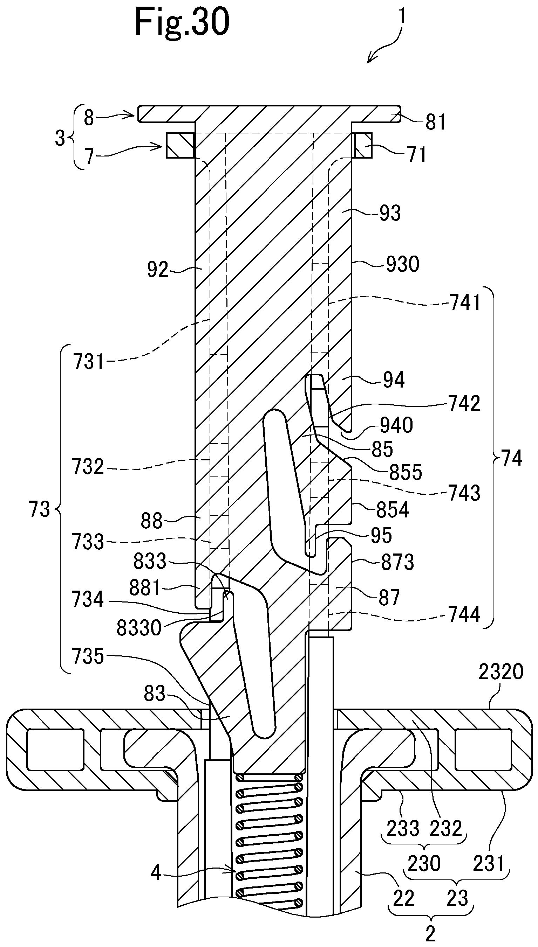

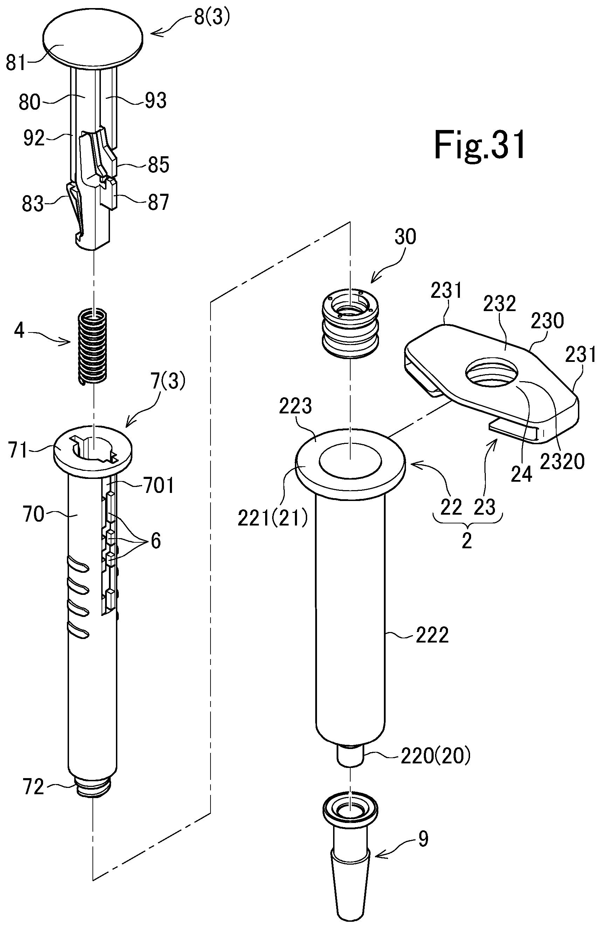

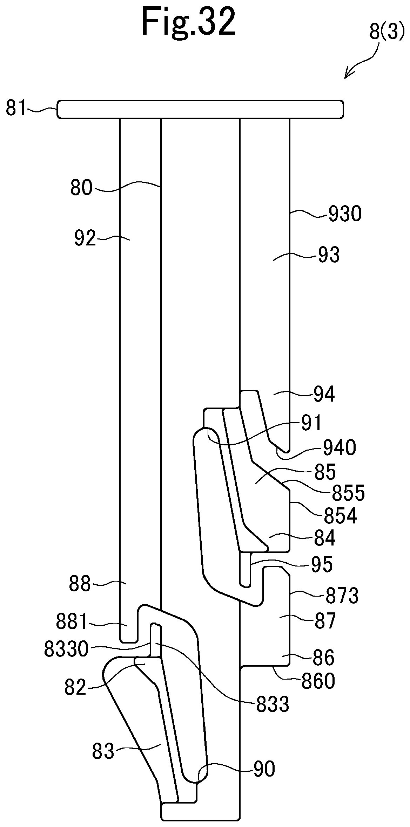

A description will be hereinbelow given for a configuration in which the second shaft 8 is provided with a rib to prevent wobbling of the second shaft 8 relative to the first shaft 7, with reference to Fig. 30 to Fig. 32. As shown in Fig. 32, the second shaft 8 includes a first rib 92 and a second rib 93. Furthermore, in this second shaft 8, an outer surface 854 of the push-in part 85 is aligned with an outer surface 873 of the discharge completion part 87 in the axial direction.

The first rib 92 is a rib that protrudes radially outward from the second main shaft 80, and in this embodiment, it extends axially from a proximal end edge of the second main shaft 80 toward the distal end up to immediately the proximal side of the restriction part 83. That is, the first rib 92 is disposed on the proximal end side in the axial direction relative to the restriction part 83. In this embodiment, the first rib 92 extends continuously between the second proximal end part 81 and the projection part 88. The projection dimension of the first rib 92, that is, the radial dimension of the first rib 92, is uniform.

The first rib 92 passes between the one-side protrusions 73, which are located adjacent to each other in the circumferential direction, in the one-side slit 700 of the first shaft 7 (see Fig. 30). The first rib 92 passes between the one-side protrusions 73, which are located adjacent to each other in the circumferential direction, in the one-side slit 700 of the first shaft 7 from the initial state through the first discharge of the medical liquid, the second discharge of the medical liquid, and until the completion of the discharges of the medical liquid, thereby preventing the second shaft 8 from wobbling relative to the first shaft 7.

The second rib 93 is a rib that protrudes radially outward from the second main shaft 80, and in this embodiment, it extends axially from the proximal end edge of the second main shaft 80 toward the distal end up to just the proximal side of the push-in locked part 84 and the discharge completion part 87 (see Fig. 32). The second rib 93 is disposed on the proximal end side in the axial direction relative to the push-in locked part 84 and the discharge completion part 87. That is, the second rib 93, the push-in locked part 84, and the discharge completion part 87 are arranged in order from the proximal end side toward the distal end side in the axial direction. The second rib 93 is continuous with the second proximal end part 81. The projection dimension of the second rib 93, that is, the radial dimension of the second rib 93, is uniform. An outer surface 930 of the second rib 93 is aligned in the axial direction with the outer surface 854 of the push-in part 85 and the outer surface 873 of the discharge completion part 87.

The second rib 93 has a tapered shape. The distal end surface 940 of the distal end part 94 of the second rib 93 is an inclined surface that extends inclined such that it is positioned more closer to the distal end side as it advances radially outward. The distal end surface 940 faces the proximal end surface 855 of the push-in part 85 in the axial direction. In this embodiment, the distal end surface 940 extends parallel to the proximal end surface 855 of the push-in part 85.

As shown in Fig. 30, the second rib 93 passes between the other-side protrusions 74, which are located adjacent to each other in the circumferential direction, in the other-side slit 701, that is, between the push-in guide parts 6, which are located adjacent to each other in the circumferential direction. The second rib 93 passes between the other-side protrusions 74, which are located adjacent to each other, in the other-side slit 701 from the initial state through the first discharge of the medical liquid, the second discharge of the medical liquid, and until the completion of the discharges of the medical liquid, thereby preventing the second shaft 8 from wobbling relative to the first shaft 7.

The first rib 92 and the second rib 93 are disposed at positions opposite each other with the central axis of the second main shaft 80 therebetween. Specifically, the first rib 92 and the second rib 93 are located opposite each other at positions 180° apart in the circumferential direction of the second main shaft 80.

Note that in the syringe 1,for each functional part, it is possible to adopt other shapes that have similar functions.

According to the present invention, it is possible to provide a syringe that can reliably discharge a medical liquid multiple times.

According to the present invention, there is provided a syringe for administering a content in separate portions, the syringe including: a barrel having a distal end part for discharging the content and a proximal end part located on an opposite side to the distal end part in an axial direction; a plunger that can be pushed into the barrel from the proximal end part toward the distal end part, the plunger including a first shaft, and a second shaft that is inserted into the first shaft and movable in the axial direction relative to the first shaft; and a biasing member that biases the second shaft toward the proximal end side relative to the first shaft, the first shaft including: a restriction locking part for restricting the movement of the second shaft relative to the first shaft by the biasing force of the biasing member, and being composed of a plurality of restriction locking parts arranged at intervals in the axial direction; and a push-in locking part, to which the second shaft is locked, when the plunger for administration of the one separate portion of the content is pushed into the barrel, the second shaft including: a push-in locked part that is locked to the push-in locking part when in the push-in; and a restriction part, the restriction part being configured to be pivotally movable about the starting end as a starting point between a restriction withdrawn position where a free end on the proximal end side of the restriction part is located radially inward and a restriction implementing position where the free end is located radially outward, and the restriction part including a restriction locked part at the free end that is locked to the restriction locking part by the biasing force of the biasing member in the restriction implementing position, the syringe further including a restriction release part that moves the restriction part from the restriction implementing position to the restriction withdrawn position at a position at which the push-in for the administration of the one separate portion of the content is completed, thereby releasing the restriction locked part from the locking engagement with the restriction locking part.

With this configuration, while the locking between the restriction locking part and the restriction locked part restricts the movement of the second shaft toward the proximal end relative to the first shaft by the biasing force of the biasing member, the locking between the push-in locking part and the push-in locked part ensures that the entire plunger is pushed in and thereby the discharge can be made when the plunger is pushed in. Further, at a position at which the pushing-in (discharge) for one separate portion of the content has been completed, the restriction release part releases the locking between the restriction locking part and the restriction locked part. As a result, the second shaft moves toward the proximal end relative to the first shaft by the biasing force of the biasing member, so that the restriction locked part forms the next lock (restriction) with the next restriction locking part located on the proximal end side in the axial direction relative to the restriction locking part to which the restriction locked part was previously locked, and thereby forms the next lock (push-in) between the push-in locking part and the push-in locked part. In this way, the syringe is reliably switched to prepare for the next discharge, ensuring that multiple discharge can be performed.

The syringe can be configured such that the restriction part is moved to the restriction withdrawn position by the restriction release part, and then moves to the restriction implementing position between the restriction locking part of the plurality of the restriction locking parts, to which the restriction part has locked for the administration of the one separate portion of the content, and the restriction locking part that is located adjacent to the restriction locking part locked for the administration of the one separate portion of the content, on the proximal end side in the axial direction.

With this configuration, the restriction part shifts to the restriction implementing position on the distal end side of the next restriction locking part, so that the restriction locked part is securely locked to the next restriction locking part.

The syringe can be configured such that the first shaft includes a first main shaft that extends in the axial direction, the push-in locking part includes a plurality of main-shaft push-in locking parts disposed on the first main shaft at intervals in the axial direction, the second shaft includes a second main shaft that extends in the axial direction, and a push-in part that is disposed on the second main shaft part, the push-in part being configured to be pivotally movable about a starting end as a starting point, which is disposed on the proximal end side of the push-in part, between a push-in withdrawn position where a free end disposed on the proximal end side of the push-in part is located radially inward and a push-in implementing position where the free end is located radially outward, the push-in locked part is a main-shaft push-in locked part disposed at the free end of the push-in part, and is composed of a main-shaft push-in locked part that is locked to the main-shaft push-in locking part when in the push-in in the push-in implementing position.

With this configuration, the push-in part pivotally moves between the push-in implementing position and the push-in withdrawn position, which enables shifting between locking and unlocking the main-shaft push-in locking part and the main-shaft push-in locked part. Thereby, the main-shaft push-in locked part is securely locked to the next main-shaft push-in locking part, which is located on the proximal end side in the axial direction relative to the main-shaft push-in locking part to which the main-shaft push-in locked part was previously locked.

The syringe can be configured such that the syringe further includes a push-in guide part that guides the push-in part from the push-in implementing position to the push-in withdrawn position between two axially adjacent ones of the main-shaft push-in locking parts, the push-in guide part being configured to retain the push-in part in the push-in withdrawn position when the push-in part moves along the axial direction from the main-shaft push-in locking part on the distal end side to the main-shaft push-in locking part on the proximal end side, of the two main-shaft push-in locking parts, and release the retaining of the push-in part when the push-in part is positioned on the proximal end side relative to the main-shaft push-in locking part on the proximal end side.

With this configuration, the push-in part shifts to the push-in implementing position at a position on the proximal end side in the axial direction of the next main-shaft push-in locking part, so that the main-shaft push-in locked part is securely locked to the next main-shaft push-in locking part.

### REFERENCE SIGNS LIST

1: Syringe 2: Barrel 3: Plunger 4: Biasing member 5: Restriction release part 6: Push-in guide part (main-shaft push-in guide part) 7: First shaft 8: Second shaft 9: Discharge nozzle 20: Distal end part 21: Proximal end part 22: Barrel body 23: Finger grip 24: Discharge completion locking part 30: Piston 70: First main shaft 71: First proximal end part 72: First distal end part 73: One-side protrusion 74: Other-side protrusion 75: Protrusion 76: Restriction locking part (main-shaft restriction locking part) 77: Push-in locking part (main-shaft push-in locking part) 80: Second main shaft 81: Second proximal end part 82: Restriction locked part 83: Restriction part 84: Push-in locked part (main-shaft push-in locked part) 85: Push-in part 86: Discharge completion locked part 87: Discharge completion part 88, 89: Projection part 90, 91: Recess 92: First rib 93: Second rib 94: Distal end part 220: Body distal end part 221: Body proximal end part 222: Body connecting part 223: Flange part 230: Grip body part 231: Grip extension portion 232: Body proximal end part 233: Body distal end part 234: Grip proximal end side extension part 240: Proximal end surface 600: Distal end surface 700: One-side slit 701: Other-side slit 702: Guide surface 710: Proximal end surface 731, 732, 733, 734, 735: One-side protrusion 741, 742, 743, 744: Other-side protrusion 751, 752, 753, 754, 755: Protrusion 760: Distal end surface 770: Proximal end surface 800: Second main wall part 801: Second sub-wall part 810: Distal end surface 820: Proximal end surface 830: Starting end 831: Free end 832: Restriction guided part 833: Restriction extension part 840: Distal end surface 841: Proximal end surface 850: Starting end 851: Free end 852: Push-in guided part 853: Push-in extension part 854: Outer surface 855: Proximal end surface 860: Distal end surface 870: Discharge guided part 871, 872: Discharge extension part 873: Outer surface 880: Projection guided part 881: Projection extension part 890: Projection guided part 891, 892, 893: Projection part 930: Outer surface 940: Distal end surface 2320: Proximal end surface 2321: End part 2340: Proximal end surface 7311: Distal end surface 7321: Distal end surface 7310, 7320, 7330, 7340, 7350: Guide surface 7331, 7341, 7351: Distal end surface 7410, 7420, 7430, 7440: Guide surface 7411, 7421, 7431: Proximal end surface 7412, 7422, 7432: Distal end surface 7510, 7520, 7530, 7540, 7550: Guide surface 7511, 7521, 7531, 7541, 7551: Distal end surface 7512, 7522: Proximal end surface 8320: Outer surface 8321: End surface 8330, 8520, 8530, 8700: Outer surface 8710, 8720: Inner surface 8800: Outer surface 8810: Inner surface 8900: Outer surface 8910: Projection guided part 8911: Outer surface 8920: Projection guided part 8921:Projection extension part 8922: Outer surface 8923: Inner surface 8930: Projection guided part 8931: Proximal-end-side projection extension part 8932: Distal-end-side projection extension part 8933: Outer surface 8934, 8935: Inner surface 901: Syringe-type injector 902: Syringe 903: Plunger 903a: First shaft member 903b: Second shaft member 931: First elastic piece 931a, 932a, 933a: Proximal end part 931b, 932b, 933b: Free end 931d, 932d, 933d: Locking projection 932: Second elastic piece 933: Third elastic piece 936: Cylindrical portion 941: Pressing shaft 942: Pressing flange 945a: First support part 945b: Second support part 945c: Third support part 946a: First pressing part 946b: Second pressing part 946c: Third pressing part

## Claims

1. A syringe for administering a content in separate portions, the syringe comprising:
a barrel having a distal end part for discharging the content and a proximal end part located on an opposite side to the distal end part in an axial direction;
a plunger that can be pushed into the barrel from the proximal end part toward the distal end part, the plunger including a first shaft, and a second shaft that is inserted into the first shaft and movable in the axial direction relative to the first shaft; and
a biasing member that biases the second shaft toward the proximal end side relative to the first shaft,
the first shaft comprising:
a restriction locking part for restricting the movement of the second shaft relative to the first shaft by a biasing force of the biasing member, and including a plurality of restriction locking parts arranged at intervals in the axial direction; and
a push-in locking part, to which the second shaft is locked, when the plunger for administration of one separate portion of the content is pushed into the barrel,
the second shaft comprising:
a push-in locked part that is locked to the push-in locking part when in the push-in; and a restriction part,
the restriction part being configured to be pivotally movable about a starting end as a starting point between a restriction withdrawn position where a free end on the proximal end side of the restriction part is located radially inward and a restriction implementing position where the free end is located radially outward, and the restriction part including a restriction locked part at the free end that is locked to the restriction locking part by the biasing force of the biasing member in the restriction implementing position,
the syringe further comprising a restriction release part that moves the restriction part from the restriction implementing position to the restriction withdrawn position at a position at which the push-in for the administration of the one separate portion of the content is completed, thereby releasing the restriction locked part from the locking engagement with the restriction locking part.

2. The syringe according to claim 1, wherein the restriction part is configured to be moved to the restriction withdrawn position by the restriction release part, and then move to the restriction implementing position between the restriction locking part of the plurality of the restriction locking parts, to which the restriction part has locked for the administration of the one separate portion of the content, and the restriction locking part that is located adjacent to the restriction locking part locked for the administration of the one separate portion of the content, on the proximal end side in the axial direction.

3. The syringe according to claim 1 or 2, wherein
the first shaft comprises a first main shaft that extends in the axial direction,
the push-in locking part comprises a plurality of main-shaft push-in locking parts disposed on the first main shaft at intervals in the axial direction,
the second shaft comprises:
a second main shaft that extends in the axial direction,
a push-in part that is disposed on the second main shaft part, the push-in part being configured to be pivotally movable about a starting end as a starting point, which is disposed on the proximal end side of the push-in part, between a push-in withdrawn position where a free end disposed on the proximal end side of the push-in part is located radially inward and a push-in implementing position where the free end is located radially outward,
the push-in locked part is a main-shaft push-in locked part disposed at the free end of the push-in part, and is composed of a main-shaft push-in locked part that is locked to the main-shaft push-in locking part when in the push-in in the push-in implementing position.

4. The syringe according to claim 3, further comprising a push-in guide part that guides the push-in part from the push-in implementing position to the push-in withdrawn position between two axially adjacent ones of the main-shaft push-in locking parts,
the push-in guide part being configured to retain the push-in part in the push-in withdrawn position when the push-in part moves along the axial direction from the main-shaft push-in locking part on the distal end side to the main-shaft push-in locking part on the proximal end side, of the two main-shaft push-in locking parts, and release the retaining of the push-in part when the push-in part is positioned on the proximal end side relative to the main-shaft push-in locking part on the proximal end side.
